# EUROPEAN PATENT APPLICATION

(11) **EP 4 349 979 A1**
(43) Date of publication of application: **10.04.2024**
(21) Application number: 22810597.9
(22) Date of filing: 25.05.2022
(51) Int. Cl.: C12N 9/22, C12N 15/113, C12Q 1/68

(54) **ENGINEERED CAS12I NUCLEASE, EFFECTOR PROTEIN AND USE THEREOF**

(30) Priority: 27.05.2021 WO PCT/CN2021/096477; 27.05.2021 CN 202110581290; 15.11.2021 CN 202111347952
(71) Applicant: Institute Of Zoology, Chinese Academy Of Sciences, Beijing 100101 (CN); Beijing Institute for Stem Cell and Regenerative Medicine, Beijing 100101 (CN)
(72) Inventor: LI, Wei, Beijing 100101 (CN); ZHOU, Qi, Beijing 100101 (CN); CHEN, Yangcan, Beijing 100101 (CN); HU, Yanping, Beijing 100101 (CN); WANG, Xinge, Beijing 100101 (CN); LUO, Shengqiu, Beijing 100101 (CN); CHEN, Yi, Beijing 100101 (CN)
(74) Representative: Lavoix
(86) International application number: PCT/CN2022/095072
(87) International publication number: WO 2022/247873

(57) **Abstract**

Provided is an engineered Cas12i nuclease, which comprises one or more of the following mutations based on a reference Cas12i nuclease: (1) replacing one or more amino acids interacting with PAM in the reference Cas12i nuclease with positively charged amino acids; and/or (2) replacing one or more amino acids involved in opening the double strands of DNA, in the reference Cas12i nuclease with amino acids with aromatic rings; and/or (3) replacing one or more amino acids, which interact with a single-stranded DNA substrate and are located in an RuvC domain in the reference Cas12i nuclease, with positively charged amino acids; and/or (4) replacing one or more amino acids interacting with a DNA-RNA double helix in the reference Cas12i nuclease with positively charged amino acids; and/or (5) replacing one or more polar or positively charged amino acids interacting with the DNA-RNA double helix in the reference Cas12i nuclease with hydrophobic amino acids. The engineered Cas12i nuclease has significantly improved gene editing efficiency and/or significantly reduced off-target phenomenon compared with the reference Cas12i nuclease.

## Description

### PRIORITY INFORMATION

The present application claims the priorities of Chinese patent application CN202111347952.7 filed on November 15, 2021, Chinese patent application CN202110581290.3 filed on May 27, 2021, and international application PCT/CN2021/096477 filed on May 27, 2021, the entire contents of which are incorporated herein by reference in their entirety.

### SEQUENCE LISTING FILE SUBMITTED AT THE SAME TIME

The entire contents of the following ASCII text file are incorporated herein by reference in their entirety: Sequence Listing in Computer Readable Format (CRF) (FF00614PCT-NSequence6th Version-20220525.txt, date: May 25, 2022, size: 218kb).

### FIELD OF THE INVENTION

The present application belongs to the field of biotechnology. More specifically, the present application relates to Cas12i nucleases with improved catalytic activity (e.g., gene editing activity), effector proteins and uses thereof.

### TECHNICAL BACKGROUND

Genome editing is an important and useful technology in genome research. Several systems are available for genome editing, including the clustered regularly interspaced short palindromic repeats (CRISPR)-Cas system, the transcription activator-like effector nuclease (TALEN) system, and the zinc finger nuclease (ZFN) system.

The CRISPR-Cas system is an efficient and cost-effective genome editing technology that can be widely used in a range of eukaryotes from yeast and plants to zebrafish and humans (see review: Van der Oost 2013, Science 339:768-770, and Charpentier and Doudna, 2013, Nature 495: 50-51). The CRISPR-Cas system provides adaptive immunity in archaea and bacteria by combining Cas 12i effector proteins and CRISPR RNA (crRNA). To date, two categories (type 1 and type 2) of CRISPR-Cas systems including six types (types I-VI) have been characterized based on the outstanding functional and evolutionary modularity of the system. Among the type 2 CRISPR-Cas systems, type II Cas9 system and type V-A/B/E/J Cas12a/Cas12b/Cas12e/Cas12j system have been exploited for genome editing and provide broad prospects for biomedical research.

However, current CRISPR-Cas systems have multiple limitations, including limited gene editing efficiency. Therefore, improved methods and systems are needed for efficient genome editing across multiple loci.

### SUMMARY OF THE INVENTION

The present application provides the following technical solutions:
1. An engineered Cas12i nuclease; comprising one or more mutations based on a reference Cas12i nuclease selected from:
   (1) replacing one or more amino acids interacting with PAM in the reference Cas12i nuclease with positively charged amino acids;
   (2) replacing one or more amino acids involved in opening the double-stranded DNA in the reference Cas12i nuclease with amino acids with an aromatic ring;
   (3) replacing one or more amino acids located in the RuvC domain and interacting with the single-stranded DNA substrate in the reference Cas12i nuclease with positively charged amino acids;
   (4) replacing one or more amino acids interacting with the DNA-RNA double helix in the reference Cas12i nuclease with positively charged amino acids; and
   (5) replacing one or more polar or positively charged amino acids interacting with the DNA-RNA double helix in the reference Cas12i nuclease with hydrophobic amino acids;
   preferably, the reference Cas12i nuclease is a wild-type Cas12i2 nuclease with an amino acid sequence SEQ ID NO. 1.
2. The engineered Cas12i nuclease of item 1, wherein the one or more amino acids interacting with the PAM are amino acids located within a distance of 9 angstroms from the PAM in three-dimensional structure, preferably, the one or more amino acids interacting with the PAM are one or more amino acids at the following positions: 176, 178, 226, 227, 229, 237, 238, 264, 447 and 563,
   further preferably, the one or more amino acids interacting with the PAM are one or more of the following amino acids: E176, E178, Y226, A227, N229, E237, K238, K264, T447 and E563,
   further preferably, the one or more amino acids interacting with the PAM are one or more of the following amino acids: E176, K238, T447 and E563,
   wherein, the amino acid position numbers are defined by the corresponding amino acid positions shown in SEQ ID NO. 1.
3. The engineered Cas12i nuclease of item 1 or 2, wherein the positively charged amino acid is R, K or H.
4. The engineered Cas12i nuclease according to any one of items 1-3, wherein the replacement of one or more amino acids interacting with the PAM in the reference Cas 12i nuclease with positively charged amino acids is one or more of the following replacements: E176R, K238R, T447R, and E563R;
   preferably, the engineered Cas12i nuclease comprises any one of the following mutations or mutation combinations: (1) E563R; (2) E176R, T447R, E176R and E563R; (3) K238R and E563R; (4) E176R, K238R and T447R; (5) E176R, K238R, and E563R; (6) E176R, T447R, and E563R; and (7) E176R, K238R, T447R, and E563R;
   wherein, the amino acid positions are defined by the corresponding amino acid positions shown in SEQ ID NO. 1.
5. The engineered Cas12i nuclease according to any one of items 1 to 4, wherein the one or more amino acids involved in opening the double-stranded DNA are the amino acids interacting with the last base pair at the 3' end in PAM relative to the target strand;
   preferably, the one or more amino acids involved in opening the double-stranded DNA are one or more amino acids at the following positions: 163 and 164;
   further preferably, the one or more amino acids involved in opening the double-stranded DNA are one or more of the following amino acids: Q163 and N164;
   further preferably, the amino acid involved in opening the double-stranded DNA is N164;
   wherein, the amino acid position numbers are defined by the corresponding amino acid positions shown in SEQ ID NO. 1.
6. The engineered Cas12i nuclease according to any one of items 1 to 5, wherein said replacing one or more amino acids involved in opening the double-stranded DNA with amino acids with an aromatic ring, and the amino acid with an aromatic ring is F, Y or W, preferably, the amino acid with an aromatic ring is F or Y.
7. The engineered Cas12i nuclease according to any one of items 1 to 6, wherein the replacement of one or more amino acids involved in opening the double-stranded DNA in the reference Cas12i nuclease with amino acids with an aromatic ring comprises one or more of the following replacements: Q163F, Q163Y, Q163W, and N164F; preferably, the Cas12i nuclease comprises N164Y or N164F mutation; further preferably, the engineered Cas12i nuclease comprises N164Y.
8. The engineered Cas12i nuclease according to any one of items 1 to 7, wherein the one or more amino acids located in the RuvC domain and interacting with the single-stranded DNA substrate are amino acids located within a distance of 9 angstroms from the PAM in three-dimensional structure;
   preferably, the one or more amino acids located in the RuvC domain and interacting with the single-stranded DNA substrate are one or more amino acids at the following positions: 323, 327, 355, 359, 360, 361, 362, 388, 390, 391, 392, 393, 414, 417, 418, 421, 424, 425, 650, 652, 653, 696, 705, 708, 709, 751, 752, 755, 840, 848, 851, 856, 885, 897, 925, 926, 928, 929, 932, and 1022;
   further preferably, the one or more amino acids located in the RuvC domain and interacting with the single-stranded DNA substrate are one or more of the following amino acids: E323, L327, V355, G359, G360, K361, D362, L388, N390 , N391, F392, K393, Q414, L417, L418, K421, Q424, Q425, S650, E652, G653, 1696, K705, K708, E709, L751, S752, E755, N840, N848, S851, A856, Q885 M897 , N925, 1926, T928, G929, Y932, and A1022;
   further preferably, the one or more amino acids located in the RuvC domain and interacting with the single-stranded DNA substrate are one or more of the following amino acids: E323, D362, Q425, N925, 1926, N391, Q424 and G929;
   wherein, the amino acid position numbers are defined by the corresponding amino acid positions shown in SEQ ID NO. 1.
9. The engineered Cas12i nuclease according to any one of items 1-8, wherein the replacement of one or more amino acids located in the RuvC domain and interacting with the single-stranded DNA substrate in the reference Cas12i nuclease with positively charged amino acids comprise replacement with R or K. preferably, the positively charged amino acid is R.
10. The engineered Cas12i nuclease according to any one of items 1-9, wherein the replacement of one or more amino acids located in the RuvC domain and interacting with the single-stranded DNA substrate in the reference Cas12i nuclease with positively charged amino acids comprise one or more of the following replacements: E323R, D362R, N391R, Q424R, Q425R, N925R, I926R and G929R;
   preferably, the engineered Cas12i nuclease comprises any one of the following mutations or mutation combinations: (1) E323R; (2) D362R; (3) Q425R; (4) N925R; (5) I926R; (6) E323R and D362R; (7) E323R and Q425R; (8) E323R and I926R; (9) Q425R and I926R; (10) D362R and I926R; (11) N925R and I926R; (12) E323R, D362R and Q425R; (13) E323R, D362R, and I926R; (14) E323R, Q425R, and I926R; (15) D362R, N925R, and I926R; and (16) E323R, D362R, Q425R, and I926R;
   wherein, the amino acid positions are defined by the corresponding amino acid positions shown in SEQ ID NO. 1.
11. The engineered Cas12i nuclease according to any one of items 1 to 10, wherein the one or more amino acids interacting with the DNA-RNA double helix are amino acids located within a distance of 9 angstroms from the DNA-RNA double helix in three-dimensional structure;
   preferably, the one or more amino acids interacting with the DNA-RNA double helix are one or more amino acids at the following positions: 116, 117, 156, 159, 160, 161, 247, 293, 294, 297, 301, 305, 306, 308, 312, 313, 316, 319, 320, 343, 348, 349, 427, 433, 438, 441, 442, 679, 683, 691, 782, 783, 797, 800, 852, 853, 855, 861, 865, 957, 958;
   further preferably, the one or more amino acids interacting with the DNA-RNA double helix are one or more of the following amino acids: G116, E117, A156, T159, E160, S161, E247, G293, E294, N297, T301, 1305, K306, T308, N312, F313, Q316, E319, Q320, E343, E348, E349, D427, K433, V438, N441, Q442, N679, E683, E691, D782, E783, E797, E800, M852, D853, L855, N861, Q865, S957, and D958;
   further preferably, the one or more amino acids interacting with the DNA-RNA double helix are one or more of the following amino acids: G116, E117, T159, S161, E319, E343 and D958; wherein the amino acid positions are defined by the corresponding amino acid positions shown in ID NO:1.
12. The engineered Cas12i nuclease according to any one of items 1-11, wherein the replacement of one or more amino acids interacting with the DNA-RNA double helix in the reference Cas12i nuclease with positively charged amino acids comprises replacement with R or K, preferably, the positively charged amino acid is R.
13. The engineered Cas12i nuclease according to any one of items 1-12, wherein the replacement of one or more amino acids interacting with the DNA-RNA double helix in the reference Cas12i nuclease with positively charged amino acids comprises one or more of the following replacements: G116R, E117R, T159R, S161R, E319R, E343R and D958R;
   preferably, the engineered Cas12i nuclease comprises D958R replacement;
   wherein, the amino acid positions are defined by the corresponding amino acid positions shown in SEQ ID NO. 1.
14. The engineered Cas12i nuclease according to any one of items 1 to 13, the one or more polar or positively charged amino acids interacting with the DNA-RNA double helix are selected from one or more of the following amino acids at positions: 357, 394, 715, 719, 807, 844, 848, 857, and 861, that is, one or more of the following amino acids: H357, K394, R715, R719, K807, K844, N848, R857, and R861;
   wherein, the amino acid positions are defined by the corresponding amino acid positions shown in SEQ ID NO. 1.
15. The engineered Cas12i nuclease according to any one of items 1-14, wherein the replacement of one or more polar or positively charged amino acids interacting with the DNA-RNA double helix in the reference Cas12i nuclease with hydrophobic amino acids comprises replacement with alanine (A).
16. The engineered Cas12i nuclease according to any one of items 1-15, comprising one or more mutations selected from the group consisting of: H357A, K394A, R715A, R719A, K807A, K844A, N848A, R857A, and R861A ;
   preferably, comprising one or more mutations selected from the group consisting of: K394A, R719A, K844A, and R857A;
   wherein, the amino acid positions are defined by the corresponding amino acid positions shown in SEQ ID NO. 1.
17. The engineered Cas12i nuclease according to any one of items 1-16, comprising R719A and K844A amino acid substitutions, or R857A and K844A amino acid substitutions; wherein the amino acid positions are defined by the corresponding amino acid positions shown in SEQ ID NO. 1.
18. The engineered Cas12i nuclease according to any one of items 1 to 17, further comprising one or more flexible region mutations, the mutations increase the flexibility of the flexible region in the reference Cas12i nuclease, the flexible region is selected from amino acid residues 439-443 or amino acid residues 925-929;
   preferably, the flexible region mutation is located at sites 439 and/or 926;
   Further preferably, the flexible region mutation is a mutation of L439 and/or 1926;
   Wherein, the amino acid positions are defined by the corresponding amino acid positions shown in SEQ ID NO. 1.
19. The engineered Cas12i nuclease according to claim 18, wherein the one or more flexible region mutations are: replacing the amino acid in the flexible region with G, and/or inserting one or two Gs thereafter;
   preferably, the one or more flexible region mutations comprise I926G, L439(L+G) or L439(L+GG);
   further preferably, the one or more flexible region mutations comprise L439(L+G) or L439(L+GG);
   wherein the amino acid position numbers are defined by the corresponding amino acid positions shown in SEQ ID NO. 1.
20. An engineered Cas12i nuclease;
   comprising any one or more of following mutations:
   (1) E563R; (2) E176R and T447R; (3) E176R and E563R; (4) K238R and E563R; (5) E176R, K238R and T447R; (6) E176R, T447R and E563R; (7) E176R, K238R and E563R; (8) E176R, K238R, T447R and E563R; (9) N164Y; (10) N164F; (11) E323R; (12) D362R; (13) Q425R; (14) N925R; (15) I926R; (16) D958R; (17) E323R and D362R; (18) E323R and Q425R; (19) E323R and I926R; (20) Q425R and I926R; (21) D362R and I926R; (22) N925R and I926R; (23) E323R, D362R and Q425R; (24) E323R, D362R and I926R; (25) E323R, Q425R and I926R; (26) D362R, N925R and I926R; (27) E323R, D362R, Q425R and I926R; (28) D362R and 1926 G; (29) N925R and I926G; (30) D362R, N925R and I926G; (31) I926R and L439(L+G); (32) I926R and L439(L+GG); (33) E323R, D362R and I926G; (34) R719A and K844A; and (35) R857A and K844A;
      preferably, comprising any one or more of following mutations: (1) E176R, K238R, T447R and E563R;
   (2) N164Y; (3) I926R; (4) E323R and D362R; (4) )I926G; (5) I926R and L439 (L+G); (6) I926R and L439 (L+GG); and (7) D958R;
   wherein the amino acid positions are defined by the corresponding amino acid positions shown in SEQ ID NO. 1.
21. An engineered Cas12i nuclease, comprising any one of the following sets of mutations: (1) E176R, K238R, T447R, E563R and N164Y; (2) E176R, K238R, T447R, E563R and I926R; (3) N164Y, E323R and D362R; (4) E176R, K238R, T447R, E563R, E323R, and D362R; (5) N164Y and I926R; (6) E176R, K238R, T447R, E563R, N164Y and I926R; (7)E176R, K238R, T447R, E563R, N164Y, E323R and D362R; (8) E176R, K238R, T447R, E563R, N164Y, I926R, E323R and D362R; (9) E176R, K238R, T447R, E563R, N164Y, E323R, D362R and I926G; (10) E176R, K238R, T447R, E563R, N164Y, E323R, D362R, I926G and L439(L+GG); (11) E176R, K238R, T447R, E563R, N164Y, E323R, D362R, I926G and L439(L + G); (12) E176R, K238R, T447R, E563R, N164Y and D958R; (13) E176R, K238R, T447R, E563R, I926R and D958R; (14) E176R, K238R, T447R, E563R, E323R, D362R and D958R; (15) N164Y, I926R and D958R; (16) N164Y, E323R, D362R and D958R; (17) E176R, K238R, T447R, E563R, N164Y, I926R and D958R; (18) E176R, K238R, T447R, E563R, N164Y, E323R, D362R and D958R; (19) E176R, K238R, T447R, E563R, N164Y, I926R, E323R, D362R and D958R; (20) E176R, K238R, T447R, E563R, N164Y, E323R, D362R, I926G and D958R; (21) E176R, K238R, T447R, E563R, N164Y, E323R, D362R, I926G, L439(L+GG) and D958R; (22)E176R, K238R, T447R, E563R, N164Y, E323R, D362R, I926G, L439(L+G) and D958R; (23) E176R, K238R, T447R, E563R, N164Y, E323R, D362R and R857A; (24) E176R, K238R, T447R, E563R, N164Y, E323R, D362R and N861A; (25) E176R, K238R, T447R, E563R, N164Y, E323R, D362R and K807A; (26) E176R, K238R, T447R, E563R, N164Y, E323R, D362R and N848A; (27) E176R, K238R, T447R, E563R, N164Y, E323R, D362R and R715A; (28)E176R, K238R, T447R, E563R, N164Y, E323R, D362R and R719A; (29) E176R, K238R, T447R, E563R, N164Y, E323R, D362R and K394A; (30) E176R, K238R, T447R, E563R, N164Y, E323R, D362R and H357A; (31) E176R, K238R, T447R, E563R, N164Y, E323R, D362R and K844A; (32) E176R, K238R, T447R, E563R, N164Y, E323R, D362R, R719A and K844A; or (33) E176R, K238R, T447R, E563R, N164Y, E323R, D362R, R857A and K844A; wherein the amino acid positions are defined by the corresponding amino acid positions shown in SEQ ID NO. 1.
22. The engineered Cas12i nuclease according to any one of items 1 to 21, comprising the engineered Cas12i nuclease with the amino acid sequence shown in any one of SEQ ID NOs. 2 to 24, or the amino acid sequence having at least 80% identity with any amino acid sequence shown in SEQ ID NOs. 2-24.
23. An engineered Cas12i effector protein, comprising the engineered Cas12i nuclease or functional derivative thereof of any one of items 1 to 22;
   optionally, the engineered Cas12i nuclease or functional derivative thereof has enzymatic activity, or the engineered Cas12i nuclease or functional derivative thereof is an enzyme inactive mutant.
24. The engineered Cas12i effector protein of item 23, wherein the Cas12i effector protein is capable of inducing double-strand breaks or single-strand breaks in DNA molecules.
25. The engineered Cas12i effector protein of item 23, wherein the engineered Cas12i nuclease or functional derivative thereof is an enzyme inactive mutant comprising one or more of the following mutations: D599A, E833A, S883A, H884A, R900A and D1019A; wherein the amino acid positions are defined by the corresponding amino acid positions shown in SEQ ID NO. 1.
26. The engineered Cas12i effector protein according to any one of items 23 to 25, further comprising a functional domain fused to the engineered Cas12i nuclease or a functional derivative thereof.
27. The engineered Cas12i effector protein of item 26, wherein the functional domain is one or more selected from the group consisting of: translation initiation domain, transcription repression domain, transactivation domain, epigenetic modification domain, nucleobase editing domain, reverse transcriptase domain, reporter domain and nuclease domain.
28. The engineered Cas12i effector protein according to any one of items 23 to 27, the engineered Cas12i effector protein comprising: a first polypeptide comprising the N-terminal portion of the engineered Cas 12i nuclease or a functional derivative thereof and a second polypeptide comprising the C-terminal portion of the engineered Cas12i nuclease or a functional derivative thereof, wherein the first polypeptide and the second polypeptide are capable of associating with each other in the presence of a guide RNA comprising a guide sequence to form a clustered regularly interspaced short palindromic repeats (CRISPR) complex that specifically binds to a target nucleic acid comprising a target sequence complementary to the guide sequence;
   preferably, the first polypeptide comprises amino acid residues 1~X of the N-terminal portion of the engineered Cas12i nuclease according to any one of items 1 to 22, the second polypeptide comprises amino acid residues X+1 to the C-terminal portion of the engineered Cas12i nuclease according to any one of items 1 to 22;
   optionally, the first polypeptide and the second polypeptide each comprise a dimerization domain;
   optionally, the dimerization domain of the first polypeptide and the dimerization domain of the second polypeptide associate with each other in the presence of an inducer.
29. An engineered CRISPR-Cas12i system, comprising:
   (a) the engineered Cas12i nuclease according to any one of items 1-28, or the engineered Cas12i effector protein according to any one of items 23-28; and
   (b) a guide RNA comprising a guide sequence complementary to a target sequence, or one or more nucleic acids encoding the guide RNA,

   wherein the engineered Cas12i nuclease or engineered Cas12i effector protein and the guide RNA can form a CRISPR complex that specifically binds to the target nucleic acid comprising the target sequence and induces modification of the target nucleic acid;
   preferably, the guide RNA is crRNA comprising the guide sequence;
   further preferably, the engineered CRISPR-Cas12i system comprises a precursor guide RNA array encoding multiple crRNAs;
   or, preferably, the engineered Cas12i nuclease or engineered Cas12i effector protein is a master editor, and the guide RNA is a prime editing guide RNA (pegRNA).
30. The engineered CRISPR-Cas12i system of item 29, comprising one or more vectors encoding the engineered Cas12i nuclease or engineered Cas12i effector protein;
   preferably, the one or more vectors are selected from the group consisting of: retroviral vectors, lentiviral vectors, adenoviral vectors, adeno-associated vectors and herpes simplex vectors;
   further preferably, the one or more vectors are adeno-associated virus AAV vectors;
   further preferably, the AAV vector also encodes the guide RNA.
31. A method for detecting target nucleic acid in a sample, comprising:
   (a) contacting the sample with the engineered CRISPR-Cas12i system of item 29 and a tagged detection nucleic acid that is single-stranded and does not hybridize to the guide sequence of the guide RNA; and
   (b) measuring a detectable signal generated by cleavage of the tagged detection nucleic acid by the engineered Cas12i nuclease or engineered Cas12i effector protein, thereby detecting the target nucleic acid.
32. A method of modifying a target nucleic acid comprising a target sequence, comprising contacting the target nucleic acid with the engineered CRISPR-Cas12i system of item 29 or 30;
   preferably, the method is performed in vitro, ex vivo or in vivo;
   further preferably, the target nucleic acid is present in a cell;
   further preferably, the cell is a bacterial cell, a yeast cell, a mammalian cell, a plant cell or an animal cell;
   further preferably, the target nucleic acid is genomic DNA;
   further preferably, the target sequence is associated with a disease or disorder;
   further preferably, the engineered CRISPR-Cas12i system comprises a precursor guide RNA array encoding multiple crRNAs, wherein each crRNA comprises a different guide sequence.
33. Use of the engineered CRISPR-Cas12i system as claimed in claim 29 or 30 in the preparation of a medicament for the treatment of a disease or disorder associated with a target nucleic acid in a cell of an individual; preferably, the disease or disorder is selected from the group consisting of: cancer, cardiovascular disease, genetic disease, autoimmune disease, metabolic disease, neurodegenerative disease, eye disease, bacterial infection and viral infection.
34. A method of treating a disease or disorder associated with a target nucleic acid in a cell of an individual, comprising using the method of item 32 to modify the target nucleic acid in the cell of the individual, thereby treating the disease or disorder; preferably, the disease or disorder is selected from the group consisting of: cancer, cardiovascular disease, genetic disease, autoimmune disease, metabolic disease, neurodegenerative disease, eye disease, bacterial infection and viral infection.
35. A method of modifying a target nucleic acid comprising a target sequence, comprising contacting the target nucleic acid with the engineered CRISPR-Cas12i system described in claim 29 or 30.
36. A composition or kit, comprising the engineered Cas12i nuclease according to any one of items 1-22, the engineered Cas12i effector protein according to any one of items 23-28.
37. An engineered cell, comprising a modified target nucleic acid, wherein the target nucleic acid is modified by the method of item 32 or 35.
38. An engineered non-human animal, comprising one or more engineered cells of item 37.

### THE BENEFICIAL EFFECTS ACHIEVED BY THE TECHNICAL SOLUTION OF THIS APPLICATION

The engineered Cas 12i nuclease and its effector protein in the present application have higher activities, such as catalytic efficiency in cutting nucleic acid substrates and gene editing efficiency in cells. The engineered Cas12i nuclease in the present application has superior gene editing efficiency in mammalian cells (such as human cells) than existing conventional Cas gene editing tools; for example, some exemplary Cas12i2 nuclease mutants in the present application are tested for gene editing efficiency at multiple sites (such as 62 sites) in human cells, and it is found that the gene editing efficiency at 57 sites exceeded about 60%, with an average gene editing efficiency of nearly 70%. In some embodiments, the engineered Cas12i nuclease and its effector protein in the present application also have one or more of the following advantages: the protein is small (1,054 aa), the constitution of the crRNA is simple, the PAM sequence is simple, and the protein itself can process precursors crRNA. In addition, this application provides a further artificially modified Cas12i nuclease with lower off-target rate and higher specificity based on the highly active engineered Cas12i nuclease (such as SEQ ID NO. 8). These advantages make the highly efficient engineered Cas12i nuclease and its effector protein of the present application very suitable for gene editing or gene regulation in vivo.

### BRIEF DESCRIPTION OF THE DRAWINGS

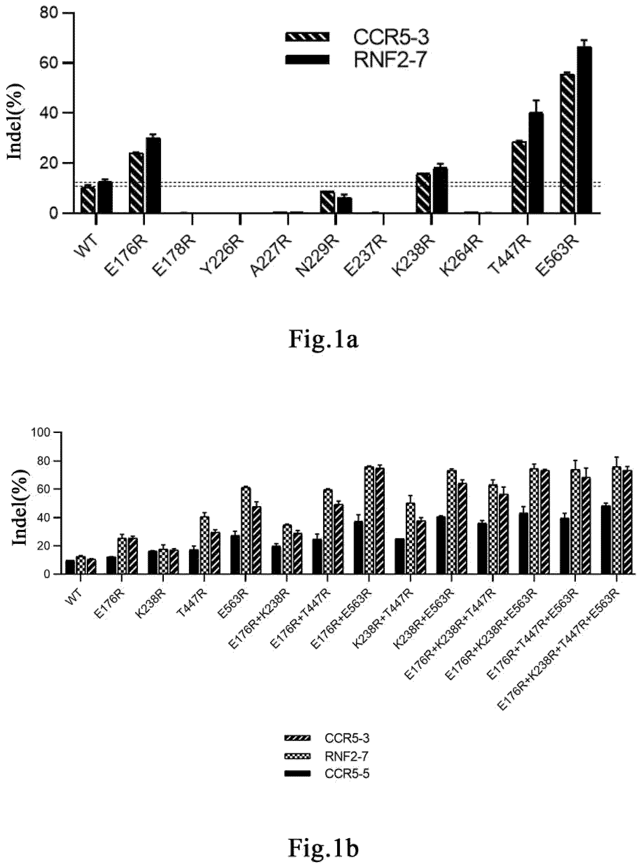

Fig. 1a: the amino acids interacting with the PAM in the reference Cas12i nuclease (wild-type Cas12i nuclease shown in SEQ ID NO. 1) are replaced with positively charged amino acids, thereby improving the efficiency of gene editing. As shown in the figure, four mutants, E176R, K238R, T447R, and E563R, can significantly improve the efficiency of gene editing in human 293T cells.
Fig. 1b: Combining the amino acid mutations (E176R, K238R, T447R, E563R) in Fig. 1a that can significantly improve gene editing efficiency, it is found that the combined mutants can exhibit higher gene editing efficiency in human 293T cells.

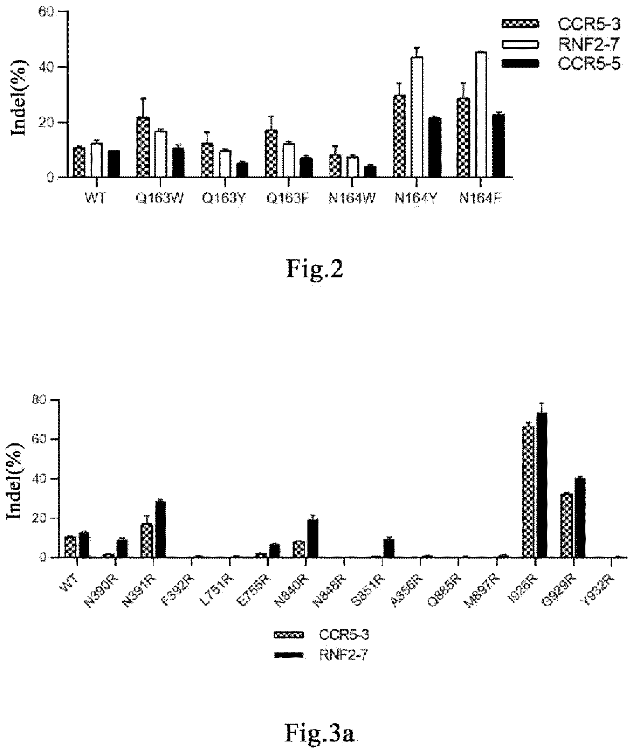

Fig. 2: The amino acids involved in opening the double-stranded DNA in the reference Cas 12i nuclease are replaced with aromatic amino acids, thereby improving the efficiency of gene editing. As shown in the figure, these mutants, Q163F, Q163Y, Q163W, N164F, and N164Y, can significantly improve the efficiency of gene editing in human 293T cells.

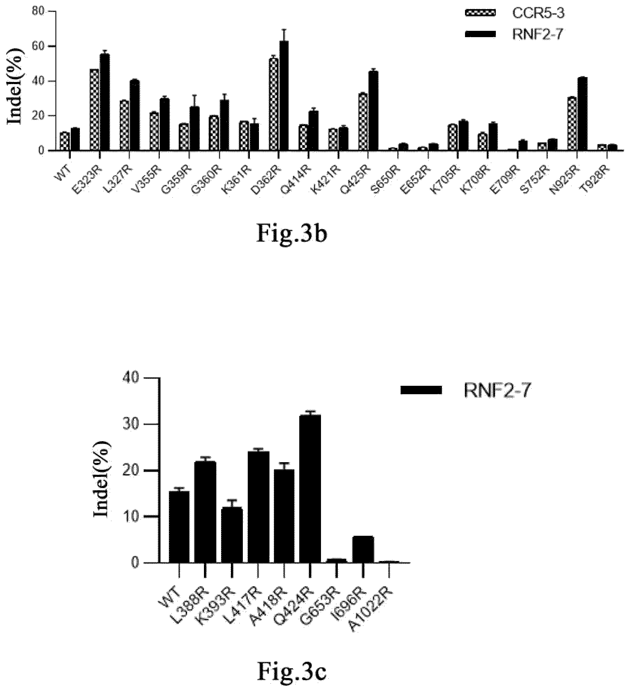

Figs. 3a, 3b, 3c: The amino acids located in the RuvC domain and interacting with the single-stranded DNA substrate in the reference Cas12i nuclease are replaced with positively charged amino acids, thereby improving the efficiency of gene editing. As shown in Figs. 3a, 3b, and 3c, mutants, such as E323R, L327R, V355R, G359R, G360R, D362R, N391R, Q424R, Q425R, N925R, I926R, and G929R, can significantly improve the efficiency of gene editing in human 293T cells.

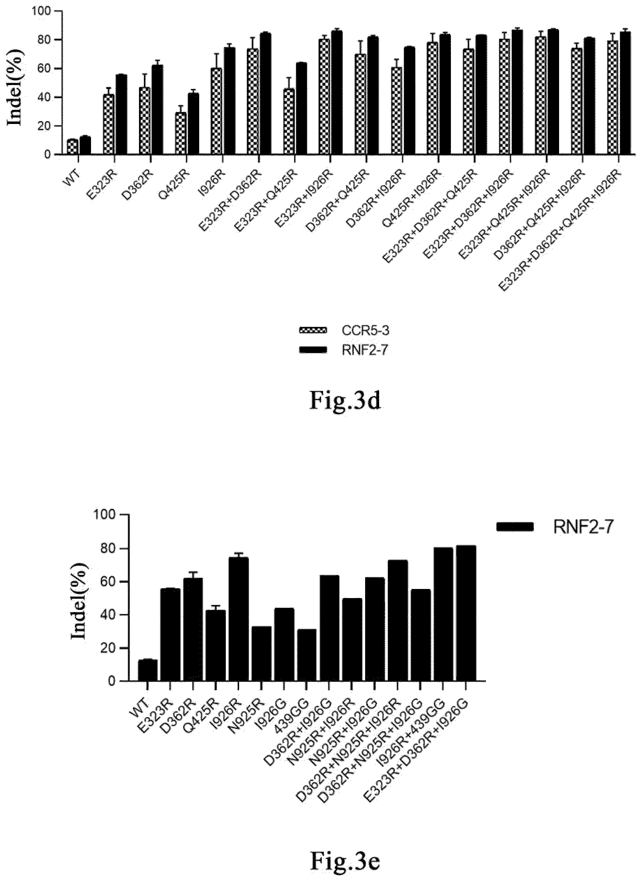

Fig. 3d: Combining the efficiency-improving point mutations in Figs. 3a and 3b, it is found that the combined mutants can exhibit higher gene editing efficiency in human 293T cells.
Fig. 3e: Combining the efficiency-improving point mutations in Figs. 3a and 3b and the mutants modified according to the principle of molecular flexibility (L439 (L+GG), I926G), it is found that the combined mutants can exhibit higher gene editing efficiency in human 293T cells.

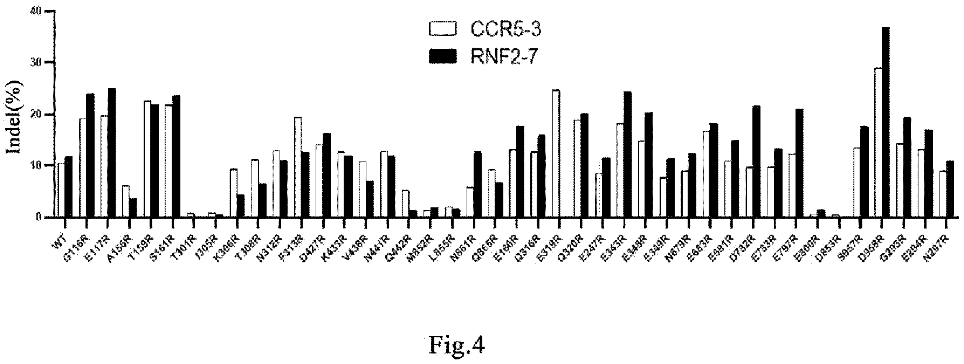

Fig. 4: the amino acids interacting with the DNA-RNA double helix in the reference Cas12i enzyme are replaced with positively charged amino acids, thereby improving the efficiency of gene editing. As shown in Fig. 4, mutants, such as G116R, E117R, T159R, S161R, E319R, E343R, and D958R, can significantly improve the gene editing efficiency in human 293T cells. Wherein, the D958R mutation is the best.

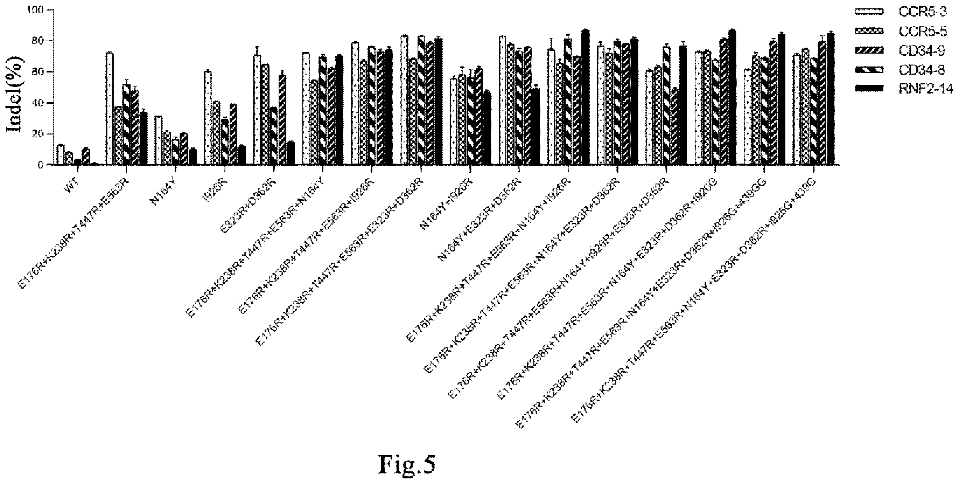

Fig. 5: Combining the high-efficiency mutants obtained by the three modification strategies in Figs. 1a to 3e and the mutants modified according to the principle of molecular flexibility (L439 (L+GG), I926G), it is found that the combined mutants can exhibit higher gene editing efficiency in human 293T cells. The combination can greatly improve gene editing efficiency. The mutant with the best gene editing effect is selected and named CasXX for use in subsequent experiments. CasXX (SEQ ID NO. 8) is a Cas12i engineering enzyme with E176R+K238R+T447R+E563R+N164Y+ E323R+D362R mutation combination (based on the reference Cas12i2 having amino acid sequence of SEQ ID NO. 1).

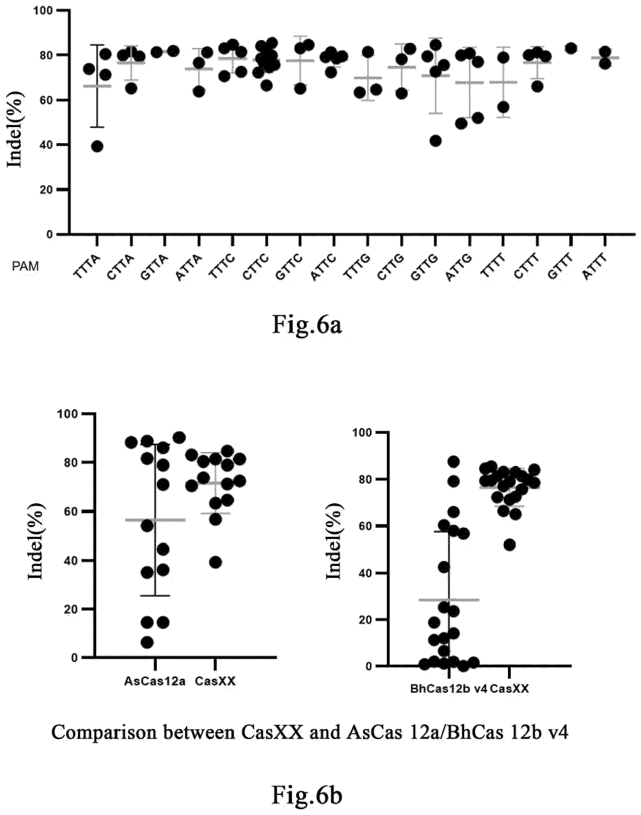

Fig. 6a: Summary of gene editing efficiency of CasXX at 62 human genome sites. PAM=NTTN.
Fig. 6b: Comparison of gene editing efficiency between CasXX, AsCas12a, and BhCas 12b v4.

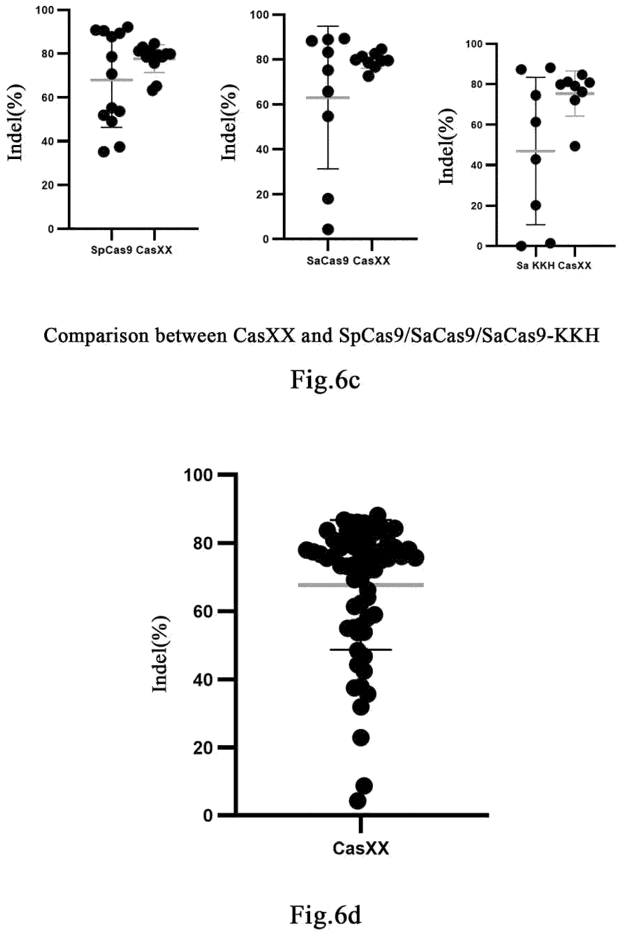

Fig. 6c: Comparison of gene editing efficiency between CasXX and SpCas9, SaCas9, SaCas9- KKH.
Fig. 6d: Statistics of gene editing efficiency of CasXX in mouse Hepa1-6 cell line. It can be seen that CasXX demonstrated powerful gene editing capabilities at 65 sites in mouse Hepa1-6 cells, with an average gene editing efficiency of more than 60%.

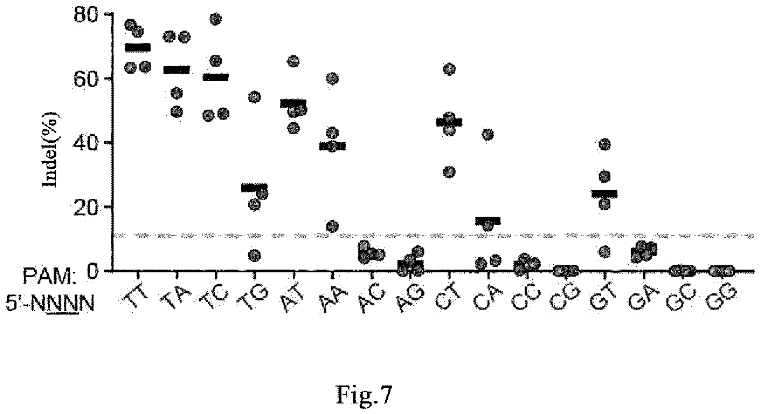

Fig. 7 shows the gene editing efficiency of CasXX at 64 human gene sites. Wherein, the PAM sequences contained in these 64 sites cover all NNNN combinations: NTTN, NTAN, NTCN, NTGN, NATN, NAAN, NACN, NAGN, NCTN, NCAN, NCCN, NCGN, NGTN, NGAN, NGCN, NGGN. As shown in Fig. 7, CasXX shows high gene editing efficiency in the PAM sequences such as NTTN, NTAN, NTCN, NTGN, NATN, NAAN, NCTN, NCAN, and NGTN. The average gene editing efficiency at these sites exceeds 40%.

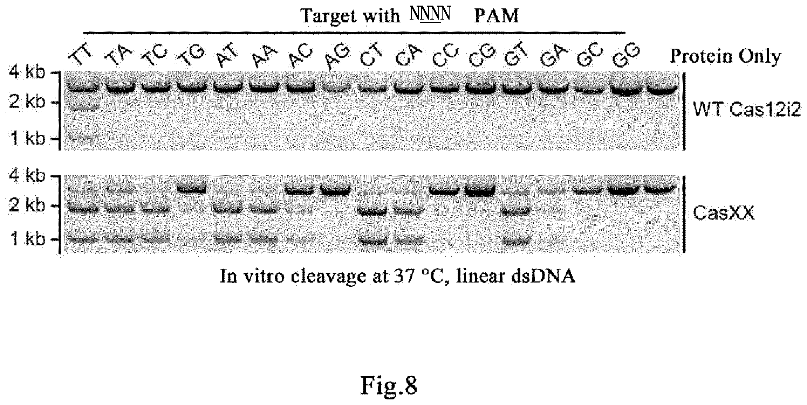

Fig. 8 shows the results of in vitro cleavage of double-stranded DNA by wild-type Cas12i2 protein (SEQ ID NO. 1) and CasXX protein. The wild-type Cas12i2 protein only has partial cleavage efficiency for double-stranded DNA comprising NTTN PAM, but has almost no cleavage activity for the remaining PAMs. However, the CasXX protein exhibits high cleavage efficiency for double-stranded DNA comprising NTTN, NTAN, NTCN, NTGN, NATN, NAAN, NACN, NCTN, NCAN, NGTN, and NGAN PAM. It can almost completely cut double-stranded DNA comprising NTTN, NTAN, NTCN, NATN, NAAN, NACN, NCTN, NCAN, and NGTN PAM.

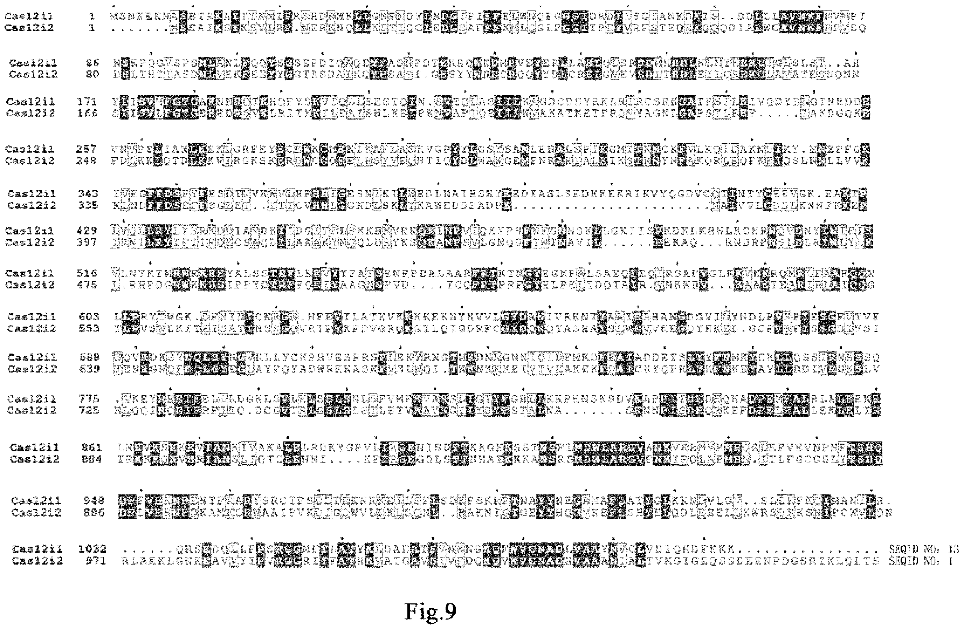

Fig. 9: Homology alignment of the amino acid sequences of Cas12i2 (SEQ ID NO. 1) and Cas12i1 (SEQ ID NO. 13). The amino acids marked with shades represent the same amino acids in the two Cas12i proteins, and the amino acids marked with white boxes represent the amino acids with similar properties in the two Cas12i proteins.

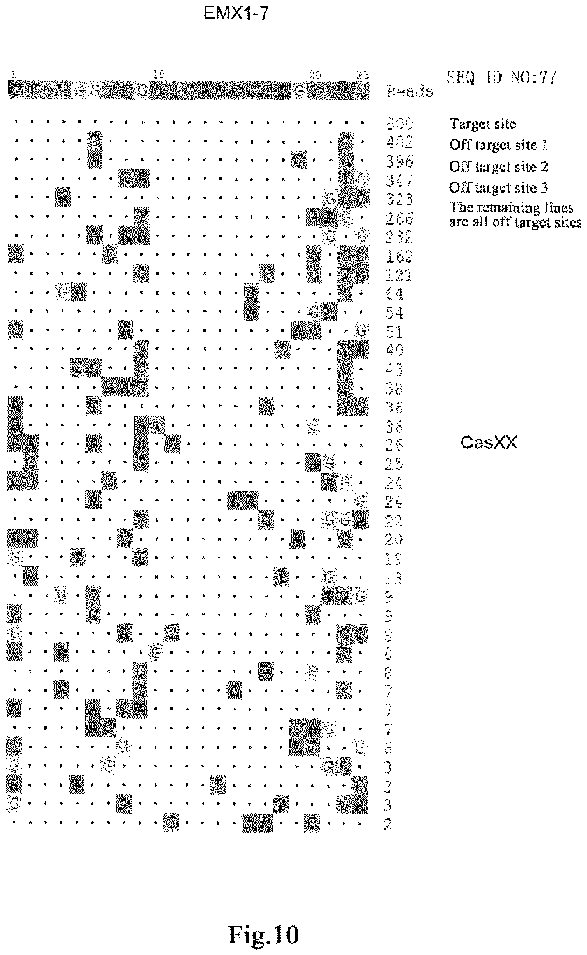

Fig. 10 shows the use of GUIDE-Seq to detect the off-target effect of CasXX at the EMX1-7 target site. The numbers after the sequence represent the reads measured for each sequence. The first row of sequences is the reference target sequence (SEQ ID NO. 77), and the following sequences represent the target sequence and off-target sequence respectively, as well as the reads enriched by double-stranded DNA tags in the target sequence and off-target sequence. This figure illustrates that CasXX has off-target effects at the EMX1-7 site.

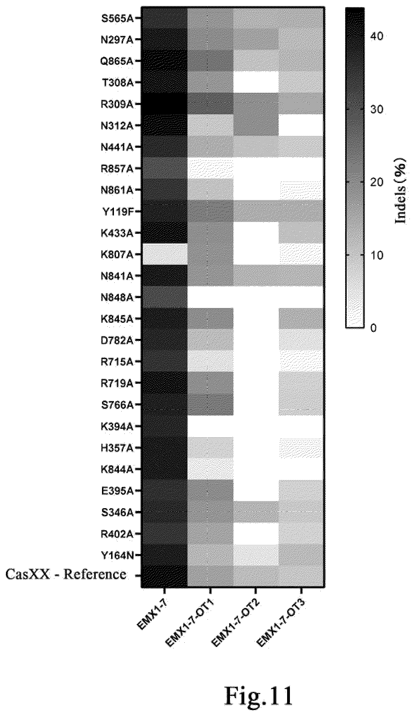

Fig. 11 shows the construction of a new HF mutant by adding new point mutations or deleting original point mutations based on the original sequence of CasXX. Experimental results show that these single point mutants based on CasXX sequences, R857A, N861A, K807A, N848A, R715A, R719A, K394A, H357A, and K844A, can effectively reduce the indel rate at EMX1-7-OT-1, EMX1-7-OT-2, and EMX1-7-OT-3.

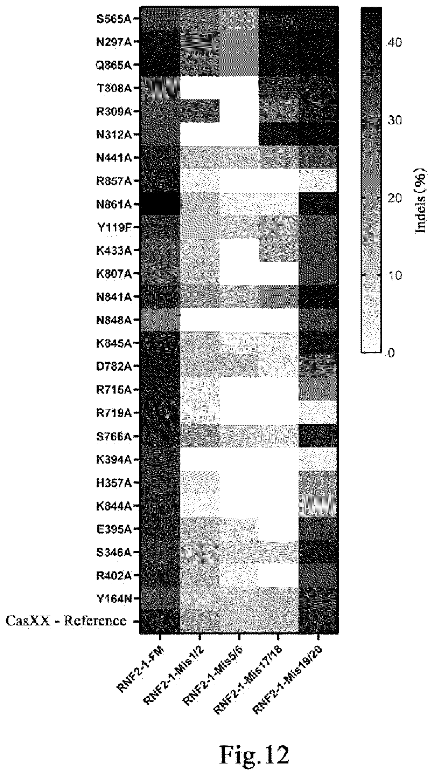

Fig. 12 shows the construction of a new HF mutant by adding new point mutations or deleting original point mutations based on the original sequence of CasXX. Experimental results show that these single point mutants based on the CasXX sequence, R857A, N861A, K807A, N848A, R715A, R719A, K394A, H357A, and K844A, are can effectively reduce the indel rate by using simulated off-target crRNA RNF2-1-Mis-1/2, crRNA RNF2-1-Mis-5/6, crRNA RNF2-1-Mis-17/18, and crRNA RNF2-1-Mis-19/20.

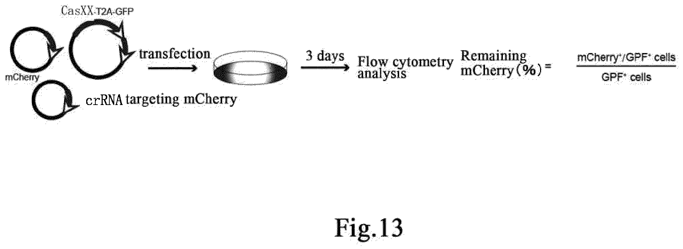

Fig. 13 is an experimental process for using a fluorescent reporter system to screen mutants (CasXX-based HF mutants) that improve the specificity of gene editing. 600ng of plasmid encoding Cas protein, 300ng of plasmid encoding crRNA and 100ng of plasmid encoding mCherry are transfected into cells cultured in a 24-well cell culture dish. Three days after transfection, flow cytometry analysis is used to calculate the proportion of remaining mCherry positive for each sample. The calculation formula is shown in the figure.

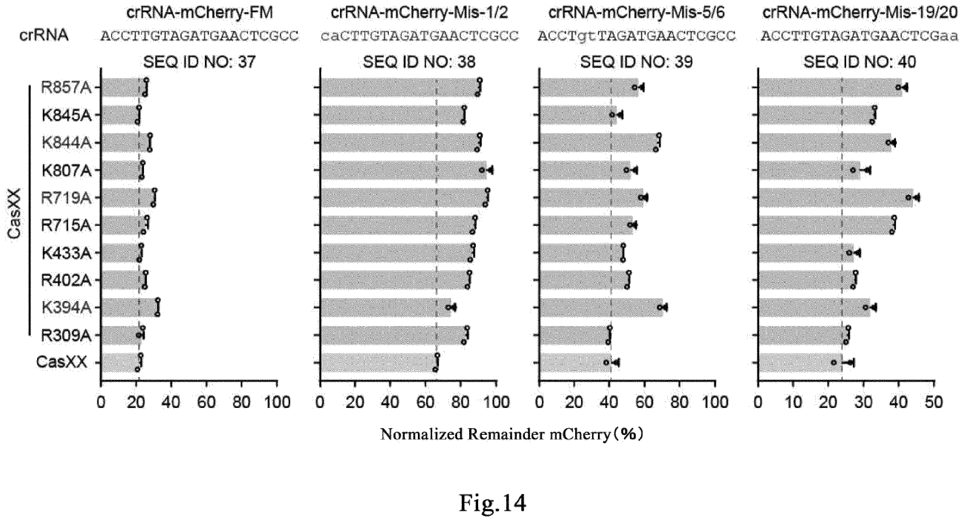

Fig. 14 shows CasXX and its mutants editing the mCherry gene located on the plasmid in 293T cells. After plasmid transfection, the cells are cultured for three days and flow cytometric analysis is performed. The higher the gene editing efficiency of Cas protein, the lower the proportion of mCherry-positive cells will be. The experimental scheme corresponds to the schematic diagram in Fig. 13; the spacer sequences for mCherry-FM, mCherry-Mis-1/2, mCherry-Mis-5/6 and mCherry-Mis-19/20 are respectively shown in SEQ ID NO. 37, 38, 39 and 40.

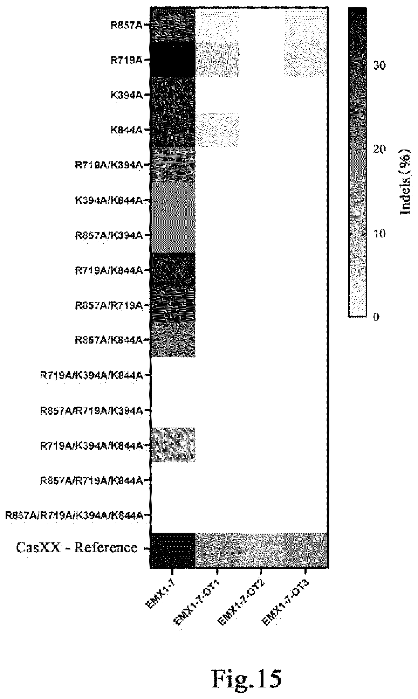

Fig. 15 shows the construction of a new HF mutant combination based on the original sequence of CasXX. Experimental results show that mutants based on CasXX sequences, R857A, R719A, K394A, K844A, R719A/K844A and R857A/K844A, can effectively reduce the off-target indel rate at EMX1-7-OT-1, EMX1-7-OT-2, and EMX1-7-OT-3 without sacrificing efficiency of on-target sites.

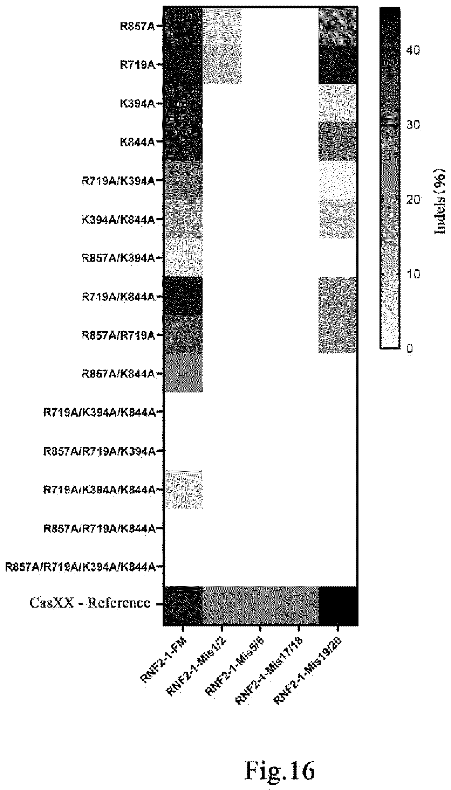

Fig. 16 shows the construction of a new HF mutant combination based on the original sequence of CasXX. Experimental results show that these CasXX sequence-based mutants, R857A, R719A, K394A, K844A, R719A/K844A and R857A/K844A, can effectively reduce the indel rate without sacrificing the efficiency of on-target sites by using simulated off-target crRNA-RNF2-1-Mis-1/2, crRNA-RNF2-1-Mis-5/6, crRNA-RNF2-1-Mis-17/18 and crRNA-RNF2-1-Mis-19/20.

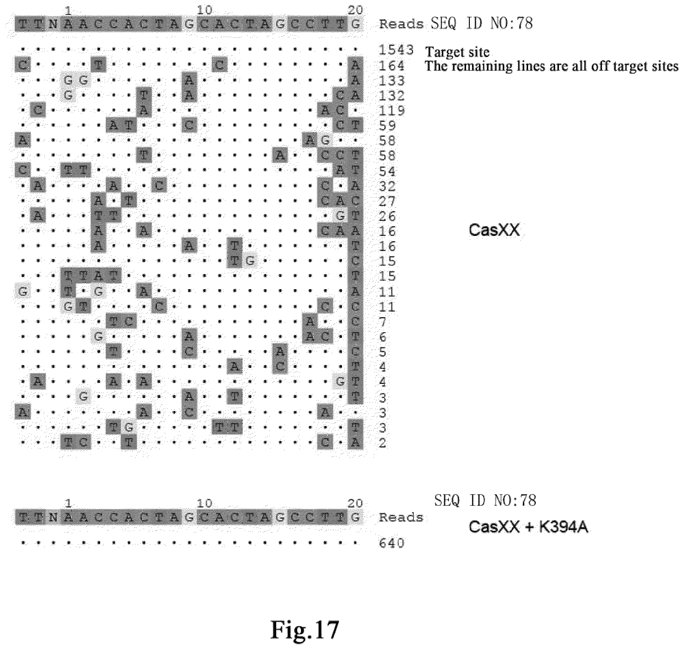

Fig. 17 shows the use of GUIDE-Seq to detect the off-target effects of CasXX and CasXX+K394A mutants at the CD34-7 target site. The number after the sequence represents the number of reads detected for each sequence. The first row of sequences is the reference target sequence (SEQ ID NO. 78), and the following sequences represent the target sequence and off-target sequence respectively, as well as the number of reads enriched by double-stranded DNA tags in the target sequence and off-target sequence. This figure shows that CasXX has an off-target effect at the CD34-7 site, but the CasXX+K394A mutant has no off-target effect at this site.

### DETAILED DESCRIPTION OF THE INVENTION

It should be noted that certain words are used in the specification and claims to refer to specific components. Those skilled in the art will understand that skilled persons may use different names to refer to the same component. This specification and the claims do not use difference in nouns as a way to distinguish components, but rather use differences in functions of the components as a criterion for distinction. As the words "comprise" or "comprise" mentioned throughout the specification and claims are open-ended terms, they should be interpreted as "include but not limited to." The following descriptions of the specification are preferred embodiments for implementing the present invention. However, the descriptions are for the purpose of general principles of the specification and are not intended to limit the scope of the present invention. The protection scope of the present invention shall be determined by the appended claims. Unless otherwise defined, all technical and scientific terms used herein have the same meaning as commonly understood by one of ordinary skill in the art to which this disclosure belongs.

### I. The term

As used herein, "effector protein" refers to a protein having an activity such as site specific binding activity, single-stranded DNA cleavage activity, double-stranded DNA cleavage activity, single-stranded RNA cleavage activity, DNA or RNA modification (e.g., cleavage, base substitution, insertion, removal) or transcriptional regulatory activity.

As used herein, "guide RNA" and "gRNA" are used interchangeably herein and refer to RNA capable of forming a complex with a Cas12i effector protein and a target nucleic acid (e.g., double-stranded DNA). This article also considers precursor guide RNA arrays that can be processed into multiple crRNAs. "crRNA" or "CRISPR RNA" comprises a guide sequence with sufficient complementarity to a target sequence of a target nucleic acid (e.g., double-stranded DNA) that directs the CRISPR complex to specifically bind to the target sequence of the target nucleic acid.

As used herein, the term "CRISPR array" refers to a segment of nucleic acid (e.g., DNA) that comprises CRISPR repeats and spacers, starting with the first nucleotide of the first CRISPR repeat and ending with the last nucleotide of the last (terminal) CRISPR repeat. Ends with one nucleotide. Typically, each spacer in a CRISPR array is located between two repeats. The term "CRISPR repeat" or "CRISPR direct repeat" or "direct repeat" as used herein refers to multiple short direct repeats that exhibit very little or no sequence change in a CRISPR array. Appropriately, direct repeats can form stem-loop structures.

The terms "nucleic acid " , "polynucleotide" , and "nucleotide sequence" are used interchangeably and refer to a polymeric form of nucleotides of any length, including deoxyribonucleotides, ribonucleotides, combinations and analogs thereof. "Oligonucleotide" and "Oligomeric nucleotide" are used interchangeably and refer to short polynucleotides of no more than about 50 nucleotides. As used herein, "complementarity" refers to the ability of a nucleic acid to form hydrogen bonds with another nucleic acid through traditional Watson-Crick base pairing. Percent complementarity represents the percentage of residues in a nucleic acid molecule that can form hydrogen bonds (i.e., Watson-Crick base pairing) with a second nucleic acid (e.g., 5, 6, 7, 8, 9, and 10 out of 10, complement each other by approximately 50%, 60%, 70%, 80%, 90%, and 100%, respectively). "Perfectly complementary" means that all contiguous residues of a nucleic acid sequence form hydrogen bonds with the same number of contiguous residues in a second nucleic acid sequence. As used herein, "substantially complementary" refers to a region with approximately 40, 50, 60, 70, 80, 100, 150, 200, 250 or more nucleotides, with a degree of complementarity of at least about any of 70%, 75%, 80%, 85%, 90%, 95%, 97%, 98%, 99% or 100, or refers to two nucleic acids that hybridize under stringent conditions.

As used herein, "stringent conditions" for hybridization refer to conditions under which a nucleic acid having complementarity to a target sequence hybridizes primarily to the target sequence and does not substantially hybridize to non-target sequences. Stringent conditions are often sequence dependent and vary depending on many factors. Generally, the longer the sequence, the higher the temperature at which the sequence will specifically hybridize to its target sequence. Non-limiting examples of stringent conditions are described in detail in "Overview of principles of hybridization and the strategy of nucleic acid probe assay " , Chapter 2, Part 1, Laboratory Techniques in Biochemistry and Molecular Biology-Hybridization with Nucleic Acid Probes, Tijssen (1993), Elsevier, N, Y.

"Hybridization" refers to a reaction in which one or more polynucleotides react to form a complex that is stabilized by hydrogen bonding between the bases of nucleotide residues. Hydrogen bonding can occur through Watson-Crick base pairing, Hoogstein binding, or in any other sequence-specific manner. A sequence capable of hybridizing to a given sequence is called the "complement" of that given sequence.

"Percent sequence identity (%)" for a nucleic acid sequence is defined as the percentage of nucleotides in a candidate sequence that are identical to those in a specific nucleic acid sequence after aligning the sequences (if necessary) by allowing for gaps to achieve the maximum percent sequence identity. "Percent sequence identity (%)" for a peptide, polypeptide or protein sequence is the percentage of amino acid residues in a candidate sequence that are replaced with the same amino acid residues in a specific peptide or amino acid sequence after aligning the sequences (if necessary) by allowing for gaps to achieve maximum percent sequence homology. For the purpose of determining percent amino acid sequence identity, alignment can be accomplished in a variety of ways within the skill of the art, for example, using a publicly available computer software, such as BLAST, BLAST-2, ALIGN or MEGALIGN^{™} (DNASTAR). Those skilled in the art can determine appropriate parameters for measuring alignment, including any algorithms required to achieve maximal alignment over the full length of the sequences being compared.

The terms "polypeptide" and "peptide" are used interchangeably herein and refer to a polymer of amino acids of any length. The polymer may be linear or branched, it may contain modified amino acids, and it may be interrupted by non-amino acids. A protein can have one or more polypeptides. The term also encompasses amino acid polymers that have been modified; for example, disulfide bond formation, glycosylation, lipidation, acetylation, phosphorylation, or any other manipulation (such as conjugation to a labeling component).

As used herein, "variant" is construed as a polynucleotide or polypeptide, respectively, that differs from a reference polynucleotide or polypeptide but retains essential properties. Typical variants of a polynucleotide differ from the nucleic acid sequence of another reference polynucleotide. Changes in the variant nucleic acid sequence may or may not alter the amino acid sequence of the polypeptide encoded by the reference polynucleotide. Nucleotide changes can result in amino acid substitutions, additions, deletions, fusions, and truncations in the polypeptide encoded by the reference sequence, as described below. Typical variants of a polypeptide differ in amino acid sequence from another reference polypeptide. Typically, the differences are limited such that the sequences of the reference polypeptide and the variant are very similar overall and identical in many regions. The amino acid sequences of the variant and reference polypeptides may differ by any combination of one or more substitutions, additions, deletions. The substitutions or inserted amino acid residue may or may not be the amino acid residue encoded by the genetic code. Variants of a polynucleotide or polypeptide may be naturally occurring (such as allelic variants), or may be unknown naturally occurring variants. Non-naturally occurring variants of polynucleotides and polypeptides can be prepared by mutagenesis techniques, by direct synthesis, and by other recombinant methods known to those skilled in the art.

As used herein, the term "wild type" has the meaning commonly understood by those skilled in the art to mean the typical form of an organism, strain, gene or trait that distinguishes it from mutants or variants as it occurs in nature. It can be isolated from natural resources and not deliberately modified.

As used herein, the terms "non-naturally occurring" or "engineered" are used interchangeably and refer to artificial involvement. When these terms are used to describe a nucleic acid molecule or polypeptide, it is meant that the nucleic acid molecule or polypeptide is at least substantially free of at least one other component with which it is naturally associated or naturally occurring.

As used herein, the term "orthologue/ortholog" has the meaning commonly understood by those of ordinary skill in the art. As further guidance, as referred to herein, an "ortholog" of a protein refers to a protein belonging to a different species that performs the same or similar function as the protein of which it is an ortholog.

As used herein, the term "identity" is used to mean a sequence match between two polypeptides or between two nucleic acids. When a position in two compared sequences is occupied by the same base or amino acid monomer subunit (for example, a position in each of two DNA molecules is occupied by adenine, or a position in each of two polypeptides is has one position occupied by lysine), then it is the same for every molecule at that position. The "percent identity" between these two sequences is a function of the number of matching positions common to both sequences divided by the number of positions being compared x 100. For example, two sequences are 60% identical if 6 out of 10 positions match. For example, the DNA sequences CTGACT and CAGGTT are 50% identical (3 matches out of 6 total positions). Typically, this comparison is made when two sequences are aligned to yield maximum identity. Such an alignment can be achieved through methods, for example, in Needleman et al.(1970)J. Mol. Biol. 48:443-453, which can be conveniently implemented by a computer program such as the Align program (DNAstar, Inc.). The PAM 120 weighted residue table can also be used, using the algorithm of E. Meyers and W. Miller (Comput. Appl Biosci., 4:11-17 (1988)) was integrated into the ALIGN program (version 2.0). A gap length weights of 12 and a gap weights of 4 are used to determine the percent identity between two amino acid sequences. Additionally, the algorithm of Needleman and Wunsch (J Mol Biol. 48:444-453 (1970)) integrated into the GAP program of the GCG software package (available at www.gcg.com) can be used to determine the percent identity between two amino acid sequences using the Blossum 62 matrix or the PAM250 matrix with gap weights of 16, 14, 12, 10, 8, 6, or 4 and length weights of 1, 2, 3, 4, 5, or 6.

As used herein, "cell" is understood to refer not only to a specific individual cell, but also to the progeny or potential progeny of that cell. Because certain modifications may occur in the progeny due to mutations or environmental influences, such progeny may in fact differ from the parent cells and yet be comprised within the scope of the terms herein.

As used herein, the terms "transduction" and "transfection" comprise methods known in the art of introducing DNA into a cell using infectious agents (such as viruses) or other means to express a protein or molecule of interest. In addition to viruses or virus-like reagents, there are also chemical-based transfection methods such as transfection methods using calcium phosphate, dendrimers, liposomes or cationic polymers (e.g., DEAE glucan or polyethylene imine); non-chemical methods such as electroporation, cell squeezing, sonoporation, optical transfection, impalefection, protoplast fusion, plasmid delivery or transposons; particle-based methods, such as the use of gene guns, magnetofection or magnet-assisted transfection, particle bombardment; and hybridization methods (such as nucleofection).

As used herein, the terms "transfected" , "transformed" , or "transduced" refer to the process of transferring or introducing exogenous nucleic acid into a host cell. A "transfected", "transformed" or "transduced" cell is a cell that has been transfected, transformed or transduced with an exogenous nucleic acid.

The term "in vivo" refers to inside of the organism from which the cells were obtained. "Ex vivo" or "in vitro" refers to outside of the organism from which the cells are obtained.

As used herein, "treatment/treating" is a method used to obtain beneficial or desired results, including clinical results. For the purposes of the present invention, beneficial or desired clinical results comprise, but are not limited to, one or more of the following: alleviating one or more symptoms caused by the disease, reducing the extent of the disease, stabilizing the disease (e.g., preventing or delaying the deterioration of diseases), preventing or delaying the spread of the disease (e.g. metastasis), preventing or delaying the recurrence of the disease, reducing the recurrence rate of the disease, delaying or slowing down the progression of the disease, improving the disease status, providing (partial or complete) remission of the disease, reducing the dose of one or more other drugs needed to treat the disease, slowing the progression of the disease, improving quality of life, and/or extending survival. "Treatment" also comprises reducing the pathological consequences of a disorder, condition or disease. The methods of the present invention contemplate any one or more of these aspects of treatment.

As used herein, the term "effective amount" refers to an amount of a compound or composition sufficient to treat a particular disorder, condition or disease (e.g., ameliorate, alleviate, reduce and/or delay one or more symptoms thereof). As is understood in the art, an "effective amount" may be administered in one or more dosage, that is, a single or multiple doses may be required to achieve the desired therapeutic endpoint.

"Subject", "individual" or "patient" are used interchangeably herein for therapeutic purposes and refer to any animal classified as a mammal, including humans, domestic and farm animals, and zoo, farm or pet animals such as dogs, horses, cats, cows. In some embodiments, the individual is a human individual.

It is to be understood that embodiments of the present invention described herein comprise embodiments "consisting of ... " and/or "essentially consisting of ... ". References herein to "about" a value or parameter comprise (and describe) variations to the value or parameter itself. For example, a description that refers to "about X" comprises description for "X".

As used herein, reference to "not" a value or parameter generally means and describes a value or parameter "other than...". For example, the method is not used to treat type X cancer, meaning that the method is used to treat cancer other than type X.

As used herein, the term "about X-Y" has the same meaning as "about X to about Y."

As used herein and in the appended claims, the singular forms "a" , "an" , and "the" comprise plural referents unless the context clearly dictates otherwise. It should also be noted that claims can be drafted to exclude any optional elements. This statement is therefore intended to serve as an antecedent basis for the use of exclusive terms such as "only" , "merely" , or the use of a "no" limitation in conjunction with the recitation of claim elements.

As used herein, the term "and/or" in words such as "A and/or B" is intended to comprise both A and B; A or B; A (alone); and B (alone). Likewise, as used herein, the term "and/or" in words such as "A, B and/or C" is intended to comprise each embodiment of: A, B and C; A, B or C; A or C; A or B; B or C; A and C; A and B; B and C; A (alone); B (alone); and C (alone).

### II. Cas12i nuclease and effector protein

### Engineered Cas12i nuclease

In some embodiments, an engineered Cas12i nuclease is provided; the engineered Cas12i nuclease comprises one or more (e.g., two, three, four or five) of the following mutations based on a reference Cas12i nuclease selected from:
(1) replacing amino acids interacting with the PAM in the reference Cas12i nuclease with positively charged amino acids (such as R, H or K);
(2) replacing amino acids involved in opening the double-stranded DNA in the reference Cas12i nuclease with amino acids with an aromatic ring (such as F, Y or W);
(3) replacing amino acids located in the RuvC domain and interacting with the single-stranded DNA substrate in the reference Cas12i nuclease with positively charged amino acids (such as R, H or K);
(4) replacing amino acids interacting with the DNA-RNA double helix in the reference Cas 12i nuclease with positively charged amino acids (such as R, H or K); and
(5) replacing one or more polar or positively charged amino acids interacting with the DNA-RNA double helix in the reference Cas12i nuclease with hydrophobic amino acids (such as A, V, I, L, M, F, Y, P, C or W).

In some embodiments, the reference Cas12i nuclease is a natural Cas12i nuclease, such as a wild-type Cas12i2 nuclease with an amino acid sequence as shown in SEQ ID NO. 1. In some embodiments, the reference Cas12i nuclease is a variant of Cas12i nuclease, such as a natural variant. In some embodiments, the reference Cas12i nuclease is an engineered Cas12i (e.g., Cas12i2 or Cas12i1) nuclease that does not comprise one or more mutations in (1)-(5) above. In some embodiments, the amino acid positions are defined by the corresponding amino acid position set forth in SEQ ID NO.1. In some embodiments, the amino acid positions are defined by the corresponding amino acid position of SEQ ID NO.1 for a wild-type Cas12i nuclease having a sequence different from SEQ ID NO.1.

As used herein, "the amino acid is at position X, wherein the amino acid positions are defined by the corresponding amino acid position of the wild-type Cas12i nuclease shown in SEQ ID NO. 1", or "the amino acid is at position X, wherein the amino acid positions are defined by the corresponding amino acid position of SEQ ID NO.1 for a wild-type Cas12i nuclease having a sequence different from SEQ ID NO.1" means that the amino acid residue is located at a certain position of the reference enzyme Cas12i, which corresponds to the X position of SEQ ID NO. 1, and the amino acid sequence of the reference enzyme Cas12i and the amino acid sequence of SEQ ID NO. 1 are aligned with each other based on sequence homology. For example, Fig. 7 shows a homology alignment of the amino acid sequences of CAS12i2 (SEQ ID NO. 1) and CAS12i1 (SEQ ID NO. 13). Those skilled in the art can use software commonly used in the art, such as Clustal Omega, to compare and align the sequence identity with the amino acid sequence of any reference Cas12i nuclease with SEQ ID NO. 1, and then obtain the amino acid position in the reference Cas12i nuclease corresponding to the amino acid position defined based on SEQ ID NO. 1 in the present application.

The present application provides methods for engineering enzymes by introducing amino acid mutations based on any one or a combination of the above five engineering principles, which results in to an increase in enzyme activity (such as DNA single-stranded DNA or double-stranded cleavage activity) in vitro and/or in vivo (for example, the gene cleavage efficiency increased by about 100 times), an increase in the number of identifiable PAMs (for example, as can be seen from Fig. 8, the wild-type enzyme can recognize one PAM, and based on the above amino acid mutations, CasXX enzyme can recognize at least 11 PAMs), and/or reduced off-target rate and improved specificity (for example, as can be seen from Table 18, HF-Cas can reduce the off-target rate by about 99% compared to CasXX). The engineered Cas12i nuclease comprises one or more specific mutations as described in sections 1)-6) below. In some embodiments, any one or more mutations described in the present application can be combined with existing Cas12i mutations (such as those mutations described in section 7) below) to provide engineered Cas12i nuclease with higher activity.

In some embodiments, an engineered Cas12i nuclease is provided; the engineered Cas12i nuclease comprises a mutation that replaces one or more amino acids interacting with the PAM in a reference Cas12i nuclease with positively charged amino acids. In some embodiments, an engineered Cas12i nuclease is provided; the engineered Cas12i nuclease comprises a mutation that replaces one or more amino acids involved in opening the double-stranded DNA in the reference Cas12i nuclease with amino acids with an aromatic ring. In some embodiments, an engineered Cas12i nuclease is provided; the engineered Cas12i nuclease comprises a mutation that replaces one or more amino acids located in the RuvC domain and interacting with the single-stranded DNA substrate in the reference Cas12i nuclease with positively charged amino acids. In some embodiments, an engineered Cas12i nuclease is provided; the engineered Cas12i nuclease comprises a mutation that replaces one or more amino acids interacting with the DNA-RNA double helix in the reference Cas12i nuclease with positively charged amino acids. In some embodiments, an engineered Cas12i nuclease is provided; the engineered Cas 12i nuclease comprises a mutation that replaces one or more polar or positively charged amino acids interacting with the DNA-RNA double helix in the reference Cas12i nuclease with hydrophobic amino acids.

In some embodiments, an engineered Cas12i nuclease is provided; the engineered Cas12i nuclease comprises: 1) a mutation that replaces one or more amino acids interacting with the PAM in the reference Cas12i nuclease with positively charged amino acids; and 2) a mutation that replaces one or more amino acids involved in opening the double-stranded DNA in the reference Cas12i nuclease with amino acids with an aromatic ring. In some embodiments, an engineered Cas12i nuclease is provided; the engineered Cas12i nuclease comprises: 1) a mutation that replaces one or more amino acids interacting with the PAM in the reference Cas12i nuclease with positively charged amino acids; and 2) a mutation that replaces one or more amino acids located in the RuvC domain and interacting with the single-stranded DNA substrate in the reference Cas12i nuclease with positively charged amino acids. In some embodiments, an engineered Cas12i nuclease is provided; the engineered Cas12i nuclease comprises: 1) a mutation that replaces one or more amino acids involved in opening the double-stranded DNA in the reference Cas12i nuclease with amino acids with an aromatic ring; and 2) a mutation that replaces one or more amino acids located in the RuvC domain and interacting with the single-stranded DNA substrate in the reference Cas12i nuclease with positively charged amino acids. In some embodiments, the engineered Cas12i nuclease further comprises a mutation that replaces one or more polar or positively charged amino acids interacting with the DNA-RNA double helix in the reference Cas12i nuclease with hydrophobic amino acids. In some embodiments, the engineered Cas12i nuclease further comprises one or more flexible region mutations (e.g., substitution with G, and/or insertion of one or two Gs thereafter) to increase the flexibility of the flexible region. In some embodiments, the engineered Cas12i enzyme with such modifications increases flexibility by at least about 10%, such as at least about 20%, 30%, 50%, 100%, 150%, 200%, 500%, 1000 % or greater flexibility.

In some embodiments, an engineered Cas12i nuclease is provided; the engineered Cas12i nuclease comprises: 1) a mutation that replaces one or more amino acids interacting with the PAM in the reference Cas12i nuclease with positively charged amino acids; and 2) a mutation that replaces one or more amino acids interacting with the DNA-RNA double helix in the reference Cas12i nuclease with positively charged amino acids. In some embodiments, an engineered Cas12i nuclease is provided; the engineered Cas12i nuclease comprises: 1) a mutation that replaces one or more amino acids involved in opening the double-stranded DNA in the reference Cas12i nuclease with amino acids with an aromatic ring; and 2) a mutation that replaces one or more amino acids interacting with the DNA-RNA double helix in the reference Cas12i nuclease with positively charged amino acids. In some embodiments, an engineered Cas12i nuclease is provided; the engineered Cas12i nuclease comprises: 1) a mutation that replaces one or more amino acids located in the RuvC domain and interacting with the single-stranded DNA substrate in the reference Cas12i nuclease with positively charged amino acids; and 2) a mutation that replaces one or more amino acids interacting with the DNA-RNA double helix in the reference Cas12i nuclease with positively charged amino acids. In some embodiments, the engineered Cas12i nuclease further comprises a mutation that replaces one or more polar or positively charged amino acids interacting with the DNA-RNA double helix in the reference Cas12i nuclease with hydrophobic amino acids. In some embodiments, the engineered Cas12i nuclease further comprises one or more flexible region mutations to increase the flexibility of the flexible region.

In some embodiments, an engineered Cas12i nuclease is provided; the engineered Cas12i nuclease comprises: 1) a mutation that replaces one or more amino acids interacting with the PAM in the reference Cas 12i nuclease with positively charged amino acids; 2) a mutation that replaces one or more amino acids involved in opening the double-stranded DNA in the reference Cas12i nuclease with amino acids with an aromatic ring; and 3) a mutation that replaces one or more amino acids located in the RuvC domain and interacting with the single-stranded DNA substrate in the reference Cas12i nuclease with positively charged amino acids. In some embodiments, an engineered Cas12i nuclease is provided; the engineered Cas12i nuclease comprises: 1) a mutation that replaces one or more amino acids interacting with the PAM in the reference Cas12i nuclease with positively charged amino acids; 2) a mutation that replaces one or more amino acids involved in opening the double-stranded DNA in the reference Cas12i nuclease with amino acids with an aromatic ring; and 3) a mutation that replaces one or more amino acids interacting with the DNA-RNA double helix in the reference Cas12i nuclease with positively charged amino acids. In some embodiments, an engineered Cas12i nuclease is provided; the engineered Cas12i nuclease comprises: 1) a mutation that replaces one or more amino acids interacting with the PAM in the reference Cas12i nuclease with positively charged amino acids; 2) a mutation that replaces one or more amino acids located in the RuvC domain and interacting with the single-stranded DNA substrate in the reference Cas12i nuclease with positively charged amino acids; and 3) a mutation that replaces one or more amino acids interacting with the DNA-RNA double helix in the reference Cas12i nuclease with positively charged amino acids. In some embodiments, an engineered Cas12i nuclease is provided; the engineered Cas12i nuclease comprises: 1) a mutation that replaces one or more amino acids involved in opening the double-stranded DNA in the reference Cas12i nuclease with amino acids with an aromatic ring; 2) a mutation that replaces one or more amino acids located in the RuvC domain and interacting with the single-stranded DNA substrate in the reference Cas12i nuclease with positively charged amino acids; and 3) a mutation that replaces one or more amino acids interacting with the DNA-RNA double helix in the reference Cas12i nuclease with positively charged amino acids. In some embodiments, the engineered Cas12i nuclease further comprises a mutation that replaces one or more polar or positively charged amino acids interacting with the DNA-RNA double helix in the reference Cas12i nuclease with hydrophobic amino acids. In some embodiments, the engineered Cas12i nuclease further comprises one or more flexible region mutations to increase the flexibility of the flexible region.

In some embodiments, an engineered Cas12i nuclease is provided; the engineered Cas12i nuclease comprises: 1) a mutation that replaces one or more amino acids interacting with the PAM in the reference Cas 12i nuclease with positively charged amino acids; 2) a mutation that replaces one or more amino acids involved in opening the double-stranded DNA in the reference Cas12i nuclease with amino acids with an aromatic ring; 3) a mutation that replaces one or more amino acids located in the RuvC domain and interacting with the single-stranded DNA substrate in the reference Cas12i nuclease with positively charged amino acids; and 4) a mutation that replaces one or more amino acids interacting with the DNA-RNA double helix in the reference Cas12i nuclease with positively charged amino acids. In some embodiments, the engineered Cas12i nuclease further comprises a mutation that replaces one or more polar or positively charged amino acids interacting with the DNA-RNA double helix in the reference Cas12i nuclease with hydrophobic amino acids. In some embodiments, an engineered Cas12i nuclease is provided; the engineered Cas12i nuclease comprises: 1) a mutation that replaces one or more amino acids interacting with the PAM in the reference Cas12i nuclease with positively charged amino acids; 2) a mutation that replaces one or more amino acids involved in opening the double-stranded DNA in the reference Cas12i nuclease with amino acids with an aromatic ring; 3) a mutation that replaces one or more amino acids located in the RuvC domain and interacting with the single-stranded DNA substrate in the reference Cas 12i nuclease with positively charged amino acids; 4) a mutation that replaces one or more amino acids interacting with the DNA-RNA double helix in the reference Cas12i nuclease with positively charged amino acids; and 5) a mutation that replaces one or more polar or positively charged amino acids interacting with the DNA-RNA double helix in the reference Cas12i nuclease with hydrophobic amino acids. In some embodiments, the engineered Cas12i nuclease further comprises one or more flexible region mutations to increase the flexibility of the flexible region.

In some embodiments, the engineered Cas12i nuclease comprises mutations relative to the corresponding amino acid positions shown in SEQ ID NO. 1: N164Y+E176R+K238R+E323R+ D362R+T447R+E563R (hereinafter designated as "CasXX"). In some embodiments, the engineered Cas12i nuclease comprises the sequence of SEQ ID NO.8.

### 1) Replacing amino acids interacting with the PAM in the reference Cas12i nuclease with positively charged amino acids

In some embodiments, the engineered Cas12i nuclease comprises one or more mutations based on a reference Cas12i nuclease (e.g., Cas12i2), wherein the mutation involves replacing the amino acids interacting with the PAM in the reference Cas12i nuclease with positively charged amino acids (such as R, H, or K). In some embodiments, the engineered Cas12i nuclease comprises substitutions of one, two, three, four, five, six, seven, eight, or more of the amino acid residues.

In some embodiments, the amino acid that interacts with the PAM is an amino acid located within a distance of 9 angstroms from the PAM in three-dimensional structure. For example, it can be: an amino acid located within a distance of 9 angstroms from the PAM in three-dimensional structure, an amino acid located within a distance of 8 angstroms from the PAM in three-dimensional structure, an amino acid located within a distance of 7 angstroms from the PAM in three-dimensional structure, an amino acid located within a distance of 6 angstroms from the PAM in three-dimensional structure, an amino acid located within a distance of 5 angstroms from the PAM in three-dimensional structure, an amino acid located within a distance of 4 angstroms from the PAM in three-dimensional structure, an amino acid located within a distance of 3 angstroms from the PAM in three-dimensional structure, an amino acid located within a distance of 2 angstroms from the PAM in three-dimensional structure, or amino acids that are closer.

The spatial structural distance between the PAM and amino acids is defined by the distance between atoms in the resolved 3D structure (PDB file) of the Cas protein-RNA-DNA three-dimensional complex. The mutual distance between atoms can be displayed through PDB file recognition software. In some embodiments, the spatial structural distance between the PAM and amino acids is defined by the minimum distance between the amino acid residue and the atoms comprised by the nucleotide.

Programs that can be used to measure the spatial structural distance between the PAM and amino acids, or PDB file recognition software are well known in the art, including but not limited to PyMOL, ChimeraX, Swiss-pdbviewer, etc.

In some embodiments, one or more mutations that replace the amino acid interacting with the PAM in the reference Cas 12i nuclease with a positively charged amino acid are mutations of one or more amino acids at the following positions: 176, 178, 226, 227, 229, 237, 238, 264, 447 and 563. In some embodiments, the one or more mutations that replace the amino acid interacting with the PAM in the reference Cas12i nuclease with a positively charged amino acid are mutations in one or more of the following amino acids: E176, E178, Y226, A227, N229, E237, K238, K264, T447 and E563. In some embodiments, the one or more mutations that replace the amino acid interacting with the PAM in the reference Cas12i nuclease with a positively charged amino acid are mutations in one or more of the following amino acids: E176, K238, T447 and E563. In some embodiments, the mutation that replaces the amino acid interacting with the PAM in the reference Cas12i nuclease with a positively charged amino acid is located at amino acid residue No. 563, such as E563. In some embodiments, the amino acid positions are defined by the corresponding amino acid position of the wild-type Cas12i nuclease shown in SEQ ID NO. 1.

In some embodiments, the engineered Cas12i nuclease comprises a mutation in one or more of the following amino acids: E176R, E178R, Y226R, A227R, N229R, E237R, K238R, K264R, T447R and E563R, the amino acid positions are defined by the corresponding amino acid position of the wild-type Cas12i nuclease shown in SEQ ID NO.1.

In the context of this specification, E176 means; in the cited amino acid sequence (e.g., relative to SEQ ID NO. 1), amino acid No. 176 E (glutamic acid); here, common amino acids and their three letters and single-letter abbreviations are listed below: Alanine is abbreviated as Ala or A; Arginine is abbreviated as Arg or R; Aspartate is abbreviated as Asp or D; Cysteine is abbreviated as Cys or C; Glutamine is abbreviated as Gln or Q; Glutamic acid is abbreviated as Glu or E; Histidine is abbreviated as His or H; Isoleucine is abbreviated as Ile or I; Glycine is abbreviated as Gly or G; Asparagine is abbreviated as Asn or N; Leucine is abbreviated as Leu or L; Lysine is abbreviated as Lys or K; Methionine is abbreviated as Met or M; Phenylalanine is abbreviated as Phe or F; Proline is abbreviated as Pro or P; Serine is abbreviated as Ser or S; Threonine is abbreviated as Thr or T; Tryptophan is abbreviated as Trp or W; Tyrosine is abbreviated as Tyr or Y; Valine is abbreviated as Val or V.

In some embodiments, the mutation that replaces the amino acid interacting with the PAM in the reference Cas12i nuclease with a positively charged amino acid is to replace the corresponding amino acid residue in the reference Cas12i nuclease with R, H or K, such as R or K. In some embodiments, the mutation that replaces the amino acid interacting with the PAM in the reference Cas12i nuclease with a positively charged amino acid is to replace the corresponding amino acid residue in the reference Cas12i nuclease with R.

In some embodiments, the engineered Cas12i nuclease comprises one or more amino acid mutations at the following positions: 176R, 238R, 447R and 563R, wherein the amino acid positions are defined by the corresponding amino acid position of the wild-type Cas12i nuclease shown in SEQ ID NO.1. In some embodiments, the engineered Cas12i nuclease comprises one or more of the following mutations based on the reference Cas12i nuclease: E176, K238, T447 and E563; wherein the amino acid position numbers are defined by the corresponding amino acid positions shown in SEQ ID NO. 1. In some embodiments, the engineered Cas12i nuclease comprises one or more of the following mutations based on the reference Cas12i nuclease: E176R, K238R, T447R and E563R; wherein the amino acid position numbers are defined by the corresponding amino acid positions shown in SEQ ID NO. 1. In some embodiments, the engineered Cas12i nuclease comprises the E563R mutation; wherein the amino acid position numbers are defined by the corresponding amino acid positions shown in SEQ ID NO. 1. In some embodiments, for the purpose of improving gene editing efficiency, it is also possible to use engineered Cas12i nucleases having at least about 85% (e.g., any of at least about 86%, 87%, 88%, 89%, 90%, 91%, 92%, 93%, 94%, 95%, 96%, 97%, 98%, 99%) sequence identity with the aforementioned engineered Cas12i nucleases (replacing one or more amino acids interacting with the PAM in the reference Cas12i nuclease with a positively charged amino acids).

In the context of this specification, E176R means that in the cited amino acid sequence, amino acid No. 176, i.e., E, glutamic acid is replaced by R, arginine.

In some embodiments, the engineered Cas12i nuclease comprises any mutation or combination of mutations at the following amino acid residue positions: (i) 176, 238, 264, 447, 563, 176+238, 176+447, 176+563, 238+447, 238+563, 447+563, 176+238+447, 176+238+563, 176+447+563, 238+447+563, 176+238+447+563; wherein, amino acids position numbers are defined by the corresponding amino acid positions shown in SEQ ID NO.1. In some embodiments, said replacing one or more amino acids interacting with the PAM in the reference Cas12i nuclease with a positively charged amino acid comprises replacing it with R, H or K, such as R or K, preferably, such as R. In some embodiments, the engineered Cas12i nuclease comprises any mutation or combination of mutations in the following amino acid residues: In some embodiments, the engineered Cas12i nuclease comprises any one mutation in the following amino acid residues or mutation combination: E176, K238, E264, T447, E563, E176+K238, E176+T447, E176+E563, K238+T447, K238+E563, T447+E563, E176+K238+T447, E176+K238+E563, E176+T447+E563, K238+T447+E563, and E176+K238+T447+E563; wherein the amino acid position numbers are defined by the corresponding amino acid positions shown in SEQ ID NO. 1.

In some embodiments, the engineered Cas12i nuclease comprises any one of the following mutation/mutation combination: E176R, K238R, E264R, T447R, E563R, E176R+K238R, E176R+T447R, E176R+E563R, K238R+T447R, K238R+E563R, T447R+E563R, E176R+K238R+ T447R, E176R+K238R+E563R, E176R+T447R+E563R, K238R+T447R+E563R, and E176R+ K238R+T447R+E563R; wherein, the amino acid position numbers are defined by the corresponding amino acid positions shown in SEQ ID NO. 1. In some embodiments, the engineered Cas12i nuclease comprises any one of the following mutations/mutation combinations: E563R, E176R+ T447R, E176R+E563R, K238R+E563R, E176R+K238R+T447R, E176R+K238R+E563R, E176R+ T447R+E563R, and E176R+K238R+T447R+E563R; wherein the amino acid position numbers are defined by the corresponding amino acid positions shown in SEQ ID NO. 1. In some embodiments, the engineered Cas12i nuclease comprises a mutation combination of E176R+K238R+T447R+ E563R; wherein the amino acid position numbers are defined by the corresponding amino acid positions shown in SEQ ID NO. 1. For the purpose of improving gene editing efficiency, it is also possible to use engineered Cas12i nucleases having at least about 85% (e.g., any of at least about 86%, 87%, 88%, 89%, 90%, 91%, 92%, 93%, 94%, 95%, 96%, 97%, 98%, 99%) sequence identity with the aforementioned engineered Cas12i nucleases (replacing one or more amino acids interacting with the PAM in the reference Cas12i nuclease with a positively charged amino acids).

### 2) Replacing the amino acids involved in opening the double-stranded DNA in the reference Cas12i nuclease with amino acids with an aromatic ring

In some embodiments, the engineered Cas12i nuclease comprises one or more mutations based on a reference Cas12i nuclease (e.g., Cas12i2), wherein the mutation involves replacing one or more amino acids involved in opening the double-stranded DNA in the reference Cas12i nuclease with amino acids with an aromatic ring (such as F, Y, or W). In some embodiments, the engineered Cas12i nuclease comprises one, two, three, four, five, six, or more substitutions of the amino acid residues.

Wherein, the one or more amino acids involved in opening the double-stranded DNA are amino acids interacting with the last base pair at the 3' end in PAM relative to the target strand. For example, the PAM sequence recognized by Cas12i2 is the 5'-NTTN-3' base pair, wherein the base pair formed by the N base at the 3' end of PAM sequence and the target strand is "the last base pair at the 3' end in PAM relative to the target strand" as described herein, and after this base pair is the sequence of the targeting site.

In some embodiments, the one or more amino acids involved in opening the double-stranded DNA are located at the following positions: 163 and/or 164; wherein the amino acid position numbers are defined by the corresponding amino acid positions shown in SEQ ID NO. 1. In some embodiments, the one or more amino acids involved in opening the double-stranded DNA are one or more of the following amino acids: Q163, N164; wherein the amino acid position numbers are defined by the corresponding amino acid positions shown in SEQ ID NO. 1. In some embodiments, the amino acid involved in opening the double-stranded DNA is N164; wherein the amino acid position numbers are defined by the corresponding amino acid positions shown in SEQ ID NO. 1.

In some embodiments, the amino acid involved in opening the double-stranded DNA is replaced with F, Y or W. In some embodiments, the amino acid involved in opening the double-stranded DNA is replaced with F. In some embodiments, the amino acid involved in opening the double strands of DNA is replaced with Y.

In some embodiments, the engineered Cas12i nuclease comprises mutations in any one or more of the following amino acid residues: 163F, 163Y, 163W, 164W, 164F or 164Y; wherein the amino acid position numbers are defined by the corresponding amino acid positions shown in SEQ ID NO. 1. In some embodiments, the engineered Cas12i nuclease comprises mutations in any one or more of the following amino acid residues: 163F, 163Y, 163W, 164F or 164Y; wherein the amino acid position numbers are defined by the corresponding amino acid positions shown in SEQ ID NO. 1.

In some embodiments, the engineered Cas12i nuclease comprises any one of the following mutations: Q163 and/or N164; wherein the amino acid position numbers are defined by the corresponding amino acid positions shown in SEQ ID NO. 1. In some embodiments, the engineered Cas12i nuclease comprises any one of the following mutations: Q163F, Q163Y, Q163W, N164W, N164F or N164Y; wherein the amino acid position numbers are defined by the corresponding amino acid positions shown in SEQ ID NO. 1. In some embodiments, the engineered Cas12i nuclease comprises any one of the following mutations: Q163F, Q163Y, Q163W, N164F or N164Y; wherein the amino acid position numbers are defined by the corresponding amino acid positions shown in SEQ ID NO. 1. In some embodiments, the engineered Cas12i nuclease comprises the N164Y or N164F mutation; wherein the amino acid position numbers are defined by the corresponding amino acid positions shown in SEQ ID NO. 1. In some embodiments, the engineered Cas12i nuclease comprises the N164Y mutation; wherein the amino acid position numbers are defined by the corresponding amino acid positions shown in SEQ ID NO. 1. In some embodiments, for the purpose of improving gene editing efficiency, it is also possible to use engineered Cas12i nucleases having at least about 85% (e.g., any of at least about 86%, 87%, 88%, 89%, 90%, 91%, 92%, 93%, 94%, 95%, 96%, 97%, 98%, 99%) sequence identity with the aforementioned engineered Cas12i nucleases (replacing one or more amino acids involved in opening the double-stranded DNA in the reference Cas12i nuclease with amino acids with an aromatic ring).

### 3) Replacing the amino acids located in the RuvC domain and interacting with the single-stranded DNA substrate in the reference Cas12i nuclease with positively charged amino acids

In some embodiments, the engineered Cas12i nuclease comprises one or more mutations based on a reference Cas12i nuclease (e.g., Cas12i2), wherein, the mutation involves replacing the amino acids located in the RuvC domain and interacting with a single stranded DNA substrate in the reference Cas12i enzyme with positively charged amino acids such as R, H, or K. In some embodiments, the engineered Cas12i enzyme comprises substitutions of one, two, three, four, five, six, or more amino acid residues.

Wherein, the one or more amino acids located in the RuvC domain and interacting with the single-stranded DNA substrate are amino acids located within a distance of 9 angstroms from the PAM in three-dimensional structure, for example, they can be: amino acid located within a distance of 8 angstroms from the PAM in three-dimensional structure, amino acid located within a distance of 7 angstroms from the PAM in three-dimensional structure, amino acid located within a distance of 6 angstroms from the PAM in three-dimensional structure, amino acid located within a distance of 5 angstroms from the PAM in three-dimensional structure, amino acid located within a distance of 4 angstroms from the PAM in three-dimensional structure, amino acid located within a distance of 3 angstroms from the PAM in three-dimensional structure, amino acid located within a distance of 2 angstroms from the PAM in three-dimensional structure, amino acids that are closer.

RuvC domain is an enzymatic domain of the Cas12i protein responsible for cutting single-stranded DNA or double-stranded DNA. In the primary sequence of the protein, the RuvC domain of Cas12i is divided into three parts: RuvC-1, RuvC-2 and RuvC-3. These three parts are adjacent in three-dimensional structure and together form a catalytic pocket with enzymatic activity. The three-dimensional crystal structure of Cas12i2, its domain composition, and description of its interaction with DNA substrates can be found in Huang X. et al., Nature Communications, 11, Article number: 5241 (2020). The three-dimensional crystal structure of Cas12i1, its domain composition, and description of its interaction with DNA substrates can be found in Zhang H. et al. Nature Structural & Molecular Biology 27, 1069-1076 (2020). By comparing and modeling (homology modeling) homologous structures, a three-dimensional structural model of the interaction between reference Cas12i and substrate can be obtained through known Cas12i three-dimensional crystal structure. Example 3 describes a modeling approach to obtain the amino acids in Cas12i2 that are located in the RuvC domain and are located within a distance of 9 angstroms from the single-stranded DNA substrate.

In some embodiments, the spatial structural distance between the amino acids in the RuvC domain and the single-stranded DNA substrate can be defined by the distance between atoms in the resolved 3D structure (PDB file) of the Cas protein-RNA-DNA three-dimensional complex, the mutual distance between atoms can be displayed by PDB file recognition software. In some embodiments, the spatial structural distance between an amino acid in the RuvC domain and the single-stranded DNA substrate is defined by the minimum distance between the amino acid residue and the atoms comprised by the nucleotide. Programs that can be used to measure the spatial structural distance between amino acids in the RuvC domain and single-stranded DNA substrates, or PDB file recognition software are well known in the art, including but not limited to PyMOL, ChimeraX, Swiss-pdbviewer, etc.

In some embodiments, the one or more amino acids located in the RuvC domain and interacting with the single-stranded DNA substrate are one or more amino acids at the following positions: 323, 327, 355, 359, 360, 361, 362, 388, 390, 391, 392, 393, 414, 417, 418, 421, 424, 425, 650, 652, 653, 696, 705, 708, 709, 751, 752, 755, 840, 848, 851, 856, 885, 897, 925, 926, 928, 929, 932, and 1022. In some embodiments, the one or more amino acids located in the RuvC domain and interacting with the single-stranded DNA substrate are one or more of the following amino acids: E323, L327, V355, G359, G360, K361, D362, L388, N390, N391, F392, K393, Q414, L417, L418, K421, Q424, Q425, S650, E652, G653, I696, K705, K708, E709, L751, S752, E755, N840, N848, S851, A856, Q885, M897, N925, I926, T928, G929, Y932, and A1022. In some embodiments, the one or more amino acids located in the RuvC domain and interacting with the single-stranded DNA substrate are one or more of the following amino acids: E323, D362, L388, N391, L417, Q424, Q425, N925, I926, and G929. In some embodiments, the amino acid positions are defined by the corresponding amino acid position of the wild-type Cas12i nuclease shown in SEQ ID NO. 1.

In some embodiments, the engineered Cas12i nuclease comprises a mutation that replaces one or more amino acids located in the RuvC domain and interacting with the single-stranded DNA substrate in the reference Cas12i nuclease with R, H or K (e.g. R or K). In some embodiments, the engineered Cas12i nuclease comprises a mutation that replaces one or more amino acids located in the RuvC domain and interacting with the single-stranded DNA substrate in the reference Cas12i nuclease with R.

In some embodiments, the engineered Cas12i nuclease comprises one or more of the following amino acid mutations or mutation combinations: N390R, N391R, F392R, L751R, E755R, N840R, N848R, S851R, A856R, Q885R, M897R, I926R , G929R, Y932R, E323R, L327R, V355R, G359R, G360R, K361R, D362R, Q414R, K421R, Q425R, S650R, E652R, K705R, K708R, E709R, S752R, N925R, T928R, E323R+D362R, E323R+Q425R, E323R+I926R, Q425R+I926R, E323R+D362R+ Q425R, E323R+D362R+I926R, E323R+Q425R+I926R, E323R+D362R+Q425R+I926R, D362R+ I926R, N925R+I926R, D362R+N925R+I926R, D362R+N925R; the amino acid positions are defined by the corresponding amino acid position of the wild-type Cas12i nuclease shown in SEQ ID NO. 1.

In some embodiments, the engineered Cas12i nuclease comprises a mutation or combination of mutations at any of the following amino acid residue positions: E323, D362, Q425, N925, I926 and G929; wherein the amino acid position numbers are defined by the corresponding amino acid positions shown in SEQ ID NO. 1. In some embodiments, the engineered Cas12i nuclease comprises a mutation or combination of mutations at any of the following amino acid residue positions: E323, D362, Q425, N925, I926, E323+D362, E323+Q425, E323+I926, D362+Q425, D362+N925, D362+I926, Q425+I926, N925+I926, E323+D362+Q425, E323+D362+I926, E323+Q425+I926, D362+N925+I926, D362+Q425+I926, E323+D362+Q425+I926; wherein the amino acid position numbers are defined by the corresponding amino acid positions shown in SEQ ID NO. 1. In some embodiments, the mutation is a mutation that replaces the amino acid residue at the position with R, H, or K (e.g., R). In some embodiments, the engineered Cas12i nuclease comprises any of the following amino acids or combinations of amino acids: 323R, 362R, 425R, 925R, 926R, 323R+362R, 323R+425R, 323R+926R, 362R+425R, 362R +926R, 425R+926R, 925R+926R, 323R+362R+425R, 323R+362R+926R, 323R+425R+926R, 362R+925R+926R, 323R++362R+425R+926R, and 362R+ 425R+926R; wherein, the amino acid position numbers are defined by the corresponding amino acid positions shown in SEQ ID NO. 1. In some embodiments, the engineered Cas12i nuclease comprises any of the following mutations or mutation combinations: E323R, D362R, Q424R, Q425R, N925R, I926R, and G929R; wherein the amino acid position numbers are defined by the corresponding amino acid positions shown in SEQ ID NO. 1. In some embodiments, the engineered Cas12i nuclease comprises any of the following mutations or mutation combinations: E323R, D362R, Q425R, N925R, I926R, E323R+D362R, E323R+Q425R, E323R+I926R, D362R+Q425R, Q425R+I926R, D362R+I926R, N925R+I926R, E323R+D362R+Q425R, E323R+D362R+I926R, E323R+Q425R+ I926R, D362R+N925R+I926R, D362R+Q425R+I926R, and E323R+D362R+Q425R+I926R; wherein, the amino acid position numbers are defined by the corresponding amino acid positions shown in SEQ ID NO. 1. In some embodiments, the engineered Cas12i nuclease comprises the I926R mutation; wherein the amino acid position numbers are defined by the corresponding amino acid positions shown in SEQ ID NO. 1. In some embodiments, the engineered Cas12i nuclease comprises the E323R+D362R mutation; wherein the amino acid position numbers are defined by the corresponding amino acid positions shown in SEQ ID NO. 1. In some embodiments, for the purpose of improving gene editing efficiency, it is also possible to use engineered Cas12i nucleases having at least about 85% (e.g., any of at least about 86%, 87%, 88%, 89%, 90%, 91%, 92%, 93%, 94%, 95%, 96%, 97%, 98%, 99%) sequence identity with the aforementioned engineered Cas12i nucleases (replacing one or more amino acids located in the RuvC domain of the reference Cas12i enzyme and interacting with a single stranded DNA substrate with positively charged amino acids).

### 4) Replacing one or more amino acids interacting with the DNA-RNA double helix in the reference Cas12i nuclease with positively charged amino acids

In some embodiments, the engineered Cas12i nuclease comprises one or more mutations based on a reference Cas12i nuclease (e.g., Cas12i2) wherein, the mutation involves replacing one or more amino acids interacting with the DNA-RNA double helix in the reference Cas12i nuclease with positively charged amino acids such as R, H, or K. In some embodiments, the engineered Cas12i enzyme comprises substitutions of one, two, three, four, five, six, or more amino acid residues.

Wherein, the one or more amino acids interacting with the DNA-RNA double helix are amino acids located within a distance of 9 angstroms from the DNA-RNA double helix in three-dimensional structure, for example, they can be: amino acids located within a distance of 8 angstroms from the DNA-RNA double helix in three-dimensional structure, amino acids located within a distance of 7 angstroms from the DNA-RNA double helix in three-dimensional structure, amino acids located within a distance of 6 angstroms from the DNA-RNA double helix in three-dimensional structure, amino acids located within a distance of 5 angstroms from the DNA-RNA double helix in three-dimensional structure, amino acids located within a distance of 4 angstroms from the DNA-RNA double helix in three-dimensional structure, amino acids located within a distance of 3 angstroms from the DNA-RNA double helix in three-dimensional structure, amino acids located within a distance of 2 angstroms from the DNA-RNA double helix in three-dimensional structure, or amino acids that are closer. Some Cas nucleases work as follows: Cas forms a complex with guide RNA (such as crRNA), wherein crRNA and targeting DNA pair with each other to form a DNA-RNA double helix, and interact with Cas nuclease to open the double-stranded targeting DNA and form an R-loop, allowing the active position of Cas to complete the cleavage of dsDNA. The description of three-dimensional crystal structure of Cas12i2, its domain composition, and its interaction with the DNA-RNA double helix can be found in Huang X. et al., Nature Communications, 11, Article number: 5241 (2020).

In some embodiments, the spatial structural distance between the DNA-RNA double helix and Cas amino acids can be defined by the minimum distance between the amino acid residues in the resolved 3D structure (PDB file) of the Cas protein-RNA-DNA three-dimensional complex and the atoms comprised by the nucleotide, the mutual distance between atoms can be displayed by PDB file recognition software. In some embodiments, the spatial structural distance between the Cas amino acid and the DNA-RNA double helix is defined by the distance between the assumed positions of the atoms. Programs that can be used to measure the spatial structural distance between Cas amino acids and DNA-RNA double helix, or PDB file recognition software are well known in the field, including but not limited to PyMOL, ChimeraX, Swiss-pdbviewer, etc.

In some embodiments, the one or more amino acids interacting with the DNA-RNA double helix are one or more amino acids at the following positions: 116, 117, 156, 159, 160, 161, 247, 293, 294, 297, 301, 305, 306, 308, 312, 313, 316, 319, 320, 343, 348, 349, 427, 433, 438, 441, 442, 679, 683, 691, 782, 783, 797, 800, 852, 853, 855, 861, 865, 957, 958. In some embodiments, the one or more amino acids interacting with the DNA-RNA double helix are one or more of the following amino acids: G116, E117, A156, T159, E160, S161, E247, G293, E294, N297, T301, I305, K306, T308, N312, F313, Q316, E319, Q320, E343, E348, E349, D427, K433, V438, N441, Q442, N679, E683, E691, D782, E783, E797, E800, M852, D853, L855, N861, Q865, S957, and D958. In some embodiments, the one or more amino acids interacting with the DNA-RNA double helix are one or more of the following amino acids: G116, E117, T159, S161, E319, E343, and D958. In some embodiments, the amino acid interacting with the DNA-RNA double helix is D958. In some embodiments, the amino acid positions are defined by the corresponding amino acid position of the wild-type Cas12i nuclease shown in SEQ ID NO. 1.

In some embodiments, the engineered Cas12i nuclease comprises a mutation that replaces one or more amino acids interacting with the DNA-RNA double helix in the reference Cas12i nuclease with R, H, or K (e.g., R or K). In some embodiments, the engineered Cas12i nuclease comprises a mutation that replaces one or more amino acids interacting with the DNA-RNA double helix in the reference Cas12i nuclease with R.

In some embodiments, the engineered Cas12i nuclease comprises one or more of the following amino acid mutations: G116R, E117R, A156R, T159R, S161R, T301R, I305R, K306R, T308R, N312R, F313R, D427R, K433R, V438R, N441R, Q442R, M852R, L855R, N861R, Q865R, E160R, Q316R, E319R, Q320R, E247R, E343R, E348R, E349R, N679R, E683R, E691R, D782R, E783R, E797R, E800R, D853R, S957R, D958R, G293R, E294R, and N297R; the amino acid positions are defined by the corresponding amino acid positions of the wild-type Cas12i nuclease shown in SEQ ID NO. 1.

In some embodiments, the engineered Cas12i nuclease comprises a mutation or combination of mutations at any one of the following amino acid residue positions: G116, E117, T159, S161, E319, E343, or D958; wherein the amino acid position numbers are defined by the corresponding amino acid positions shown in SEQ ID NO.1. In some embodiments, the mutation is a mutation that replaces the amino acid residue at the position with R, H, or K (e.g., R). In some embodiments, the engineered Cas12i nuclease comprises any of a mutation or mutation combinations of the following sites: 116R, 117R, 159R, 161R, 319R, 343R, or 958R; wherein the amino acid position numbers are defined by the corresponding amino acid positions shown in SEQ ID NO. 1. In some embodiments, the engineered Cas12i nuclease comprises any one of the following mutations or mutation combinations: G116R, E117R, T159R, S161R, E319R, E343R, or D958R; wherein the amino acid position numbers are defined by the corresponding amino acid positions shown in SEQ ID NO. 1. In some embodiments, the engineered Cas12i nuclease comprises the D958R mutation; wherein the amino acid position numbers are defined by the corresponding amino acid positions shown in SEQ ID NO. 1. In some embodiments, for the purpose of improving gene editing efficiency, it is also possible to use engineered Cas12i nucleases having at least about 85% (e.g., any one of at least about 86%, 87%, 88%, 89%, 90%, 91%, 92%, 93%, 94%, 95%, 96%, 97%, 98%, 99%) sequence identity with the engineered Cas12i nucleases.

### 5) Replacing one or more polar or positively charged amino acids interacting with the DNA-RNA double helix in the reference Cas12i nuclease with hydrophobic amino acids

In some embodiments, the engineered Cas12i nuclease comprises one or more mutations based on a reference Cas12i nuclease (e.g., Cas12i2) wherein, the mutation involves replacing one or more polar or positively charged amino acids interacting with the DNA-RNA double helix in the reference Cas12i nuclease with hydrophobic amino acids (such as A, V, I, L, M, F, Y, P, C, or W). The mutation (also referred to herein as "high specificity" or "HF" mutation) can reduce the off-target rate of Cas12i nuclease (i.e., increase specificity). In some embodiments, compared to CasXX, the off-target rate of CasXX-HF can be reduced by at least about 10%, 20%, 30%, 40%, 50%, 60%, 70%, 80%, 90%, 95%, 99% or more. In some embodiments, the engineered Cas12i enzyme comprises substitutions of one, two, three, four, five, six, or more amino acid residues.

Wherein, the one or more amino acids interacting with the DNA-RNA double helix are amino acids located within a distance of 9 angstroms from the DNA-RNA double helix in three-dimensional structure. For example, they can be: amino acids located within a distance of 8 angstroms from the DNA-RNA double helix in three-dimensional structure, amino acids located within a distance of 7 angstroms from the DNA-RNA double helix in three-dimensional structure, amino acids located within a distance of 6 angstroms from the DNA-RNA double helix in three-dimensional structure, amino acids located within a distance of 5 angstroms from the DNA-RNA double helix in three-dimensional structure, amino acids located within a distance of 4 angstroms from the DNA-RNA double helix in three-dimensional structure, amino acids located within a distance of 3 angstroms from the DNA-RNA double helix in three-dimensional structure, amino acids located within a distance of 2 angstroms from the DNA-RNA double helix in three-dimensional structure, or amino acids that are closer. The three-dimensional crystal structure of Cas12i2, its domain composition, and description of its interaction with the DNA-RNA double helix can be found in Huang X. et al., Nature Communications, 11, Article number: 5241 (2020).

In some embodiments, the one or more polar or positively charged amino acids interacting with the DNA-RNA double helix are one or more amino acids at the following positions: 119, 164, 297, 308, 309, 312, 346, 357, 394, 395, 402, 441, 433, 565, 715, 719, 766, 782, 807, 841, 844, 845, 848, 857, 861 and 865. In some embodiments, the one or more polar or positively charged amino acids interacting with the DNA-RNA double helix are one or more of the following amino acids: Y119, Y164, N297, T308, R309, N312, S346, H357, K394, E395, R402, N441, K433, S565, R715, R719, S766, D782, K807, N841, K844, K845, N848, R857, N861 and Q865. In some embodiments, the one or more polar or positively charged amino acids interacting with the DNA-RNA double helix are one or more of the following amino acids: R857, R861, K807, N848, R715, R719, K394, H357 and K844. In some embodiments, the one or more polar or positively charged amino acids interacting with the DNA-RNA double helix are one or more of the following amino acids: R857, R719, K394, and K844. Wherein, the above-mentioned amino acid positions are defined as the corresponding amino acid positions shown in SEQ ID NO. 1 or 8.

In some embodiments, the engineered Cas12i nuclease comprises one or more of the following mutations: S565A, N297A, Q865A, T308A, R309A, N312A, N441A, R857A, N861A, Y119F, K433A, K807A, N841A, N848A, K845A, D782A, R715A, R719A, S766A, K394A, H357A, K844A, E395A, S346A, R402A, Y164N; wherein the amino acid position numbers are defined by the corresponding amino acid positions shown in SEQ ID NO. 1 or 8.

In some embodiments, the engineered Cas12i nuclease comprises a mutation that replaces one or more polar or a positively charged amino acid located at 357, 394, 715, 719, 807, 844, 848, 857, and 861 in the reference Cas12i nuclease with a hydrophobic amino acid. In some embodiments, the hydrophobic amino acid is selected from the group consisting of A, V, L, I, P and F, such as A, V, L or I. In some embodiments, the engineered Cas12i nuclease comprises a mutation that replaces one or more polar or a positively charged amino acid located at H357, K394, R715, R719, K807, K844, N848, R857 and/or R861 in the reference Cas12i nuclease with A. Wherein, the amino acid position numbers are defined by the corresponding amino acid positions shown in SEQ ID NO. 1 or 8. In some embodiments, the engineered Cas12i nuclease comprises mutations in any of the following amino acids or combinations of amino acids: H357A, K394A, R715A, R719A, K807A, K844A, N848A, R857A, and R861A; wherein the amino acid position numbers is defined by the corresponding amino acid positions shown in SEQ ID NO. 1 or 8.

In some embodiments, the engineered Cas12i nuclease comprises a mutation or combination of mutations at any of the following amino acid residue positions: 857, 719, 394, or 844; wherein the amino acid position numbers are defined by the corresponding amino acid positions shown in SEQ ID NO. 1. In some embodiments, the mutation is a mutation that replaces the amino acid residue at the position with a hydrophobic amino acid (e.g., A). In some embodiments, the engineered Cas12i nuclease comprises mutations in any of the following amino acids or combinations of amino acids: R857, R719, K394, and K844; wherein the amino acid position numbers are defined by the corresponding amino acid positions shown in SEQ ID NO. 1 or 8. In some embodiments, the engineered Cas12i nuclease comprises a mutation or combination of mutations at any of the following amino acid residue positions: R857, R719, K394, K844, R719+K394, K394+K844, R857+K394, R719+K844, R857+R719, R857+K844, R719+K394+K844, R857+R719+K394, R857+K394+K844, R857+R719+K844, R857+R719+K394+K844; wherein, the amino acid position numbers are defined by the corresponding amino acid positions shown in SEQ ID NO. 1 or 8; the mutation is a mutation that replaces the amino acid residue at the position with a hydrophobic amino acid (such as A). In some embodiments, the engineered Cas12i nuclease comprises a mutation or combination of mutations at any of the following amino acid residue positions: R857A, R719A, K394A, K844A, R719A+K394A, K394A+K844A, R857A+K394A, R719A+K844A, R857A+ R719A, R857A+K844A, R719A+K394A+K844A, R857A+R719A+K394A, R857A+K394A+ K844A, R857A+R719A+K844A, R857A+R719A+K394A+K844A; where, the amino acid position numbers are defined by the corresponding amino acid positions shown in SEQ ID NO. 1 or 8. In some embodiments, the engineered Cas12i nuclease comprises any one of the following mutations or mutation combinations: R857A, R719A, K394A, or K844A; wherein the amino acid position numbers are defined by the corresponding amino acid positions shown in SEQ ID NO. 1 or 8. In some embodiments, the engineered Cas12i nuclease comprises the K844A mutation; wherein the amino acid position numbers are defined by the corresponding amino acid positions shown in SEQ ID NO. 1 or 8. In some embodiments, the engineered Cas12i nuclease comprises the R719A and K844A mutations; wherein the amino acid position number are defined by the corresponding amino acid positions shown in SEQ ID NO. 1 or 8. In some embodiments, the engineered Cas12i nuclease comprises R857A and K844A mutations; wherein the amino acid position numbers are defined by the corresponding amino acid positions shown in SEQ ID NO. 1 or 8. In some embodiments,

for the purpose of improving gene editing efficiency, it is also possible to use engineered Cas12i nucleases having at least about 85% (e.g., any one of at least about 86%, 87%, 88%, 89%, 90%, 91%, 92%, 93%, 94%, 95%, 96%, 97%, 98%, 99%) sequence identity with the aforementioned engineered Cas12i nuclease (replacing one or more polar or positively charged amino acids interacting with the DNA-RNA double helix in the reference Cas12i nuclease with hydrophobic amino acids).

### 6) Other mutations

Unless otherwise specified, the mutations described herein may comprise one or more: insertion, deletion, substitution, and may be a mutation of a single amino acid or multiple amino acids.

Any one or more mutations described in sections 1) to 5) may be combined with any one or more mutations known to increase Cas12i activity, such as target binding, double-stranded cleavage activity, nickase activity and/or gene editing activity. Exemplary mutations can be seen in the following literature, for example PCT/CN2020/0134249 and CN112195164A, which are incorporated herein by reference in their entirety. Any one or more mutations described in sections 1) to 5) may also be combined with any one or more mutations known to reduce Cas12i activity, such as target binding, double-stranded cleavage activity, nickase activity, and/or gene editing activity.

In some embodiments, the engineered Cas12i nuclease (such as an engineered Cas12i nuclease comprising one or more mutations in above sections 1) to 5)) also comprises one or more flexible region mutations, the mutation increases the flexibility of the flexible region (such as increasing by at least about any one of 10%, 20%, 50%, 60%, 70%, 80%, 90%, 1x, 1.1x, 1.2x, 1.5x, 2x, 3x, 4x, 5 x, 10 x, 20x, 50x, 100x or more) in the reference Cas12i nuclease (or the engineered Cas12i comprising any one or more mutations in sections (1)-(5) nucleases. The flexible region in the reference Cas12i nuclease can be determined using any method known in the art. In some embodiments, multiple flexible regions are determined based solely on the amino acid sequence of the reference Cas12i nuclease. In some embodiments, multiple flexible regions are determined based on structural information of the reference Cas12i nuclease, including, for example, secondary structure, crystal structure, NMR structure, and the like.

The method for engineering Cas12i nuclease flexible regions described herein comprises: (a) obtaining a plurality of engineered Cas12i nucleases, each engineered Cas12i nuclease comprising one or more mutations that increase the flexibility of the flexible region in one or more flexible regions of a reference Cas12i nuclease; and (b) selecting one or more engineered Cas12i nucleases from the plurality of engineered Cas12i nucleases, wherein said one or more engineered Cas12i nucleases have increased activity (e.g., target binding, double-strand cleavage activity, nickase activity, and/or gene editing activity) compared to the reference Cas12i nuclease. In some embodiments, the method further comprises determining one or more flexible regions in the reference Cas12i nuclease. In some embodiments, the method further comprises measuring the activity of the engineered Cas12i nuclease in eukaryotic cells, such as mammalian cells (e.g., human cells).

In some embodiments, a plurality of flexible regions is determined using a program selected from the group consisting of: PredyFlexy, FoldUnfold, PROFbval, Flexserv, FlexPred, DynaMine, and Disomine. In some embodiments, the one or more flexible regions are located at random curls. In some embodiments, the one or more flexible regions are located in the domain of the reference Cas12i nuclease interacting with DNA and/or RNA (or the engineered Cas12i nuclease comprising any one or more mutations in sections 1)-5)). In some embodiments, the flexible region is at least about 5 (e.g., 5) amino acids in length.

In some embodiments, the one or more mutations comprise the insertion of one or more (e.g., 2) glycine (G) residues in the flexible region. In some embodiments, the one or more G residues are inserted into N-terminal of a flexible amino acid residue in the flexible region, wherein the flexible amino acid residue is selected from the group consisting of: G, serine (S), aspartate amide (N), aspartic acid (D), histidine (H), methionine (M), threonine (T), glutamic acid (E), glutamine (Q), lysine (K), arginine (R), alanine (A) and proline (P). In some embodiments, the flexible amino acid residues are selected according to the following priority: G>S>N>D>H>M>T>E>Q>K>R>A>P. In some embodiments, the one or more mutations comprise replacing one or more non-G residues with one or more G residues.

In some embodiments, the one or more mutations comprise replacing a hydrophobic amino acid residue in the flexible region with a G residue, wherein the hydrophobic amino acid residue is selected from the group consisting of: leucine (L), isoleucine (I), valine (V), cysteine (C), tyrosine (Y), phenylalanine (F) and tryptophan (W).

In some embodiments, the activity is site specific nuclease activity. In some embodiments, the activity is gene editing activity in eukaryotic cells (e.g., human cells). In some embodiments, the gene editing efficiency is measured using a T7 endonuclease 1 (T7E1) assay, sequencing of target DNA, Tracking of Indels by Decomposition (TIDE) assay, or Indel Detection by Amplicon Analysis (IDAA) assay.

In some embodiments, the engineered Cas12i nuclease (e.g., the engineered Cas12i nuclease comprising any one or more mutations in sections 1)-5)) comprises one or more flexible region mutations, the flexible region mutation increases the flexibility of the flexible region in the reference Cas12i nuclease (such as Cas12i2 nuclease, or the engineered Cas12i nuclease comprising any one or more mutations in sections 1)-5)), the flexible region is selected from the group consisting of corresponding to the following regions: amino acid residues 228-232, amino acid residues 439-443, amino acid residues 478-482, amino acid residues 500-504, amino acid residues 775-779 and amino acid residues 925-929, wherein the amino acid residue numbers are defined by the corresponding amino acid positions shown in SEQ ID NO. 1. In some embodiments, the flexible region is selected from amino acid residues 439-443 or amino acid residues 925-929, wherein the amino acid residue numbers are defined by the corresponding amino acid positions shown in SEQ ID NO. 1. In some embodiments, the reference Cas12i enzyme is Cas12i2 (SEQ ID NO. 1). In some embodiments, one or more flexible region mutations comprise inserting one or more (e.g., 2) G residues into the flexible region. In some embodiments, the one or more G residues are inserted into the N-terminal of the flexible amino acid residue in the flexible region, wherein the flexible amino acid residue is selected from the group consisting of: G, S, N, D, H, M, T, E, Q, K, R, A and P. In some embodiments, the flexible amino acid residues are selected according to the following priority: G>S>N>D>H> M>T>E>Q>K>R>A>P. In some embodiments, the one or more flexible region mutations comprise replacing a hydrophobic amino acid residue in the flexible region with a G residue, wherein the hydrophobic amino acid residue is selected from the group consisting of: A, V, I, L, M, F, Y, P, C and W; preferably, selected from the group consisting of: L, I, V, C, Y, F and W.

In some embodiments, the flexible region mutations are located at 439 and/or 926. In some embodiments, they are one or more of the following amino acids: L439, I926. Wherein, the amino acid positions are defined by the corresponding amino acid positions shown in SEQ ID NO. 1.

In some embodiments, the engineered Cas12i nuclease (e.g., the engineered Cas12i nuclease comprising any one or more mutations in sections 1)-5)) comprises 926G and/or 439(L+G) mutations. In some embodiments, the engineered Cas 12i nuclease comprises one or more of the following flexible region mutations: I926G, L439(L+G), and L439(L+GG). In some embodiments, the engineered Cas12i nuclease comprises the I926G mutation. In some embodiments, the engineered Cas12i nuclease comprises the L439 (L+G) mutation. In some embodiments, the engineered Cas12i nuclease comprises the L439 (L+GG) mutation. Wherein, the amino acid residue number is based on SEQ ID NO. 1.

In the context of this specification, The meaning of L439 (L+G) is that in the cited amino acid sequence (such as SEQ ID NO: 1), a glycine (G) is inserted after amino acid number 439, leaving the original L sequence at position 439 unchanged; in the context of this application and the accompanying drawings, it is sometimes represented as 439G. The meaning of L439 (L+GG) is that in the cited amino acid sequence, two glycines (GG) are inserted after amino acid number 439, leaving the original L sequence at position 439 unchanged; in the context of this application and the accompanying drawings, it is sometimes represented as 439GG.

In some embodiments, the engineered Cas12i nuclease (e.g., the engineered Cas12i nuclease comprising any one or more mutations in sections 1)-5)) comprises mutations or mutation combinations at any of the following amino acid residues: 926, 439, 925+926, 362+925+926, 439+926, 323+362+926 (e.g., I926, L439, N925+I926, D362+N925+I926, L439+I926, E323+D362+I926); wherein the amino acid position numbers are defined by the corresponding amino acid positions shown in SEQ ID NO. 1. In some embodiments, the mutation located at amino acid position 323, 362, 925 or 926 is a mutation that replaces the amino acid residue at the position with R, H or K (such as R); wherein the amino acid position numbers are defined by the corresponding amino acid positions shown in SEQ ID NO: 1. In some embodiments, the mutation located at amino acid position 439 or 926 is a mutation that replaces the amino acid residue at the position with G or inserts G or GG after the amino acid residue; wherein, the amino acid position numbers are defined by the corresponding amino acid positions shown in SEQ ID NO. 1.

In some embodiments, the engineered Cas12i nuclease (e.g., the engineered Cas12i nuclease comprising any one or more mutations in sections 1)-5)) comprises mutations in any of the following amino acid residues or combinations of amino acid residues: 926G, 439(L+GG), 925R+926G, 362R +925R+926G, 439(L+GG)+926R, or 323R+362R+926G; wherein, the amino acid position numbers are defined by the corresponding amino acid positions shown in SEQ ID NO:1.

In some embodiments, the engineered Cas 12i nuclease comprises any one or combination of mutations: I926G, L439(L+GG), L439(L+GG)+I926R, N925R+I926G, D362R+N925R+I926G, E323R+D362R+I926G; wherein the amino acid position numbers are defined by the corresponding amino acid positions shown in SEQ ID NO. 1.

In some embodiments, for the purpose of improving gene editing efficiency, it is also possible to use engineered Cas12i nucleases having at least about 85% (e.g., any one of at least about 86%, 87%, 88%, 89%, 90%, 91%, 92%, 93%, 94%, 95%, 96%, 97%, 98%, 99%) sequence identity with the aforementioned engineered Cas12i nuclease (for example, the engineered Cas12i nuclease comprising any one or more mutations in sections 1)-5), and/or engineered Cas 12i nuclease comprising the flexible region mutations described above).

### 7) Combinatorial mutations

Engineered Cas12i nucleases obtained by using a combination of mutations described in sections 1)-6) of this specification and one or more amino acid substitutions/insertions in Tables 1 to 5, Table 9, Table 12, Table 14, Tables 16-18 are within the scope of protection claimed in the present application.

In some embodiments, the engineered Cas12i nuclease comprises a mutation or combination of mutations at any of the following amino acid residue positions: 164, 176, 238, 323, 357, 362, 394, 439, 447, 563, 715, 719, 807, 844, 848, 857, 861, 925, 926, 958, 176+238+447+563, 323+362, 176+238+447+563+164, 176+238+447+563+926, 176+238+447+563+323+362, 164+926, 164+ 323+362, 176+238+447+563+164+926, 176+238+447+563+164+323+362, 176+238+447+563+ 164+926+323+362, 176+238+447+563+164+323+362+926+439, 719+844, 857+844, 362+926, 925+926, 362+925+926, 439+926, 323+362+926; wherein the amino acid position numbers are defined by the corresponding amino acid positions shown in SEQ ID NO. 1. In some embodiments, the mutation at amino acid position 176, 238, 323, 362, 447, 563, 926, or 958 is a mutation that replaces the amino acid residue at the position with R, H, or K (such as R). In some embodiments, the mutation at amino acid position 164 is a mutation that replaces the amino acid residue at that position with Y or F (e.g., Y). In some embodiments, the mutation at amino acid position 439 or 926 is a mutation that replaces the amino acid residue at the position with G or inserts G or GG after the amino acid residue. In some embodiments, the mutation at amino acid position 357, 394, 715, 719, 807, 844, 848, 857, or 861 is a mutation that replaces the amino acid residue at the position with a hydrophobic amino acid (such as A). Wherein, the amino acid position numbers are defined by the corresponding amino acid positions shown in SEQ ID NO. 1.

In some embodiments, the engineered Cas12i nuclease comprises mutations in any of the following amino acid residues or combinations of amino acid residues:
N164, E176; K238; E323, D362, T447; E563, I926, I926, D958, L439, R857, N861, K807, N848, R715, R719, K394, H357, K844,
E176+K238+T447+E563, E323+D362, E176+K238+T447+E563+N164,
E176+K238+T447+E563+I926, E176+K238+T447+E563+E323+D362, N164+1926,
N164+E323+D362, E176+K238+T447+E563+N164+I926,
E176+K238+T447+E563+NI64+E323+D362,
E176+K238+T447+E563+N164+I926+E323+D362,
E176+K238+T447+E563+N164+E323+D362+I926,
E176+K238+T447+E563+N164+E323+D362+I926+L439,
E176+K238+T447+E563+N164+E323+D362+I926+L439;
E176+K238+T447+E563+N164+D958;
E176+K238+T447+E563+I926+D958;
E176+K238+T447+E563+E323+D362+D958;
N164+I926+D958;
N164+E323+D362+D958;
E176+K238+T447+E563+N164+D958;
E176+K238+T447+E563+N164+I926+D958;
E176+K238+T447+E563+N164+E323+D362+D958;
E176+K238+T447+E563+N164+I926+E323+D362+D958;
E176+K238+T447+E563+N164+E323+D362+I926+D958;
E176+K238+T447+E563+N164+E323+D362+I926+L439+D958;
E176+K238+T447+E563+N164+E323+D362+I926+L439+D958;
R719+K844;
R857+K844;
E176+K238+T447+E563+N164+E323+D362+R857;
E176+K238+T447+E563+N164+E323+D362+N861;
E176+K238+T447+E563+N164+E323+D362+K807;
E176+K238+T447+E563+N164+E323+D362+N848;
E176+K238+T447+E563+N164+E323+D362+R715;
E176+K238+T447+E563+N164+E323+D362+R719;
E176+K238+T447+E563+N164+E323+D362+K394;
E176+K23 8+T447+ES 63+N 164+E323+D3 62+H3 57;
E176+K238+T447+E563+N164+E323+D362+K844;
E176+K238+T447+E563+N164+E323+D362+R719+K844;
E176+K238+T447+E563+N164+E323+D362+R857+K844; or
E176+K238+T447+E563+N164+E323+D362+K394;
wherein, the amino acid position numbers are defined by the corresponding amino acid positions shown in SEQ ID NO. 1.

In some embodiments, the engineered Cas12i nuclease comprises any one or combination of the following mutations:
N164Y; E176R; K238R; E323R; D362R; T447R; E563R; I926R; D958R; L439(L+G);
L439(L+GG); R857A; N861A; K807A; N848A; R715A; R719A; K394A; H357A; K844A;
R719A+K844A; N164Y+I926R; E323R+D362R; R857A+K844A; R857A+K844A;
N164Y+I926R+D958R; N164Y+E323R+D362R; N164Y+E323R+D362R+D958R;
E176R+K238R+T447R+E563R; E176R+K238R+T447R+E563R+N164Y;
E176R+K238R+T447R+E563R+I926R; E176R+K238R+T447R+E563R+E323R+D362R;
E176R+K238R+T447R+E563R+N164Y+I926R;
E176R+K23 8R+T447R+E563R+N 164Y+E323R+D3 62R;
E176R+K23 8R+T447R+E563R+N 164Y+I926R+E323R+D3 62R;
E176R+K23 8R+T447R+E563R+N 164Y+E323R+D3 62R+I926G;
E176R+K238R+T447R+E563R+N164Y+E323R+D362R+I926G+L439(L+GG);
E176R+K238R+T447R+E563R+N164Y+E323R+D362R+I926G+L439(L+G);
E176R+K23 8R+T447R+E563R+N 164Y+D95 8R;
E176R+K238R+T447R+E563R+I926R+D958R;
E176R+K238R+T447R+E563R+E323R+D362R+D958R;
E176R+K23 8R+T447R+E563R+N 164Y+D95 8R;
E176R+K23 8R+T447R+E563R+N 164Y+I926R+D95 8R;
E176R+K238R+T447R+E563R+N164Y+E323R+D362R+D958R;
E176R+K238R+T447R+E563R+N164Y+I926R+E323R+D362R+D958R;
E176R+K238R+T447R+E563R+N164Y+E323R+D362R+I926G+D958R;
E176R+K238R+T447R+E563R+N164Y+E323R+D362R+I926G+L439(L+GG)+D958R;
E176R+K238R+T447R+E563R+N164Y+E323R+D362R+I926G+L439(L+G)+D958R;
E176R+K238R+T447R+E563R+N164Y+E323R+D362R+R857A;
E176R+K238R+T447R+E563R+N164Y+E323R+D3 62R+N861A;
E176R+K238R+T447R+E563R+N164Y+E323R+D362R+K807A;
E176R+K238R+T447R+E563R+N164Y+E323R+D362R+N848A;
E176R+K238R+T447R+E563R+N164Y+E323R+D362R+R715A;
E176R+K238R+T447R+E563R+N164Y+E323R+D362R+R719A;
E176R+K23SR+T447R+E563R+N164Y+E323R+D362R+K394A;
E176R+K238R+T447R+E563R+N164Y+E323R+D362R+H357A;
E176R+K238R+T447R+E563R+N164Y+E323R+D362R+K844A;
E176R+K238R+T447R+E563R+N164Y+E323R+D362R+R719A+K844A;
E176R+K238R+T447R+E563R+N164Y+E323R+D362R+R857A+K844A; or
E176R+K238R+T447R+E563R+N164Y+E323R+D362R+K3 94A;
wherein, the amino acid position numbers are defined by the corresponding amino acid positions shown in SEQ ID NO. 1.

In some embodiments, the engineered Cas12i nuclease comprises the following combination of mutations: E176R+K238R+T447R+E563R+N164Y+E323R+D362R, wherein the amino acid position numbers are defined by the corresponding amino acid positions shown in SEQ ID NO. 1. This mutant is named CasXX below, and its sequence number is SEQ ID NO. 8.

In some embodiments, the engineered Cas12i nuclease comprises any one of the following mutation combinations:
E176R+K238R+T447R+E563R+N164Y+E323R+D362R+R857A;
E176R+K238R+T447R+E563R+N164Y+E323R+D362R+R719A;
E176R+K238R+T447R+E563R+N164Y+E323R+D362R+K394A;
E176R+K238R+T447R+E563R+N164Y+E323R+D362R+K844A;
E176R+K238R+T447R+E563R+N164Y+E323R+D362R+R719A+K844A;
E176R+K238R+T447R+E563R+N164Y+E323R+D362R+R857A+K844A; wherein the amino acid position numbers are defined by the corresponding amino acid positions shown in SEQ ID NO. 1. These Cas12i mutants are optimized mutants obtained by further optimization based on CasXX (SEQ ID NO. 8).

In some embodiments, the engineered Cas12i nuclease comprises the following combination of mutations: E176R+K238R+T447R+E563R+N164Y+E323R+D362R+K394A, wherein the amino acid position numbers are defined by the corresponding amino acid positions shown in SEQ ID NO. 1. This mutant is named "HF-20" below, and its sequence is shown in SEQ ID NO. 20.

In some embodiments, the engineered Cas12i nuclease comprises E176R, K238R, T447R, E563R, N164Y, E323R and D362R mutations. In some embodiments, the engineered Cas12i nuclease comprises E176R, K238R, T447R, E563R, N164Y and I926R mutations. In some embodiments, the engineered Cas12i nuclease comprises E176R, K238R, T447R, E563R, E323R and D362R mutations. In some embodiments, the engineered Cas12i nuclease comprises E176R, K238R, T447R, E563R, N164Y, E323R, D362R and I926G mutations. In some embodiments, the engineered Cas12i nuclease comprises E176R, K238R, T447R, E563R, N164Y, E323R, D362R, I926G and L439 (L+GG) mutations. In some embodiments, the engineered Cas12i nuclease comprises E176R, K238R, T447R, E563R, N164Y, E323R, D362R, I926G and L439(L+G) mutations. In some embodiments, the engineered Cas12i nuclease comprises E176R, K238R, T447R, E563R, N164Y and D958R mutations. In some embodiments, the engineered Cas12i nuclease comprises E176R, K238R, T447R, E563R, N164Y, I926R and D958R mutations. In some embodiments, the engineered Cas12i nuclease comprises E176R, K238R, T447R, E563R, N164Y, E323R, D362R and D958R mutations. In some embodiments, the engineered Cas12i nuclease comprises E176R, K238R, T447R, E563R, N164Y, I926R, E323R, D362R and D958R mutations. In some embodiments, the engineered Cas12i nuclease comprises E176R, K238R, T447R, E563R, N164Y, E323R, D362R, I926G, and D958R mutations. In some embodiments, the engineered Cas12i nuclease comprises E176R, K238R, T447R, E563R, N164Y, E323R, D362R, I926G, L439(L+GG), and D958R mutations. In some embodiments, the engineered Cas12i nuclease comprises E176R, K238R, T447R, E563R, N164Y, E323R, D362R, and K844A mutations. In some embodiments, the engineered Cas12i nuclease comprises E176R, K238R, T447R, E563R, N164Y, E323R, D362R, R719A, and K844A mutations. In some embodiments, the engineered Cas12i nuclease comprises E176R, K238R, T447R, E563R, N164Y, E323R, D362R, R857A, and K844A mutations. Wherein, the amino acid position numbers are defined by the corresponding amino acid positions shown in SEQ ID NO. 1.

In some embodiments, for the purpose of improving gene editing efficiency, it is also possible to use engineered Cas12i nucleases having at least about 80% (e.g., any one of at least about 81%, 82%, 83%, 84%, 85%, 86%, 87%, 88%, 89%, 90%, 91%, 92%, 93%, 94%, 95%, 96%, 97%, 98%, 99%) sequence identity with the aforementioned engineered Cas12i nuclease.

In some embodiments, an engineered Cas12i nuclease is provided, comprising any amino acid sequence shown in SEQ ID NOs. 2-24, or an amino acid sequence having at least about 80% (e.g., any one of at least about 81%, 82%, 83%, 84%, 85%, 86%, 87%, 88%, 89%, 90%, 91%, 92%, 93%, 94%, 95%, 96%, 97%, 98%, 99%) sequence identity with any amino acid sequence shown in SEQ ID NOs. 2-24.

### Reference Cas12i nuclease

In some embodiments, the reference Cas12i nuclease is Cas12i1, Cas12i2, or an ortholog thereof. In some embodiments, the reference Cas12i nuclease is native Cas12i1, or a variant thereof (e.g., a naturally occurring variant). In some embodiments, the reference Cas12i nuclease is native Cas12i2 (as set forth in SEQ ID NO. 1), or a variant thereof (e.g., a naturally occurring variant). In some embodiments, the reference Cas12i nuclease is an engineered Cas12i nuclease (an engineered Cas12i comprising any one or more mutations in sections 1)-5) as described in the present invention). nuclease). In some embodiments, the reference Cas12i nuclease is CasXX (SEQ ID NO.8).

Type V-I CRISPR-Cas 12i has been identified as an RNA-guided DNA endonuclease system. Unlike CRISPR-Cas systems such as Cas12b or Cas9, Cas12i-based CRISPR systems do not require tracrRNA sequences. In some embodiments, the RNA guide sequence comprises crRNA. Generally, crRNA as described herein comprises direct repeat sequences and spacer sequences. In certain embodiments, the crRNA comprises, consists essentially of, or consists of a direct repeat sequence linked to a guide sequence or spacer sequence. In some embodiments, the crRNA comprises a direct repeat, spacer, and direct repeat (DR-spacer-DR) sequence, which is a typical feature of precursor crRNA (pre-crRNA) configuration in other CRISPR systems . In some embodiments, the crRNA comprises truncated direct repeat and spacer sequences, which is a typical feature of processed or mature crRNA. In some embodiments, the CRISPR-Cas12i effector protein forms a complex with an RNA guide sequence, and the spacer sequence directs the complex to sequence-specific binding to a target nucleic acid that is complementary (e.g. at least 70% complementary) to the spacer sequence.

In some embodiments, the engineered Cas12i of the present application is an endonuclease that binds to a specific site of a target sequence and cleaves under the guidance of a guide RNA, and has both DNA and RNA endonuclease activities. In some embodiments, the Cas12i is capable of autonomous crRNA biogenesis by processing precursor crRNA arrays. Autonomous precursor crRNA processing facilitates the delivery of Cas12i, enabling dual-nicking applications since two separate genomic positions can be targeted by a single crRNA transcript. The Cas 12i protein then processes the CRISPR array into two homologous crRNAs, forming a paired nicking complex. Multiplexing of type V-I (Cas12i) effector proteins is accomplished by utilizing the precursor crRNA processing capabilities of the effector proteins, wherein multiple targets with different sequences can be programmed on a single RNA guide sequence. In this way, multiple genes or DNA targets can be manipulated simultaneously for therapeutic applications. In some embodiments, the guide RNA comprises a precursor crRNA expressed from a CRISPR array consisting of target sequences interleaved with unprocessed DR sequences, repeated by intrinsic precursor crRNA processing of the effector protein to enable simultaneous targeting of one, two or multiple sites.

Cas12i nucleases from various organisms can be used as the reference Cas12i nucleases to provide engineered Cas12i nucleases and effector proteins of the present application. Exemplary Cas12i nucleases have been described, for example, in WO2019/201331A1 and US2020/0063126A1, which are incorporated herein by reference in their entirety. In some embodiments, the reference Cas12i nuclease has enzymatic activity. In some embodiments, the reference Cas12i is a nuclease that cleaves both strands of a target duplex nucleic acid (e.g., duplex DNA). In some embodiments, the reference Cas12i is a nickase that cleaves a single strand of a target duplex nucleic acid (e.g., duplex DNA). In some embodiments, the reference Cas12i nuclease is enzymatically inactive. In some embodiments, the reference Cas12i nuclease is Cas12i1, Cas12i2, or Cas12i-Phi. In some embodiments, the reference Cas12i nuclease comprises the sequence of SEQ ID NO.1. Orthologs having a certain sequence identity (e.g., at least any of about 60%, 70%, 80%, 85%, 90%, 95%, 96%, 97%, 98%, 99%, or more) with Cas12i (such as Cas12i2) or its functional derivatives can be used as a basis for designing the engineered Cas12i nuclease or effector protein of the present application.

### Functional variants of engineered Cas12i nucleases

In some embodiments, the engineered Cas 12i nuclease is a functional variant (or functional derivative) based on a naturally occurring Cas12i nuclease. In some embodiments, when compared to the amino acid sequence of the corresponding engineered Cas12i nuclease (such as an engineered Cas12i nuclease comprising any one or more mutations in 1)-7) above), amino acid sequences of functional variants (or functional derivatives) have at least one different amino acid residue (e.g., have deletions, insertions, substitutions and/or fusions). In some embodiments, the functional variants have one or more mutations, such as amino acid substitutions, insertions and deletions. For example, compared with the wild-type naturally occurring Cas12i nuclease, or compared with the aforementioned engineered Cas12i nuclease, the functional variant may comprise substitutions of any one of 1, 2, 3, 4, 5, 6, 7, 8, 9, 10 or more amino acids. In some embodiments, the one or more substitutions are conservative substitutions. In some embodiments, the functional variant has all domains of a naturally occurring Cas12i nuclease. In some embodiments, the functional variant does not possess one or more domains of a naturally occurring Cas12i nuclease. In some embodiments, the functional variant has all domains of an engineered Cas12i nuclease. In some embodiments, the functional variant does not have one or more domains of the engineered Cas12i nuclease. In some embodiments, the biological activity of functional variants of Cas12i nuclease changes due to changes in their amino acids, e.g., the transformation from a natural nuclease to an enzyme inactivated mutant.

For any of the Cas12i variant proteins described herein (e.g., nickase Cas12i protein, inactive or catalytically inactive Cas12i (dCas12i)), the Cas12i variant may comprise a Cas12i protein sequence having the same parameters described above (e.g., the presence of domain, percent identity, etc.).

In some embodiments, the functional variant of the engineered Cas12i nuclease has enzymatic activity (such as DNA double-stranded or single-strand cleavage activity), or at least have approximately 60% (such as at least about any of 65%, 70%, 80%, 90%, 95%, 96%, 97%, 98%, 99%, or 100%) enzyme activity of the reference Cas12i nuclease (or its parent engineered Cas12i nucleic acid enzyme). In some embodiments, the functional variant of the engineered Cas12i nuclease has an enzymatic activity that is at least 1.1 times that of its parent engineered Cas12i nuclease (such as at least 1.2 times, 1.5 times, 2 times, 3 times, 4 times, 5 times, 10 times or more).

In some embodiments, the functional variant of the engineered Cas12i nuclease has a different catalytic activity than a non-functional variant mutant form of the engineered Cas12i nuclease. In some embodiments, the functional variant mutations (e.g., amino acid substitutions, insertions, and/or deletions) are in the catalytic domain of the Cas12i nuclease (e.g., the RuvC domain). In some embodiments, the functional variants of the engineered Cas12i nuclease comprise mutations in one or more catalytic domains. A Cas12i nuclease that cleaves one strand of a double-stranded target nucleic acid without cleaving the other strand is referred to herein as a "nickase" (e.g., "Cas12i nickase"). Herein, a Cas12i nuclease that has essentially no nuclease activity is referred to as a deactivated Cas12i protein ("dCas12i") (to which a heterologous polypeptide (fusion partner) fused to can provide nuclease activity, see "engineered Cas12i effector protein" section below). In some embodiments, when the DNA cleavage activity of the functional variant mutant enzyme is less than about 25%, 10%, 5%, 1%, 0.1%, 0.01% or less relative to the mutated form of the non-functional variant thereof, the Cas12i nuclease functional variant is considered to lack substantially all DNA cleavage activity. Mutation of one or more amino acid residues in the active site of Cas12i nuclease will lead to Cas12i with reduced or lost enzymatic activity (dCas12i), which is also called "Cas12i with missing nuclease activity" or "enzyme inactivating mutant" in the present invention. In some embodiments, the engineered Cas12i nucleases provided herein can be modified to have reduced or deleted nuclease activity, e.g., compared to a wild-type Cas12i nuclease (or its parent engineered Cas12i nuclease), a Cas12i lacking nuclease activity (such as DNA double-stranded or single-stranded cleavage activity) is reduced by at least about 50% (such as at least any of about 60%, 70%, 80%, 90%, 95%, 96%, 97%, 98%, 99%, or 100%). The Cas12i nuclease activity can be reduced by several methods, for example, by introducing mutations into one or more domains of the Cas12i nuclease: the domain that interacts with PAM, the domain involved in opening double-stranded DNAs, RuvC domain, regions interacting with nucleic acids (DNA/RNA), etc. In some embodiments, the catalytic residue for Cas12i nuclease activity (e.g., the catalytic residue identified by any universal identification method) can be replaced with a different amino acid residue (e.g., glycine or alanine) to reduce the nuclease activity. Examples of such mutations for Cas12i1 (shown in SEQ ID NO. 13) comprise D647A, E894A and/or D948A. Examples of such mutations for Cas12i2 (shown in SEQ ID NO. 1) comprise D599A, E833A, S883A, H884A, D886A, R900A and/or D1019A. In some embodiments, the engineered Cas12i nuclease or functional derivative thereof (such as an engineered Cas12i nuclease comprising any one or more mutations in 1)-7) above) comprises one or more of the following nuclease activity deletion mutations: D599A, E833A, S883A, H884A, R900A and D1019A; wherein the amino acid positions are defined by the corresponding amino acid positions shown in SEQ ID NO. 1.

### Characteristics of engineered Cas12i nucleases or functional variants thereof

In some embodiments, the engineered Cas12i nuclease (or functional variant thereof) has increased activity compared to the reference Cas12i nuclease. In some embodiments, the engineered Cas12i nuclease (or functional variant thereof) has reduced activity compared to the reference Cas 12i nuclease. In some embodiments, the activity is target DNA binding activity. In some embodiments, the activity is site specific nuclease activity. In some embodiments, the activity is an activity that opens double-stranded DNAs. In some embodiments, the activity is double-stranded DNA cleavage activity. In some embodiments, the activity is single-stranded DNA cleavage activity, including, for example, site specific DNA cleavage activity or non-specific DNA cleavage activity. In some embodiments, the activity is single-stranded RNA cleavage activity, such as site specific RNA cleavage activity or non-specific RNA cleavage activity. In some embodiments, the activity is measured in vitro. In some embodiments, the activity is measured in cells, such as bacterial cells, plant cells, or eukaryotic cells.

In some embodiments, the activity is measured in mammalian cells, such as rodent cells or human cells. In some embodiments, the activity is measured in human cells such as 293T cells. In some embodiments, the activity is measured in mouse cells, such as Hepal-6 cells. In some embodiments, the engineered Cas12i nuclease (or functional variant thereof) has an increase in activity of any one of at least about 10%, 20%, 30%, 40%, 50%, 60%, 70%, 80%, 90%, 95%, 1 times, 1.1 times, 1.5 times, 2 times, 3 times, 4 times, 5 times, 10 times or more compared to a reference Cas12i nuclease (one or more activities as described above, such as site specific nuclease activity). In some embodiments, the engineered Cas12i nuclease (or functional variant thereof) has a reduction in activity of any one of at least about 10%, 20%, 30%, 40%, 50%, 60%, 70%, 80%, 90%, 95%, 1 times, 1.1 times, 1.5 times, 2 times, 3 times, 4 times, 5 times, 10 times or more compared to a reference Cas12i nuclease (one or more activities as described above, such as site specific nuclease activity). The site specific nuclease activity of the engineered Cas12i nuclease (or functional variant thereof) can be measured using methods known in the art, including, for example, gel shift assays, such as the in vitro cleavage assay based on agarose gel electrophoresis in the examples provided herein.

In some embodiments, the activity is gene editing activity in the cells. In some embodiments, the cells are bacterial cells, plant cells, or eukaryotic cells. In some embodiments, the cells are mammalian cells, such as rodent cells or human cells. In some embodiments, the cells are 293T cells. In some embodiments, the activity is measured in mouse cells, such as Hepal-6 cells. In some embodiments, the activity is formed by the insertion and deletion of target genomic sites in cells, such as site specific cleavage of target nucleic acids through the engineered Cas12i nuclease (or functional variant thereof) and DNA repair through nonhomologous end joining (NHEJ) mechanism. In some embodiments, the activity is the activity of inserting an exogenous nucleic acid sequence into a target genomic site in the cell, such as site specific cleavage of the target nucleic acid by the engineered Cas12i nuclease (or functional variant thereof) and DNA repair via homologous recombination (HR; by further introducing a repair template) mechanism. In some embodiments, compared to a reference Cas12i nuclease, the engineered Cas12i nuclease (or functional variant thereof) increases the gene editing (e.g., cleavage, indel formation, or repair) activity of any of at least about 10%, 20%, 30%, 40%, 50%, 60%, 70%, 80%, 90%, 95%, 1 times, 1.1 times, 1.5 times, 2 times, 3 times, 4 times, 5 times, 10 times or more at the target genomic site of the cell (e.g., a human cell such as a 293T cell, or a mouse Hepa1-6 cell).In some embodiments, compared to the reference Cas12i2 nuclease, the engineered Cas12i nuclease (or functional variant thereof) increases gene editing (e.g., cleavage, indel formation, or repair) activity by any of at least about 10%, 20%, 30%, 40%, 50%, 60%, 70%, 80%, 90%, 95%, 1 times, 1.1 times, 1.5 times, 2 times, 3 times, 4 times, 5 times, 10 times or more at multiple (e.g., 2, 3, 4, 5, 6, 7, 8, 9, 10 or more) target genomic sites in cells (e.g., human cells such as 293T cells or mouse Hepa1-6 cells). In some embodiments, compared to the reference Cas12i nuclease, the engineered Cas12i nuclease (or functional variant thereof) is capable of editing a greater number (e.g., 2, 3, 4, 5, 6, 7, 8, 9, 10, 20, 30, 40, 50, 60, 70 or more) of genomic sites, such as recognizing more the PAM sequences (such as 2, 3, 4, 5, 6, 7, 8, 9, 10, 20, 30, 40, 50, 60, 70 or more). In some embodiments, the consensus the PAM sequence of the engineered Cas12i nuclease (or functional variant thereof) is identical to that of the reference Cas12i nuclease.

The gene editing efficiency of engineered Cas12i nuclease (or functional variants thereof) in vitro or in cells can be determined using methods known in the art, including, for example, T7 endonuclease 1 (T7E1) assay, sequencing of target DNA (including for example, Sanger sequencing, as well as next-generation sequencing), tracking of indels by decomposition (TIDE) assays, or indel detection by amplicon analysis (IDAA) assays. See, for example, Sentmanat MF et al., "A survey of validation strategies for CRISPR-Cas9 editing" , Scientific Reports, 2018, 8, article number 888, which is incorporated herein by reference in its entirety. In some embodiments, for example, as described in the Examples herein, targeted next-generation sequencing (NGS) is used to measure the gene editing efficiency of the engineered Cas12i nuclease in cells. Exemplary genomic sites that can be used to determine the gene editing efficiency of the engineered Cas 12i nuclease (or functional variants thereof) include, but are not limited to, CCR5, AAVS, CD34, RNF2, and EMX1. In some embodiments, the gene editing efficiency of the engineered Cas12i nuclease (or functional variant thereof) is the average gene editing efficiency of the engineered Cas12i nuclease at at least 5, 10, 15, 20, 25, 30, 35, 40, 45, 50, 55, 60, 65 or more sites (such as human cell genomic sites). In some embodiments, the gene editing efficiency (e.g., indel rate) of the engineered Cas12i nuclease (or functional variant thereof) achieve at least 10%, 20%, 30%, 40%, 45%, 50%, 55%, 60%, 65%, 70%, 75%, 80%, 85%, 90%, 95% or higher.

In some embodiments, the engineered Cas12i nuclease (or functional variant thereof) has increased target specificity compared to the reference Cas12i nuclease, with reduced off-target rates (e.g., reduced identified off-target sites, and/or reduced editing efficiency for one or more off-target sites), and/or increased target sequence editing efficiency. In some embodiments, the engineered Cas12i nuclease (or functional variant thereof) has a reduction of at least about 5% (e.g., a reduction of any one of at least about 10%, 20%, 30%, 40%, 50%, 60%, 70%, 80%, 90%, 95%, or 100%) off-target rate compared to the reference Cas12i nuclease. In some embodiments, the engineered Cas12i nuclease (or functional variant thereof) reduces at least one (e.g., at least 2, 3, 4, 5, 6, 7, 8, 9, 10, 20, 50 or more) off-target sites compared to the reference Cas12i nuclease. In some embodiments, the editing efficiency of the engineered Cas12i nuclease (or functional variant thereof) for the target sequence is the same or similar (such as within 1.1 times) compared to the reference Cas12i nuclease, or the editing efficiency of the target sequence is increased (e.g., at least 1.2 times, 1.5 times, 2 times, 3 times, 5 times, 10 times or more compared to the reference Cas12i nuclease). In some embodiments, the engineered Cas12i nuclease (or functional variant thereof) reduces the off-target rate by at least about 5% compared to the reference Cas12i nuclease, and the editing efficiency of the target sequence is the same, similar (such as within 1.1 times), or increased.

### Guide RNA or crRNA

In some embodiments, the guide RNA or crRNA comprises or consists of the following from 5' to 3': a direct repeat sequence, a spacer sequence. In some embodiments, the guide RNA comprises or consists of the following from 5' to 3': a direct repeat sequence, a spacer sequence, a nucleotide sequence constructed in tandem of a direct repeat sequence. In some embodiments, the RNA guide comprises crRNA. In some embodiments, the guide RNA does not comprise tracrRNA.

Generally, the crRNA described herein comprise a direct repeat sequence and a spacer sequence. In certain embodiments, the crRNA comprises, consists essentially of, or consists of a direct repeat sequence linked to a guide sequence or a spacer sequence. In some embodiments, the crRNA comprises a direct repeat sequence - a spacer sequence - a direct repeat sequence (DR-spacer-DR), which is a typical precursor crRNA (pre-crRNA) configuration in other CRISPR systems. In some embodiments, the crRNA comprises truncated a direct repeat sequence and a spacer sequence that are typical of processed or mature crRNA. In some embodiments, the CRISPR-Cas effector protein forms a complex with the RNA guide, and the spacer sequence directs the complex to sequence-specific binding to a target nucleic acid complementary to the spacer sequence.

In some embodiments, the RNA guide comprises a direct repeat. In some embodiments, the RNA guide can form secondary structures, for example, the stem-loop structure as described herein.

In some embodiments, the CRISPR systems described herein comprise multiple RNA guides (e.g., 2, 3, 4, 5, 10, 15, or more) or multiple nucleic acids encoding multiple RNA guides. In some embodiments, the CRISPR systems described herein comprise a single RNA strand or nucleic acid encoding a single RNA strand, wherein the RNA guides are arranged in tandem. The single RNA strand can comprise multiple copies of the same RNA guide, multiple copies of different RNA guides, or a combination thereof. In some embodiments, each RNA guide is specific for a different target nucleic acid.

In some embodiments, the CRISPR systems described herein comprise an RNA guide or a nucleic acid encoding an RNA guide. In some embodiments, an RNA guide comprises or consists of a direct repeat sequence and a spacer sequence capable of hybridizing to the target nucleic acid (e.g., hybridizing under appropriate conditions).

In some embodiments, the RNA guide sequence can be modified in a manner that allows CRISPR effector complex formation and successful binding to the target sequence, while not allowing successful nuclease activity (i.e., no nuclease activity/no indels induced). These modified guide sequences are called "inactivated guides" or "inactivated guide sequences." These inactivation guides or inactivation guide sequences may be catalytically inactive or conformationally inactive for nuclease activity. The inactivating guide sequence is generally shorter than the corresponding guide sequence that results in active RNA cleavage. In some embodiments, the inactivated guide is at least about 5%, 10%, 20%, 30%, 40%, or 50% shorter than a corresponding RNA guide having nuclease activity. The length of the inactivating guide sequence of the RNA guide can be 13 to 15 nucleotides (e.g., 13, 14, or 15 nucleotides), 15 to 19 nucleotides, or 17 to 18 nucleotides (e.g., 17 nucleotides). In some embodiments, the inactivated guide RNA is capable of hybridizing to the target sequence such that the CRISPR system is directed to the genomic loci of interest in the cell without detectable cleavage activity.

The sequence and length of the RNA guides and crRNA described herein can be optimized. In some embodiments, the optimal length of an RNA guide can be determined by identifying the processed form of crRNA or by empirical length studies of RNA guides for crRNA. In some embodiments, the RNA guide sequence comprises base modifications.

In some embodiments, the present invention also provides all possible variants of a nucleic acid (e.g., cDNA) that can be prepared by selecting combinations based on possible codon selection. These combinations are made according to the standard triplet genetic code applied to polynucleotides encoding naturally occurring variants, and all such variants are considered specifically disclosed.

### Spacer sequence

In some embodiments, the spacer sequence (or spacer, guide sequence) can be for example, at least about 70% (e.g., at least about 75%, 80%, 85%, 90%, 91%, 92%, 93%, 94%, 95%, 96%, 97%, 98%, 99% or 100%)complementary to a target sequence of a target nucleic acid (e.g., DNA). In some embodiments, the spacer sequence is complementary to a target sequence of a target nucleic acid (e.g., DNA) with at least 15 (e.g., at least 16, 17, 18, 19, 20, 21, 22, 23, 24, 25, 26, 27, 28 , 29, 30, 35, 40, 45, 50 or more)nucleotides.

It is known in the art that perfect complementarity is not required if the complementarity works sufficiently. Cleavage efficiency adjustment can be exploited by introducing mismatches (e.g. one or more mismatches, e.g. 1 or 2 mismatches between the spacer sequence and the target sequence (including the location of the mismatch along the spacer/target)). Mismatches, such as double mismatches, are more centrally located (i.e., not at the 3' or 5' end); the greater the impact on cleavage efficiency. Therefore, by choosing the location of the mismatch along the spacer sequence, the cleavage efficiency can be adjusted. For example, if less than 100% cleavage of the target is desired (e.g., in a population of cells), 1 or 2 mismatches between the spacer sequence and the target sequence can be introduced in the spacer sequence.

The guide sequence may be of appropriate length. The spacer length of the RNA guide may range from about 11 to 50 (e.g., about 15 to 50) nucleotides. In some embodiments, the guide sequence is between about 18 to about 35 nucleotides, including, for example, 18, 19, 20, 21, 22, 23, 24, 25, 26, 27, 28, 29, 30 , 31, 32, 33, 34 or 35 nucleotides. In some embodiments, the spacer for RNA guide is at least 16 nucleotides, at least 17 nucleotides, at least 18 nucleotides, at least 19 nucleotides, at least 20 nucleotides, at least 21 nucleotides or at least 22 nucleotides in length. In some embodiments, the spacer is 15 to 17 nucleotides, 15 to 23 nucleotides, 16 to 22 nucleotides, 17 to 20 nucleotides, 20 to 24 nucleotides (For example, 20, 21, 22, 23 or 24 nucleotides), 23 to 25 nucleotides (for example, 23, 24 or 25 nucleotides), 24 to 27 nucleotides, 27 to 30 nucleotides, 30 to 45 nucleotides (e.g., 30, 31, 32, 33, 34, 35, 40, or 45 nucleotides), 30 or 35 to 40 nucleotides, 41 to 45 nuclei nucleotides, 45 to 50 nucleotides, or longer in length. In some embodiments, the spacer for RNA guide is 31 nucleotides in length. In some embodiments, the direct repeat for RNA guide is at least 21 nucleotides, or from 21 to 37 nucleotides (e.g., 23, 24, 25, 30, 35, or 36 nucleotides) in length. In some embodiments, the spacer sequence comprises or consists of about 15 to about 34 nucleotides (e.g., 16, 17, 18, 19, 20, 21, or 22 nucleotides). In some embodiments, the spacer is 17 to 31 nucleotides in length. In some embodiments, the spacer sequence is 15 to 24 nucleotides in length. In some embodiments, the direct repeat for RNA guide is 23 or 20 nucleotides in length.

### Direct repeat (DR) sequence

The direct repeat sequence can guide the Cas12i protein (such as any engineered Cas12i nuclease, functional variant or effector protein thereof of the present invention) to bind to the guide gRNA (or crRNA) to form a CRISPR-Cas complex targeting the target sequence. Any DR that can guide the Cas12i nuclease or effector protein engineered in the present application to bind to the guide gRNA (or crRNA) to form a CRISPR-Cas complex targeting the target sequence can be used in the present invention, such as the DR sequences described in US 11168324 (the entire content of which are incorporated herein by reference in their entirety).

Direct repeats can consist of two stretches of nucleotides that can be complementary to each other, separated by intervening nucleotides, such that the direct repeats can hybridize to form double-stranded RNA duplexes (dsRNA duplexes), resulting in a stem-loop structure , in which two complementary stretches of nucleotides form a stem and intervening nucleotides form a loop or hairpin. For example, the intermediate nucleotides forming a "loop" have a length from about 6 nucleotides to about 8 nucleotides, or about 7 nucleotides. In various embodiments, the stem may comprise at least 2, at least 3, at least 4 or 5 base pairs.

In some embodiments, a direct repeat may comprise two complementary stretches of nucleotides of about 4 to about 7 (e.g., 4, 5, 6, 7) nucleotides in length separated by about 5 to about 9 (such as 5, 6, 7, 8, 9) nucleotides. Those skilled in the art can simulate known direct repeating structures.

Direct repeats may comprise or consist of about 13 to about 23 nucleotides, about 22 to about 40 nucleotides, or about 23 to about 38 nucleotides, or about 23 to about 36 nucleotides.

In some embodiments, the direct repeat sequence comprises a stem-loop structure near the 3' end (immediately adjacent to the spacer sequence). In some embodiments, the direct repeat sequence comprises a stem loop near the 3' end, where the stem is 5 nucleotides in length. In some embodiments, the direct repeat sequence comprises a stem loop near the 3' end, wherein the stem is 5 nucleotides in length and the loop is 7 nucleotides in length. In some embodiments, the direct repeat sequence comprises a stem-loop near the 3' end, where the stem is 5 nucleotides in length and the loop is 6, 7, or 8 nucleotides in length.

In some embodiments, the direct repeat sequence comprises the sequence near the 3' end 5'-CCGUCNNNNNNUGACGG-3' (SEQ ID NO.68), wherein N refers to any nucleobase. In some embodiments, the direct repeat sequence comprises the sequence near the 3' end 5'-GUGCCNNNNNUGGCAC-3' (SEQ ID NO.69), wherein N refers to any nucleobase.

In some embodiments, the direct repeat sequence comprises the sequence near the 3' end 5'-GUGUCN₅₋₆UGACAX₁-3' (SEQ ID NO.70 or 71), where N₅₋₆ refers to any continuous sequence of 5 or 6 nucleobases, and X₁ refers to C or T or U. In some embodiments, the direct repeat sequence comprises the sequence near the 3' end 5'-UCX₃UX₅X₆X₇UUGACGG-3' (SEQ ID NO.72), wherein X₃ refers to C or T or U, and X₅ refers to A or T or U, X₆ refers to A or C or G, and X₇ refers to A or G. In some embodiments, the direct repeat sequence comprises the sequence near the 3' end 5'-CCX₃X₄X₅CX₇UUGGCAC-3' (SEQ ID NO.73), wherein X₃ refers to C or T or U, and X₄ refers to A or T or U, X₅ refers to C or T or U, and X₇ refers to A or G. In some embodiments, the nucleotides encoding the direct repeat sequence comprise or consist of nucleotide sequences with at least about 80% identity, such as at least about 85%, 90%, 91%, 92%, 93%, 94%, 95%, 96%, 97%, 98%, 99% or 100% identity with SEQ ID NO.59 (AGAAATCCGTCTTTCATTGACGG) or SEQ ID NO.79 (GTTGCAAAACCCAAGAAATCCGTCTTTCATTGACGG). In some embodiments, the nucleotides encoding the direct repeat sequence comprise at least 21 (e.g., 21, 22, 23, 24, 25, 26, 27, 28, 29, 30, 31, 32, 33, 34, 35 or 36) nucleotides of SEQ ID NO.79.

"Stem-loop structure" refers to a nucleic acid having a secondary structure that comprises a nucleotide region known or predicted to form a double stranded (stem portion), which is connected on one side by a region (ring portion) mainly composed of single stranded nucleotides. The terms "hairpin" and "turn-back" structures are also used herein to refer to stem-loop structures. Such structures are well known in the art, and these terms are used consistent with their commonly known meanings in the art. As is known in the art, stem-loop structures do not require precise base pairing. Thus, a stem may comprise one or more base mismatches. Alternatively, base pairing may be exact, i.e., not comprise any mismatches. In some embodiments, the direct repeat stem consists of 5 complementary nucleobases that hybridize to each other, and the loop length is 6, 7, or 9 nucleotides.

In some embodiments, the sequence encoding the direct repeat comprises or consists of the sequence shown in SEQ ID NO.79. In some embodiments, the sequence encoding the direct repeat comprises or consists of a truncated nucleic acid having the first three 5' nucleotides of the nucleic acid sequence of SEQ ID NO.79. In some embodiments, the sequence encoding the direct repeat comprises or consists of a truncated nucleic acid having the first four 5' nucleotides of the SEQ ID NO.79 nucleic acid sequence. In some embodiments, the sequence encoding the direct repeat comprises or consists of a truncated nucleic acid having the first five 5' nucleotides of the nucleic acid sequence of SEQ ID NO.79. In some embodiments, the sequence encoding the direct repeat comprises or consists of a truncated nucleic acid having a first six 5' nucleotides of the SEQ ID NO.79 nucleic acid sequence. In some embodiments, the sequence encoding the direct repeat comprises or consists of a truncated nucleic acid having the first seven 5' nucleotides of the SEQ ID NO.79 nucleic acid sequence. In some embodiments, the sequence encoding the direct repeat comprises or consists of a truncated nucleic acid having the first eight 5' nucleotides of the SEQ ID NO.79 nucleic acid sequence. In some embodiments, the sequence encoding the direct repeat comprises or consists of the sequence shown in SEQ ID NO.59.

In some embodiments, the direct repeat is a "functional variant" of the RNA sequence encoded by SEQ ID NO.59 or 79, such as a "functional truncated version", a "functional extended version", or a "functional replacement version", such as part of SEQ ID NO.79 (truncated version), still has the DR function. A "functional variant" of a DR is a DR sequence that, after extension (a functionally extended version) or truncation (a functionally truncated version) of the 5 'and/or 3' ends of the reference DR (e.g., a parental DR), and/or insertion, deletion, and/or substitution (functional replacement version) of one or more nucleotides in the reference DR sequence, still possesses at least 20% (such as at least about any 30%, 40%, 50%, 60%, 70%, 80%, 90%, 95%, or more) of the function of the reference DR, that is, the function of mediating the binding of Cas12i protein to the corresponding crRNA. DR functional variants generally retain the stem-loop-like secondary structure or portions thereof that can be bound by Cas 12i protein. In some embodiments, DR or functional variants thereof comprise a stem-loop-like secondary structure or a portion thereof that can be bound by Cas12i protein. In some embodiments, the DR or functional variant thereof comprises at least two (such as 2, 3, 4, 5 or more) stem-loop-like secondary structures or portions thereof that can be bound by Cas12i protein.

### Prime editing guide RNA (pegRNA)

The PEgRNA is altered (relative to the standard guide RNA) to contain an extension that provides a DNA synthesis template sequence encoding a single-stranded DNA flap that is homologous to the strand targeting the endogenous DNA sequence to be edited but comprises the desired one or multiple nucleotide changes and are incorporated into the target DNA molecule after synthesis by a polymerase (e.g., reverse transcriptase). PEgRNA has been described, for example, in WO2020191246, WO2021226558, which are incorporated herein by reference in their entirety.

In various embodiments, the extended guide RNA comprises (a) a guide RNA and (b) an RNA extension at the 5' or 3' end of the guide RNA or in an intramolecular location of the guide RNA. Preferably, the intramolecular positioning of the extension portion does not disrupt the function of the original spacer. RNA extension can comprise (i) a reverse transcription template sequence comprising the desired nucleotide changes, (ii) a reverse transcription primer binding site, and (iii) optional linker sequences. In various embodiments, the reverse transcription template sequence may encode a single-stranded DNA flap that is complementary to an endogenous DNA sequence adjacent the nick position, wherein the single-stranded DNA flap comprises the desired nucleotide change. Single-stranded DNA flaps can substitute endogenous single-stranded DNA at the nick position. In various embodiments, the desired nucleotide change incorporated into the target DNA may be a single nucleotide change (e.g., a transition or transversion), the insertion of one or more nucleotides, or the deletion of one or more nucleotides.

In various embodiments, the desired nucleotide change may be a single nucleotide substitution (e.g., a transition or transversion change), a deletion, or an insertion. For example, a desired nucleotide change may be (1) G to T substitution, (2) G to A substitution, (3) G to C substitution, (4) T to G substitution, (5) T to A substitution, (6) T to C substitution, (7) C to G substitution, (8) C to T substitution, (9) C to A substitution, (10) A to T substitution, (11) A to G substitution, or ( 12) A to C substitution.

### PAM

The engineered Cas12i nuclease (or functional variant thereof) of the present invention is able to recognize the protospacer adjacent motif (PAM). In some embodiments, the target nucleic acid comprises PAM. In some embodiments, the PAM is located at the 5' end of a sequence in the target nucleic acid that is complementary to the targeting sequence of the guide RNA. In some embodiments, the PAM comprises or consists of the nucleic acid sequence 5'-TTN-3', 5'-TTH-3', 5'-TTY-3', or 5'-TTC-3'. In some embodiments, a the PAM suitable for the engineered Cas12i nuclease (or functional variant thereof) of the present invention comprises or consisting of the nucleic acid sequence 5'-NNNN-3'(N=A, T, G, C), such as 5'-NTTN-3', 5'-NTAN-3', 5'-NTCN-3', 5'-NTGN-3', 5'-NATN-3', 5'-NAAN-3', 5'-NACN-3', 5'-NAGN-3', 5'-NCTN-3', 5'-NCAN-3', 5'-NCCN-3', 5'-NCGN-3', 5'-NGTN-3', 5'-NGAN-3', 5'-NGCN-3', 5'-NGGN-3'. In some embodiments, the PAM comprises or consists of the nucleic acid sequence 5'-TTTN-3'. In some embodiments, the PAM comprises or consists of the nucleic acid sequence 5'-TTN-3'.

In some embodiments, PAMs suitable for the engineered Cas12i nuclease (or functional variants thereof) of the present invention comprise the nucleic acid sequences 5'-TTTA-3', 5'-CTTA-3', 5'-GTTA-3', 5'-ATTA-3', 5'-TTTC-3', 5'-CTTC-3', 5'-GTTC-3', 5'-ATTC-3', 5'-TTTG-3', 5'-CTTG-3', 5'-GTTG-3', 5'-ATTG-3', 5'-TTTT-3', 5'-CTTT-3', 5'-GTTT-3' ,5'-ATTT-3'.

### Engineered Cas12i effector proteins

The present application provides engineered Cas12i (such as Cas12i2) effector proteins, which comprise any engineered Cas12i nuclease described in the present invention or functional variants thereof (such as CasXX shown in SEQ ID NO.8), which have improved activity, such as target binding, double-stranded cleavage activity, nickase activity and/or gene editing activity. In some embodiments, engineered Cas12i effector proteins (e.g., Cas12i nucleases, Cas12i nickases, Cas12i fusion effector proteins, or split Cas12i effector proteins) are provided, comprising any of the engineered Cas12i nucleic acid described herein or functional derivatives thereof (such as dCas 12i). In some embodiments, the engineered Cas12i effector protein comprises, consists essentially of, or consists of any of the engineered Cas12i nucleases described herein or functional variants thereof.

Engineered Cas12i effector proteins based on any of the engineered Cas12i2 nucleases described herein or functional variants thereof (e.g., CasXX set forth in SEQ ID NO.8) are also provided. In some embodiments, the engineered Cas12i effector protein has enzymatic activity (e.g., DNA double-strand cleavage activity). In some embodiments, the engineered Cas12i effector protein has nuclease activity that cleaves both strands of a target duplex nucleic acid (e.g., duplex DNA). In some embodiments, the engineered Cas12i effector protein has nickase activity, i.e., cleaves a single strand of a target duplex nucleic acid (e.g., duplex DNA). In some embodiments, the engineered Cas12i effector protein comprises an enzyme-inactive mutant of the engineered Cas12i nuclease.

### Split Cas12i effector protein

The present application also provides split Cas12i effector proteins based on any one of the engineered Cas12i nucleases described herein or functional variants thereof (e.g., CasXX shown in SEQ ID NO.8).

Split Cas12i effector proteins may be advantageous for delivery. In some embodiments, the engineered Cas12i effector protein is split into two parts of the enzyme, which can be reconstituted together to provide a substantially functional Cas12i effector protein. Cas effector proteins can be provided using known methods, for example, split versions of Cas12 and Cas9 proteins have been described in, for example, WO2016/112242, WO2016/205749 and PCT/CN 2020/111057, which are incorporated herein by reference in their entirety.

In some embodiments, a split Cas12i effector protein is provided, comprising a first polypeptide and a second polypeptide, the N-terminal portion of the first polypeptide comprises any of the engineered Cas12i nucleases described herein or functional derivatives thereof, the second polypeptide comprises the C-terminal portion of any one of the engineered Cas12i nuclease or functional derivatives thereof, wherein the first polypeptide and the second polypeptide are able to associate with each other in the presence of the guide RNA comprising the guide sequence to form a CRISPR complex that specifically binds to a target nucleic acid comprising a target sequence complementary to the guide sequence. In some embodiments, the first polypeptide and the second polypeptide each comprise a dimerization domain. In some embodiments, the first dimerization domain and the second dimerization domain associate with each other in the presence of an inducer (e.g., rapamycin). In some embodiments, the first polypeptide and second polypeptide do not comprise a dimerization domain. In some embodiments, the split Cas12i effector protein is autoinduced.

The engineered Cas12i effector protein of the present invention can be divided in a manner that does not affect the catalytic domain. Cas12i effector proteins can function as nucleases (including nickases) or can be inactivated enzymes, which are essentially RNA-guided DNA-binding proteins with little or no catalytic activity (e.g. due to mutations in their catalytic domain) .

In some embodiments, the nuclease lobe and alpha-helical lobe of the engineered Cas12i effector protein are expressed as separate polypeptides. Although the nuclease lobe and α-helical lobe do not interact on their own, the RNA guide sequence recruits them into a complex that recapitulates the activity of the full-length Cas12i nuclease and catalyzes site specific DNA cleavage. In some embodiments, modified RNA guide sequences can be used to eliminate the activity of the splitting enzyme by preventing dimerization, thereby allowing the development of an inducible dimerization system. Such split enzymes are described, for example, in Wright, Addison V, et al. "Rational design of a split-Cas9 enzyme complex," Proc. Nat'l. Acad. Sci., 112.10 (2015): 2984-2989, which is incorporated by reference in its entirety.

Split Cas12i effector protein portions described herein can be designed by splitting (i.e., splitting) a reference engineered Cas12i effector protein (e.g., a full-length engineered Cas12i nuclease) into two halves at a split position, the position is the point where the N-terminal portion of the reference Cas12i effector protein separates from the C-terminal portion. In some embodiments, the N-terminal portion comprises amino acid residues 1 to X of the reference Cas12i effector protein, and the C-terminal portion comprises amino acid residues X+1 to the C-terminus of the reference Cas12i effector protein . In this embodiment, the numbering is consecutive, but this is not required, as it is also contemplated that the amino acids (or the nucleotides encoding them) may be arbitrary from any of the split ends and/or mutations (e.g., insertions, deletions, and substitutions) within an internal region of the polypeptide chain, provided that the reconstructed engineered Cas 12i effector protein retains sufficient DNA binding activity (if desired), DNA nickase or cleavage activity, e.g., compared to the reference Cas12i effector protein, it has at least about 40% (such as at least about 50%, 60%, 70%, 80%, 90%, 95%, or more) activity.

Split points can be designed in silico and cloned into the construct. During this process, mutations can be introduced into split Cas12i effector proteins and non-functional domains can be removed. In some embodiments, the two portions or fragments of the split Cas12i effector protein (i.e., the N-terminal and C-terminal fragments) can form a complete Cas 12i effector protein, which comprises, for example, at least about 70% (e.g., at least about 80%, 90%, 95%, 96%, 97%, 98%, 99% or more) of the complete Cas12i effector protein sequence.

The split Cas12i effector proteins may each comprise one or more dimerization domains. In some embodiments, the first polypeptide comprises a first dimerization domain fused to a first split Cas12i effector protein portion, and the second polypeptide comprises a second dimerization domain fused to a second split Cas 12i effector protein portion. The dimerization domain can be fused to the split Cas 12i effector protein portion via a peptide linker (e.g., a flexible peptide linker such as a GS linker) or chemical bond. In some embodiments, the dimerization domain is fused to the N-terminus of the split Cas12i effector protein portion. In some embodiments, the dimerization domain is fused to the C-terminus of the split Cas12i effector protein portion.

In some embodiments, the split Cas12i effector protein does not comprise any dimerization domain.

In some embodiments, the dimerization domain promotes the association of two split Cas12i effector protein portions. In some embodiments, the split Cas12i effector protein portion is induced by an inducer to associate or dimerize into a functional Cas12i effector protein. In some embodiments, the split Cas12i effector protein comprises an inducible dimerization domain. In some embodiments, the dimerization domain is not an inducible dimerization domain, that is, the dimerization domain dimerizes in the absence of an inducer.

The inducer may be an inducing energy source or inducing molecule other than guide RNA (e.g., crRNA). The inducer reconstitutes the two split Cas12i effector protein portions into a functional Cas12i effector protein through induced dimerization of the dimerization domain. In some embodiments, the inducer brings together two split Cas12i effector protein portions through inducing association of an inducible dimerization domain. In some embodiments, the two split Cas12i effector protein portions do not associate with each other to reconstitute into a functional Cas12i effector protein in the absence of an inducer. In some embodiments, two separate Cas12i effector protein portions can associate with each other in the presence of a guide RNA (e.g., crRNA) to reconstitute a functional Cas12i effector protein in the absence of an inducer.

The inducer of the present application may be heat, ultrasound, electromagnetic energy or chemical compounds. In some embodiments, the inducer is an antibiotic, a small molecule, a hormone, a hormone derivative, a steroid or a steroid derivative. In some embodiments, the inducer is abscisic acid (ABA), doxymycin (DOX), cumate, rapamycin, 4-hydroxytamoxifen (4OHT), estrogen or ecdysone. In some embodiments, the split Cas12i effector system is an inducer-controlled system selected from the group consisting of: antibiotic-based induction systems, electromagnetic energy-based induction systems, small molecule-based induction systems, nuclear receptor-based induction systems and hormone-based induction systems. In some embodiments, the split Cas12i effector system is an inducer-controlled system selected from the group consisting of: tetracycline (Tet)/DOX induction system, light induction system, ABA induction system, cumate repressor/operator system, 4OHT/estrogen induction system, ecdysone-based induction system and FKBP12/FRAP (FKBP12-rapamycin complex) induction system. Such inducers are also discussed herein and in PCT/US2013/051418, which is incorporated herein by reference in its entirety. The FRB/FKBP/rapamycin system has been described in Paulmurugan and Paulmurugan and Gambhir, Cancer Res, August 15, 2005 65; 7413, and Crabtree et al., Chemistry & Biology 13, 99-107, Jan 2006, which are incorporated herein by reference in their entirety.

In some embodiments, pairs of split Cas12i effector proteins are separated and inactive until dimerization of the dimerization domain (e.g., FRB and FKBP) is induced, which results in reassembly of functional Cas12i effector protein nucleases. In some embodiments, the first split Cas12i effector protein comprising the first half of the inducible dimer (e.g., FRB) is delivered separately and/or in a position separate from the second split Cas12i effector protein comprising the second half of the inducible dimer (e.g., FKBP).

Other exemplary FKBP-based induction systems that may be used in the inducer-controlled split Cas12i effector systems described herein include, but are not limited to: FKBP that dimerizes with calcineurin (CNA) in the presence of FK506; FKBP that dimerizes with CyP-Fas in the presence of FKCsA; FKBP that dimerizes with FRB in the presence of rapamycin; GyrB that dimerizes with GryB in the presence of coumamycin; GAI that dimerizes with GID1 in the presence of mycin; or Snap-tag that dimerizes with HaloTag in the presence of HaXS.

Alternatives within the FKBP family itself were also considered. For example, FKBP homodimerizes (i.e., one FKBP dimerizes with another FKBP) in the presence of FK1012.

In some embodiments, the dimerization domain is FKBP and the inducer is FK1012. In some embodiments, the dimerization domain is GryB and the inducer is coumamycin. In some embodiments, the dimerization domain is ABA and the inducer is gibberellin.

In some embodiments, the split Cas12i effector protein portion can be autoinduced (i.e., autoactivated or autoinduced) in the absence of an inducer to associate/dimerize into a functional Cas12i effector protein. Without being bound by any theory or hypothesis, auto-induction of the split Cas12i effector protein portion may be mediated by binding to a guide RNA such as crRNA. In some embodiments, the first polypeptide and second polypeptide do not comprise a dimerization domain. In some embodiments, the first polypeptide and second polypeptide comprise a dimerization domain.

In some embodiments, reconstituted Cas12i effector proteins of split Cas12i effector systems (including inducer-controlled and auto-induced systems) described herein have an editing efficiency of at least about 60% (for example, any of at least about 70%, 75%, 80%, 85%, 90%, 95%, 98%, 99% or more) compared to the reference Cas12i effector protein editing efficiency.

In some embodiments, the reconstituted Cas12i effector protein of the inducer-controlled split Cas12i effector system described herein has an editing efficiency of no more than about 50% (such as any of about 45%, 40%, 35%, 30%, 25%, 20%, 15%, 10%, 5%, or less) compared to the reference Cas12i effector protein editing efficiency in the absence of the inducer (i.e., due to autoinduction).

### Fusion Cas12i effector protein

The application also provides engineered Cas12i effector proteins that contain additional protein domains and/or components, such as linkers, nuclear localization/export sequences, functional domains, and/or reporter proteins.

In some embodiments, the engineered Cas12i effector protein is a protein complex comprising one or more heterologous protein domains (e.g., about or greater than about 1, 2, 3, 4, 5, 6, 7, 8, 9, 10 or more domains) and the nucleic acid targeting domain of any of the engineered Cas12i nucleases of the present invention or a functional derivative thereof. In some embodiments, the engineered Cas12i effector protein is a fusion protein comprising one or more heterologous protein domains (e.g., about 1, 2, 3, 4, 5, 6, 7, 8, 9, 10 or more domains) fused to the engineered Cas12i nuclease or a functional variant thereof (e.g., CasXX shown in SEQ ID NO.8).

In some embodiments, the engineered Cas 12i effector proteins of the present application may comprise (e.g., via a fusion protein, such as via one or more peptide linkers, such as GS peptide linkers, etc.) one or more functional domains or be associated with it (for example, through the co-expression of multiple proteins). In some embodiments, the one or more functional domains are enzymatic domains. These functional domains can have multiple activities, such as DNA and/or RNA methylase activity, nucleotide deaminase activity (such as adenosine deaminase activity, cytidine deaminase activity), demethylase activity, transcription activating activity, transcription repressing activity, transcription release factor activity, histone modification activity, RNA cleavage activity, DNA cleavage activity (such as double-stranded endonuclease activity, nickase activity), nucleic acid binding activity and switch activity (e.g., photoinduced or chemically induced). In some embodiments, the one or more functional domains are a transcriptional activation domain (i.e., a transactivation domain) or a repressor domain. In some embodiments, the one or more functional domains are histone modification domains. In some embodiments, the one or more functional domains are a transposase domain, an HR (homologous recombination) machinery domain, a recombinase domain, and/or an integrase domain. In some embodiments, the functional domain is Krüppe1-associated box (KRAB), VP64, VP16, Fok1, P65, HSF1, MyoD1, biotin-APEX, APOBEC1, AID, PmCDAl, Tad1, and M-MLV reverse transcriptase. In some embodiments, the functional domain is selected from the group consisting of: a translation initiation domain, a transcription repression domain, a transactivation domain, an epigenetic modification domain, a nucleobase editing domain (e.g., CBE or ABE domain), reverse transcriptase domain, reporter domain (e.g., fluorescent domain), and nuclease domain.

In some embodiments, the functional domain has the activity of modifying target DNA or target DNA-related proteins, and the activity is selected from one or more of nuclease activity (e.g., HNH nuclease, RuvC nuclease, Trex1 nuclease, Trex2 nuclease), methylation activity, demethylation activity, DNA repair activity, DNA damage activity, deamination activity, dismutase activity, alkylation activity, depurination activity, oxidation activity, pyrimidine dimer formation activity, integrase activity , transposase activity, recombinase activity, polymerase activity, ligase activity, helicase activity, photolyase activity, glycosylase activity, acetyltransferase activity, deacetylase activity, kinase activity, phosphatase activity, ubiquitin ligase activity, deubiquitination activity, adenylation activity, deadenylation activity, SUMOylation activity, deSUMOylation activity, ribosylation activity, deribosylation activity, myristoylation activity, demyristoylation activity, glycosylation activity (e.g., from O-GlcNAc transferase), deglycosylation activity, transcription repression activity, and transcription activation activity. Target DNA-related proteins refer to proteins that can bind to target DNA, or proteins that can bind to proteins that can bind to target DNA, such as histones, transcription factors, Mediator, etc.

In some embodiments, the positioning of one or more functional domains in the engineered Cas12i effector protein allows for the correct spatial orientation of the functional domains to affect the target with the conferred functional role. For example, if the functional domain is a transcriptional activator (e.g., VP16, VP64, or p65), the transcriptional activator is placed in a spatial orientation that enables it to affect target transcription. Likewise, a transcriptional repressor is positioned to affect the transcription of a target, and a nuclease (e.g., Fokl) is positioned to cleave or partially cleave the target. In some embodiments, the functional domain is located at the N-terminus of the engineered Cas12i effector protein. In some embodiments, the functional domain is located at the C-terminus of the engineered Cas12i effector protein. In some embodiments, the engineered Cas12i effector protein comprises a first functional domain at the N-terminus and a second functional domain at the C-terminus. In some embodiments, the engineered Cas12i effector protein comprises a catalytically inactive mutant (dCas12i) of any of the engineered Cas12i nucleases described herein fused to one or more functional domains.

In some embodiments, the engineered Cas12i effector protein is a transcriptional activator. In some embodiments, the engineered Cas12i effector protein comprises an enzyme-inactive variant of any of the engineered Cas12i nucleases described herein fused to a transactivation domain. In some embodiments, the transactivation domain is selected from the group consisting of VP64, p65, HSF1, VP16, MyoD1, HSF1, RTA, SET7/9, and combinations thereof. In some embodiments, the transactivation domain comprises VP64, p65, and HSF1. In some embodiments, the engineered Cas12i effector protein comprises two split Cas12i effector polypeptides, each fused to a transactivation domain.

In some embodiments, the engineered Cas12i effector protein is a transcriptional repressor. In some embodiments, the engineered Cas12i effector protein comprises an enzyme-inactive variant of any of the engineered Cas12i nucleases described herein fused to a transcription repression domain. In some embodiments, the transcription repressor domain is selected from the group consisting of Krüppe1-associated box (KRAB), EnR, NuE, NcoR, SID, SID4X, and combinations thereof. In some embodiments, the engineered Cas 12i effector protein comprises two split Cas 12i effector polypeptides, each fused to a transcription repression domain.

In some embodiments, the engineered Cas12i effector protein is a base editor, such as a cytosine editor or an adenosine editor. In some embodiments, the engineered Cas12i effector protein comprises an enzyme-inactive variant of any of the engineered Cas12i nucleases described herein fused to a nucleobase editing domain, the nucleobase The editing domain is such as a cytosine base editing (CBE) domain or an adenosine base editing (ABE) domain. In some embodiments, the nucleobase editing domain is a DNA editing domain. In some embodiments, the nucleobase editing domain has deaminase activity. In some embodiments, the nucleobase editing domain is a cytosine deaminase domain. In some embodiments, the nucleobase editing domain is an adenosine deaminase domain. Exemplary Cas nuclease-based base editors are described, for example, in WO2018/165629A1 and WO2019/226953A1, which are incorporated herein by reference in their entirety. Exemplary CBE domains include, but are not limited to: activation-induced cytidine deaminase or AID (e.g., hAID), apolipoprotein B mRNA editing complex, or APOBEC (e.g., rat APOBEC1, hAPOBEC3A/B/C/D/E /F/G) and PmCDA1. Exemplary ABE domains include, but are not limited to: TadA, ABE8, and variants thereof (see, e.g., Gaudelli et al., 2017, Nature 551: 464-471; and Richter et al., 2020, Nature Biotechnology 38: 883-891). In some embodiments, the functional domain is an APOBEC1 domain, such as a rat APOBEC1 domain. In some embodiments, the functional domain is a TadA domain, such as an *E. coli* TadA domain. In some embodiments the engineered Cas12i effector protein further comprises one or more nuclear localization sequences.

### Adenosine deaminase

As used herein, the term "adenosine deaminase" or "adenosine deaminase protein" refers to a protein, a polypeptide, or one or more functional domains of a protein or polypeptide that are capable of catalyzing the hydrolytic deamination reaction of the conversion of adenine (or the adenine moiety of a molecule) into hypoxanthine (or the hypoxanthine moiety of the molecule), which is shown below. In some embodiments, the adenine-comprising molecule is adenosine (A) and the hypoxanthine-comprising molecule is inosine (I). Adenine-comprising molecules can be deoxyribonucleic acid (DNA) or ribonucleic acid (RNA).

Adenosine deaminases that may be used in conjunction with enzyme-inactive variants of any of the engineered Cas12i nucleases of the present invention include, but are not limited to, members of the family of enzymes known as adenosine deaminase acting on RNA (ADAR), a member of the enzyme family known as adenosine deaminase acting on tRNA (ADAT), and other family members comprising an adenosine deaminase domain (ADAD). Adenosine deaminase targets adenine in RNA/DNA and RNA duplexes. In fact, Zheng et al. (Nucleic Acids Res. 2017, 45(6): 3369-3377) demonstrated that ADAR can perform adenosine to inosine editing reactions on RNA/DNA and RNA/RNA duplexes. In some embodiments, adenosine deaminase can be modified to increase its ability to edit DNA in an RNA/DNA heteroduplex of an RNA duplex.

In some embodiments, adenosine deaminase is derived from one or more metazoan species, including but not limited to mammals, birds, frogs, squid, fish, flies, and worms. In some embodiments, the adenosine deaminase is a human, squid, or Drosophila adenosine deaminase.

In some embodiments, the adenosine deaminase is a human ADAR, including hADAR1, hADAR2, hADAR3. In some embodiments, the adenosine deaminase is a *Caenorhabditis elegans* ADAR protein, including ADR-1 and ADR-2. In some embodiments, the adenosine deaminase is a Drosophila ADAR protein, including dAdar. In some embodiments, the adenosine deaminase is a squid (*Loligo pealeii*) ADAR protein, including sqADAR2a and sqADAR2b. In some embodiments, the adenosine deaminase is a human ADAT protein. In some embodiments, the adenosine deaminase is a Drosophila ADAT protein. In some embodiments, the adenosine deaminase is a human ADAD protein, including TENR (hADAD1) and TENRL (hADAD2). In some embodiments, the adenosine deaminase is TadA8e.

In some embodiments, the adenosine deaminase is a TadA protein, such as *E. coli* TadA. See Kim et al., Biochemistry 45:6407-6416 (2006); Wolf et al., EMBO J. 21:3841-3851 (2002). In some embodiments, the adenosine deaminase is mouse ADA. See Grunebaum et al., Curr. Opin. Allergy Clin. Immunol. 13:630-638(2013). In some embodiments, the adenosine deaminase is human ADAT2. See Fukui et al., J. Nucleic Acids 2010:260512 (2010). In some embodiments, the deaminase (e.g., adenosine or cytidine deaminase) is one or more of those described in: Cox et al., Science. November 24, 2017; 358(6366):1019-1027; Komore et al., Nature. May 19, 2016; 533(7603):420-4; and Gaudelli et al., Nature. November 23, 2017; 551(7681):464- 471.

In some embodiments, an adenosine deaminase protein comprises one or more deaminase domains. Without wishing to be bound by a particular theory, it is contemplated that the deaminase domain functions to recognize one or more target adenosine (A) residues contained in the double-stranded nucleic acid substrate and convert them to an inosine (I) residue.

### Cytidine deaminase

In some embodiments, the deaminase is cytidine deaminase. As used herein, the term "cytidine deaminase" or "cytidine deaminase protein" refers to a protein, a polypeptide, or one or more functional domains of a protein or polypeptide that are capable of catalyzing a hydrolytic deamination reaction of the conversion of cytosine (or the cytosine moiety of a molecule) into uracil (or the uracil moiety of the molecule). In some embodiments, the cytosine-comprising molecule is cytidine (C) and the uracil-comprising molecule is uridine (U). The cytosine-comprising molecule may be deoxyribonucleic acid (DNA) or ribonucleic acid (RNA).

Cytidine deaminase enzymes that may be used in conjunction with enzyme-inactive variants of any of the engineered Cas12i nucleases of the present invention comprise, but are not limited to, a member of the enzyme family of family deaminase enzymes known as the apolipoprotein B mRNA editing complex (APOBEC), activation-induced deaminase (AID), or cytidine deaminase 1 (CDA1). In some embodiments, the deaminase is selected from a group consisting of deaminase in APOBEC1 deaminase, APOBEC2 deaminase, APOBEC3A deaminase, APOBEC3B deaminase, APOBEC3C deaminase and APOBEC3D deaminase, APOBEC3E deaminase, APOBEC3F deaminase, APOBEC3G deaminase, APOBEC3H deaminase or APOBEC4 deaminase.

In some embodiments, cytidine deaminase is capable of targeting cytosine in a single strand of DNA. In some embodiments, a cytidine deaminase can edit on a single strand present outside the binding component. In some embodiments, a cytidine deaminase can be edited at a localized vesicle, such as a localized vesicle formed by a target editing site but directing the formation of a sequence mismatch.

In some embodiments, the cytidine deaminase may contain mutations that contribute to focused activity, such as those described in Kim et al., Nature Biotechnology (2017) 35(4):371-377 (doi: 10.1038/nbt.3803.

In some embodiments, the cytidine deaminase is derived from one or more metazoan species, including but not limited to mammals, birds, frogs, squid, fish, flies, and worms. In some embodiments, the cytidine deaminase is a human, primate, bovine, dog, rat or mouse cytidine deaminase.

In some embodiments, the cytidine deaminase is a human APOBEC, including hAPOBEC1 or hAPOBEC3. In some embodiments, the cytidine deaminase is human AID.

In some embodiments, a cytidine deaminase protein comprises one or more deaminase domains. Without wishing to be bound by theory, it is expected that the deaminase domain functions to recognize one or more target cytosine (C) residues contained in single-stranded vesicles of the RNA duplex and convert them to uracil (U) residues.

In some embodiments, the engineered Cas12i effector protein is the master editor. Cas9-based master editors are described, for example, in A. Anzalone et al., Nature, 2019, 576(7785): 149-157, which is incorporated herein by reference in its entirety. In some embodiments, the engineered Cas12i effector protein comprises a nickase variant of any of the engineered Cas12i nucleases described herein fused to a reverse transcriptase domain. In some embodiments, the functional domain is a reverse transcriptase domain. In some embodiments, the reverse transcriptase domain is M-MLV reverse transcriptase or a variant thereof, such as an M-MLV reverse transcriptase having one or more mutations of D200N, T306K, W313F, T330P, and L603W. In some embodiments, engineered CRISPR/Cas 12i systems comprising the master editor are provided. In some embodiments, the engineered CRISPR/Cas12i system further comprises a second Cas12i nickase, e.g., based on the same engineered Cas12i nuclease as the master editor. In some embodiments, the engineered CRISPR/Cas12i system comprises a prime editing guide RNA (pegRNA) that comprises a primer binding site and a reverse transcriptase (RT) template sequence.

In some embodiments, the present application provides one or more split Cas12i effector system with functional domains (e.g., 1, 2, 3, 4, 5, 6 or more) associated (i.e. bound or fused) with one or both of the segmented Cas12i effector protein moieties. The functional domain may be provided as part of the first and/or second split Cas 12i effector protein as a fusion within the construct. The functional domain is typically fused to other portions of the split Cas12i effector protein (e.g., portions of the split Cas12i effector protein) through a peptide linker (such as a GS linker). These functional domains can be used to reconfigure the function of the split Cas12i effector system based on catalytically inactive Cas12i effector proteins.

In some embodiments, the engineered Cas12i effector protein comprises one or more nuclear localization sequences (NLS) and/or one or more nuclear export sequences (NES). Exemplary NLS sequences comprise, for example, PKKKRKVPG (SEQ ID NO.66) and ASPKKKRKV (SEQ ID NO.67). NLS and/or NES can be operably linked to the N-terminus and/or C-terminus of the engineered Cas12i effector protein or to a polypeptide chain in the engineered Cas12i effector protein. In some embodiments, NLS and/or NES can be linked to the N-terminus and/or C-terminus of any engineered Cas12i nuclease described herein or functional variant thereof.

In some embodiments, the engineered Cas 12i effector protein can encode additional components, such as a reporter protein. In some embodiments, the engineered Cas12i effector protein comprises a fluorescent protein, such as GFP. Such systems could allow imaging of genomic sites (see, for example, "Dynamic Imaging of Genomic sites in Living Human Cells by an Optimized CRISPR/Cas System" Chen B et al. Cell 2013). In some embodiments the engineered Cas12i effector protein is an inducible split Cas12i effector system useful for imaging genomic sites.

In some embodiments, an engineered Cas12i effector protein is provided, wherein the effector protein is capable of inducing double-strand breaks or single-strand breaks in DNA molecules.

In some embodiments, an engineered Cas12i effector protein is provided, wherein the functional derivative of the engineered Cas12i nuclease is an enzyme-inactive mutant, for example, a Cas12i2 nuclease-inactivating mutant comprising D599A, E833A, S883A, H884A, D886A, R900A and/or D1019A (the amino acid positions are defined by the corresponding amino acid positions shown in SEQ ID NO.1), and a Cas12i1 nuclease-inactivating mutant comprising D647A, E894A, and/or D948A (the amino acid positions are defined by the corresponding amino acid positions shown in SEQ ID NO.13). Known enzyme-inactivating mutants of Cas12i2 nuclease, such as any enzyme-inactivating mutation of Cas12i2 nuclease described in US10808245B2 and Huang X. et al., Nature Communications, 11, Article number: 5241 (2020) can be combined with the mutations in this application to provide functional derivatives of engineered Cas12i nucleases and their corresponding effector proteins.

### Engineered CRISPR-Cas12i system

In some embodiments, an engineered CRISPR-Cas12i system is provided, including: (a) any engineered Cas12i effector protein (such as engineered Cas12i nuclease or functional variant thereof) described in this application, for example, CasXX shown in SEQ ID NO.8); and (b) a guide RNA comprising a guide sequence complementary to the target sequence, or one or more nucleic acids encoding the guide RNA;

Wherein, the engineered Cas12i effector protein and the guide RNA can form a CRISPR complex that specifically binds to a target nucleic acid comprising the target sequence and induces modification of the target nucleic acid (such as double-stranded or single-stranded cleavage, base editing, etc.). In the context of this specification, the term "modification" encompasses cleavage, base editing, substitution, repair, etc. of a nucleic acid at a target site on either a double-stranded or single-stranded nucleic acid by a nuclease.

In some embodiments, the engineered CRISPR-Cas12i system comprises: (a) any of the engineered Cas12i effector proteins described herein (e.g., any of the engineered Cas12i nucleases or functional variants thereof; or a nickase, split Cas12i, transcription repressor, transcription activator, base editor or master editor based on the engineered Cas12i nuclease or functional variant thereof); and (b) a guide RNA comprising a guide sequence complementary to the target sequence, or one or more nucleic acids encoding the guide RNA; wherein, the engineered Cas12i effector protein and the guide RNA can form a CRISPR complex that specifically binds to a target nucleic acid comprising the target sequence and induces modification of the target nucleic acid (such as double-stranded or single-stranded cleavage, base editing, etc.). In some embodiments, the engineered CRISPR-Cas12i system comprises encoding the engineered Cas12i effector protein (such as an engineered Cas12i nuclease or a functional variant thereof, such as CasXX shown in SEQ ID NO.8) and/ or one or more nucleic acids of the guide RNA. In some embodiments, the engineered CRISPR-Cas12i system comprises an array of precursor guide RNAs that can be processed into multiple crRNAs, e.g., by the engineered Cas12i effector protein. In some embodiments, the engineered CRISPR-Cas12i system comprises one or more vectors encoding the engineered Cas12i effector protein and/or the guide RNA. In some embodiments, the engineered CRISPR-Cas12i system comprises a ribonucleoprotein (RNP) complex comprising the engineered Cas12i effector protein bound to the guide RNA.

The engineered CRISPR-Cas12i system of the present application may contain any suitable guide RNA. Guide RNA (gRNA) may comprise a guide sequence capable of hybridizing to a target sequence in a target nucleic acid of interest, such as a genomic site of interest in a cell. In some embodiments, the gRNA comprises a CRISPR RNA (crRNA) sequence comprising the guide sequence.

Generally, crRNA as described herein comprises direct repeat sequences and spacer sequences. In certain embodiments, the crRNA comprises, consists essentially of, or consists of a direct repeat sequence linked to a guide sequence or spacer sequence. In some embodiments, the crRNA comprises direct repeats, spacer sequences, and direct repeats (DR-spacer-DR), which are typical characteristics of precursor crRNA (pre-crRNA) configurations. In some embodiments, the crRNA comprises truncated direct repeat and spacer sequences that are typical characteristics of processed or mature crRNA. In some embodiments, the CRISPR-Cas12i effector protein forms a complex with an RNA guide sequence, and the spacer sequence directs the complex to sequence-specific binding to a target nucleic acid that are complementary (e.g., at least 70% complementary) to the spacer sequences.

In some embodiments, the guide RNA is a crRNA comprising a guide sequence. In some embodiments, the engineered CRISPR-Cas12i system comprises a precursor guide RNA array encoding a plurality of crRNAs. In some embodiments, the Cas12i effector protein cleaves the precursor guide RNA array to generate multiple crRNAs. In some embodiments, the engineered CRISPR-Cas12i system comprises precursor guide RNA arrays encoding multiple crRNAs, wherein each crRNA comprises a different guide sequence.

### Constructs and vectors

Also provided herein are constructs, vectors, and expression systems encoding any of the engineered Cas12i effector proteins described herein (e.g., engineered Cas12i nucleases or functional variants thereof). In some embodiments, the construct, vector or expression system further comprises one or more gRNA or crRNA arrays.

A "vector" is a composition of substance that comprises an isolated nucleic acid and can be used to deliver the isolated nucleic acid into the interior of a cell. Many vectors are known in the art, including, but not limited to, linear polynucleotides, polynucleotides associated with ionic or amphipathic compounds, plasmids, and viruses. Typically, a suitable vector will contain an origin of replication functional in at least one organism, a promoter sequence, convenient restriction endonuclease sites, and one or more selectable markers. The term "vector" should also be interpreted to comprise non-plasmid and non-viral compounds that facilitate the transfer of nucleic acids into cells, such as, for example, polylysine compounds, liposomes, and the like.

In some embodiments, the vector is a viral vector. Examples of viral vectors comprise, but are not limited to: adenovirus vectors, adeno-associated virus vectors, lentiviral vectors, retroviral vectors, vaccinia vectors, herpes simplex virus vectors, and derivatives thereof. In some embodiments, the vector is a phage vector. Viral vector technology is well known in the art and is described, for example, by Sambrook et al. (2001, Molecular Cloning: A Laboratory Manual, Cold Spring Harbor Laboratory, New York), and other virology and molecular biology manuals.

A number of virus-based systems have been developed for gene transfer into mammalian cells. For example, retroviruses provide a convenient platform for gene delivery systems. Heterologous nucleic acids can be inserted into vectors and packaged in retroviral particles using techniques known in the art. The recombinant virus can then be isolated and delivered to the engineered mammalian cells in vitro or ex vivo. Many retroviral systems are known in the art. In some embodiments, adenoviral vectors are used. Many adenoviral vectors are known in the art. In some embodiments, lentiviral vectors are used. In some embodiments, self-inactivating lentiviral vectors are used.

In certain embodiments, the vector is an adeno-associated virus (AAV) vector, such as AAV2, AAV8, or AAV9, adenovirus or adeno-associated virus can be administered in a single dose comprising at least 1×10⁵ particles (also known as particle units, pu). In some embodiments, the dosage amount is at least about 1 × 10⁶ particles, at least about 1 × 10⁷ particles, at least about 1 × 10⁸ particles, or at least about 1 × 10⁹ particles of adeno-associated virus. Delivery methods and dosage amounts are described, for example, in WO 2016205764 and US Patent No. 8,454,972, which are incorporated herein by reference in their entirety.

In some embodiments, the vector is a recombinant adeno-associated virus (rAAV) vector. For example, in some embodiments, modified AAV vector delivery may be used. Modified AAV vectors can be based on one or more of several capsid types, including AAV1, AV2, AAV5, AAV6, AAV8, AAV8.2, AAV9, AAV rh10, modified AAV vectors (e.g., modified AAV2, modified AAV3, modified AAV6) and pseudotyped AAV (e.g., AAV2/8, AAV2/5 and AAV2/6). Exemplary AAV vectors and techniques that can be used to produce rAAV particles are known in the art (see, e.g., Aponte-Ubillus et al. (2018) Appl. Microbiol. Biotechnol. 102(3): 1045-54; Zhong et al. (2012) J. Genet. Syndr. Gene Ther. S1: 008; West etal. (1987) Virology 160: 38-47 (1987); Tratschin et al. (1985) Mol. Cell. Biol. 5: 3251-60; U.S. Patent Nos. 4,797,368 and 5,173,414; International Publication Nos. WO2015/054653 and WO93/24641, each of which is incorporated herein by reference).

Any known AAV vector used to deliver Cas9 and other Cas proteins can be used to deliver the engineered Cas12i system of the present application.

In some embodiments, rAAV constructs can be administered to a subject enterally. In some embodiments, rAAV constructs can be administered parenterally to a subject. In some embodiments, rAAV particles can be administered subcutaneously, intraocularly, intravitreally, subretinally, intravenously (IV), intracerebroventricularly, intramuscularly, intrathecally (IT), intracisternally, intraperitoneally, via inhalation, topically, or by direct injection into one or more cells, tissues or organs. In some embodiments, rAAV particles can be administered to a subject by injection into the hepatic artery or portal vein.

Methods of introducing vectors into mammalian cells are known in the art. Vectors can be transferred into host cells by physical, chemical or biological means.

Physical methods used to introduce vectors into host cells comprise: calcium phosphate precipitation, lipofection, particle bombardment, microinjection, electroporation, etc. Methods of generating cells comprising vectors and/or exogenous nucleic acids are well known in the art. See, e.g., Sambrook et al.( 2001) Molecular Cloning: A Laboratory Manual, Cold Spring Harbor Laboratory, New York. In some embodiments, the vector is introduced into the cell by electroporation.

Biological methods for introducing heterologous nucleic acids into host cells comprise the use of DNA and RNA vectors. Viral vectors have become the most widely used method of inserting genes into mammalian, such as human cells.

Chemical methods used to introduce vectors into host cells comprise colloidal dispersion systems such as macromolecular complexes, nanocapsules, microspheres, beads, and lipid-based systems including oil-in-water emulsions, micelles, mixed micelles and liposomes. Exemplary colloidal systems useful as delivery vectors in vitro are liposomes (e.g., artificial membrane vesicles). In some embodiments, the engineered CRISPR-Cas12i system is delivered in the form of RNPs in nanoparticles.

In some embodiments, vectors or expression systems encoding the CRISPR-Cas12i system or components thereof comprise one or more selectable or detectable markers that provide a means of isolating or effectively selecting cells that contain and/or have been modified by the CRISPR-Cas12i system (e.g. in early and large-scale).

Reporter genes can be used to identify potentially transfected cells and assess the function of regulatory sequences. Typically, a reporter gene is a gene that is not present or expressed in the recipient organism or tissue, and the expression of the polypeptide encoded by it is demonstrated by some easily detectable property (such as enzymatic activity). Reporter gene expression is measured at appropriate times after introduction of DNA into recipient cells. Suitable reporter genes may comprise genes encoding luciferase, β-galactosidase, chloramphenicol acetyltransferase, secreted alkaline phosphatase, or green fluorescent protein genes (e.g. Ui-Tei et al. FEBS Letters 479: 79-82(2000)).

Other methods of confirming the presence of heterologous nucleic acids in a host cell comprise, for example, molecular biological assays well known to those skilled in the art, such as Southern and Northern blotting, RT-PCR and PCR; biochemical assays, for example detecting the presence or absence of specific peptides through immunological methods such as ELISA and Western blotting.

In some embodiments, the nucleic acid sequence encoding the engineered Cas12i effector protein and/or the guide RNA is operably linked to a promoter. In some embodiments, the promoter is an endogenous promoter of cells that have been engineered using the engineered CRISPR-Cas 12i system. For example, the nucleic acid encoding the engineered Cas12i effector protein can be knocked into downstream of an endogenous promoter in the genome of an engineered mammalian cell using any method known in the art. In some embodiments, the endogenous promoter is the promoter of an abundant protein such as β-actin. In some embodiments, the endogenous promoter is an inducible promoter, e.g., inducible by an endogenous activation signal of the engineered mammalian cell. In some embodiments, wherein the engineered mammalian cell is a T cell, the promoter is a T cell activation-dependent promoter (such as an IL-2 promoter, NFAT promoter, or NPκB promoter).

In some embodiments, the promoter is a heterologous promoter relative to the cell engineered using the engineered CRISPR-Cas12i system. A variety of promoters have been explored to express genes in mammalian cells, and any promoter known in the art can be used in this application. Promoters can be broadly classified as constitutive promoters or regulated promoters, such as inducible promoters.

In some embodiments, the nucleic acid sequence encoding the engineered Cas12i effector protein and/or the guide RNA is operably linked to a constitutive promoter. Constitutive promoters allow constitutive expression of heterologous genes (also called transgenes) in host cells. Exemplary constitutive promoters considered herein include, but are not limited to: cytomegalovirus (CMV) promoter, human elongation factor-1α (hEF1α), ubiquitin C promoter (UbiC), phosphoglycerol kinase promoter (PGK), the simian virus 40 early promoter (SV40) and the chicken β-actin promoter, which are coupled to the CMV early enhancer (CAG). In some embodiments, the promoter is a CAG promoter comprising a cytomegalovirus (CMV) early enhancer element, the promoter, the first exon and the first intron of the chicken β-actin gene, and the splice acceptor of the rabbit β-globin gene.

In some embodiments, the nucleic acid sequence encoding the engineered CRISPR-Cas12i effector protein and/or the guide RNA is operably linked to an inducible promoter. Inducible promoters are a type of regulated promoter. The inducible promoter can be induced by one or more conditions, such as physical conditions, microenvironment or physiological state of the host cell, inducing substances (i.e., inducers), or a combination thereof. In some embodiments, the induction conditions are selected from the group consisting of inducer, irradiation (e.g., ionizing radiation, light), temperature (e.g., heat), redox state, tumor environment, and the activation state of cells to be engineered through the CRISPR-Cas12i system. In some embodiments, the promoter is inducible by small molecule inducers such as compounds. In some embodiments, the small molecule is selected from the group consisting of: doxycycline, tetracycline, alcohol, metal, or steroid. Chemically induced promoters have been most extensively studied. Such promoters comprise promoters whose transcriptional activity is modulated by the presence or absence of small molecule chemicals such as doxycycline, tetracyclines, alcohols, steroids, metals, and other compounds. The doxycycline-inducible system with reverse tetracycline controlled transactivator (rtTA) and tetracycline response element promoter (TRE) is currently the most mature system. WO9429442 describes the tight control of gene expression in eukaryotic cells by tetracycline-responsive promoters. WO9601313 discloses tetracycline-regulated transcriptional regulators. Additionally, Tet technologies such as Tet-on systems have been described on a website such as TetSystems.com, for example. In this application, any known chemically regulated promoter may be used to drive expression of the engineered CRISPR-Cas12i protein and/or the guide RNA.

In some embodiments, the nucleic acid sequence encoding the engineered Cas12i effector protein is codon optimized.

In some embodiments, expression constructs are provided that comprise a codon-optimized sequence encoding the engineered Cas12i effector protein ligated to a BPK2104-ccdB vector. In some embodiments, the expression construct encodes a tag (e.g., a 10×His tag) operably linked to the C-terminus of the engineered Cas12i effector protein.

In some embodiments, each engineered split Cas12i construct encodes a fluorescent protein such as GFP or RFP. The reporter protein can be used to assess co-localization and/or dimerization of the engineered Cas12i protein, for example using microscopy. Nucleic acid sequences encoding engineered Cas12i effector proteins can be fused to nucleic acid sequences encoding additional components using sequences encoding self-cleaving peptides such as T2A, P2A, E2A or F2A peptides.

In some embodiments, expression constructs for mammalian cells (e.g., human cells) are provided, the constructs comprising nucleic acid sequences encoding the engineered Cas12i effector proteins. In some embodiments, the expression construct comprises a codon-optimized sequence encoding the engineered Cas 12i effector protein inserted into a pCAG-2A-eGFP vector, such that the Cas 12i protein is operably linked to eGFP. In some embodiments, a second vector is provided for expression of a guide RNA (e.g., crRNA or precursor crRNA array) in mammalian cells (e.g., human cells). In some embodiments, the sequence encoding the guide RNA is expressed in the pUC19-U6-i2-cr RNA vector backbone.

In some embodiments, one or more vectors expressing one or more elements of the CRISPR-Cas12i system are introduced into the host cell such that expression of the elements of the CRISPR-Cas12i system directs the formation of the nucleic acid targeting complex at one or more target sites. For example, the Cas12i nucleic acid-targeting effector enzyme and the nucleic acid-targeting guide RNA can each be operably linked to separate regulatory elements on separate vectors. The RNA of the nucleic acid targeting system can be delivered to a transgenic Cas12i nucleic acid targeting effector protein animal or mammal, for example, an animal or mammal that constitutively or inducibly or conditionally expresses the nucleic acid targeting effector protein; or otherwise expresses the nucleic acid targeting the effector protein or an animal or mammal having cells comprising the nucleic acid targeting effector protein, for example, by previously administering thereto one or more vectors encoding and expressing the nucleic acid targeting effector protein in vivo. Alternatively, two or more elements expressing the same or different regulatory elements can be combined in a single vector, with one or more additional vectors providing any components of the nucleic acid targeting system not contained in the first vector. Nucleic acid targeting system elements combined in a single vector may be arranged in any suitable orientation, for example one element is located 5' ("upstream") relative to a second element or 3' ("downstream") relative to a second element. The coding sequence of one element may be on the same or oppo-position strand and oriented in the same or oppo-position direction to the coding sequence of a second element. In some embodiments, a single promoter drives expression of transcripts encoding a Cas12i nucleic acid-targeting effector protein and a nucleic acid-targeting guide RNA that are embedded within one or more intronic sequences (e.g., each in a different intron, two or more in at least one intron, or all in a single intron). In some embodiments, the nucleic acid-targeting effector protein and the nucleic acid-targeting guide RNA can be operably linked to and expressed from the same promoter. Delivery vehicles, vectors, particles, nanoparticles, formulations and components thereof for expression of one or more elements of a nucleic acid targeting system are as used in aforementioned documents such as WO 2014/093622 (PCT/US2013/074667). In some embodiments, a vector comprises one or more insertion sites, such as restriction endonuclease recognition sequences (also known as "cloning positions"). In some embodiments, one or more insertion sites (e.g., about or greater than about 1, 2, 3, 4, 5, 6, 7, 8, 9, 10 or more insertion sites) are located in a or upstream and/or downstream of one or more sequence elements of one or more vectors. When multiple different guide sequences are used, a single expression construct can be used to target the nucleic acid targeting activity to multiple different corresponding target sequences within the cell. For example, a single vector may contain about or greater than about 1, 2, 3, 4, 5, 6, 7, 8, 9, 10, 15, 20 or more guide sequences. In some embodiments, about or greater than about 1, 2, 3, 4, 5, 6, 7, 8, 9, 10 or more such guide sequence-comprising vectors may be provided and optionally delivered to cell. In some embodiments, the vector comprises a regulatory element operably linked to an enzyme coding sequence encoding a nucleic acid targeting effector protein. The Cas12i nucleic acid-targeting effector protein or one or more nucleic acid-targeting guide RNAs may be delivered separately; and advantageously, at least one of these is delivered via a particle complex. The nucleic acid targeting effector protein mRNA can be delivered before the nucleic acid targeting guide RNA to allow time for expression of the Cas12i nucleic acid targeting effector protein. The nucleic acid-targeting effector protein mRNA can be administered 1-12 hours (preferably about 2-6 hours) before administering the nucleic acid-targeting guide RNA. Alternatively, nucleic acid-targeting effector protein mRNA and nucleic acid-targeting guide RNA can be administered together. Advantageously, the second booster dose of guide RNA can be administered 1-12 hours (preferably about 2-6 hours) after the initial administration of nucleic acid targeting effector protein mRNA + guide RNA. Additional administration of nucleic acids targeting effector protein mRNA and/or guide RNA may be useful in achieving the most efficient level of genome modification.

In some embodiments, a CRISPR-Cas12i system is provided, which comprises: (1) any one of the engineered Cas12i effector proteins (such as any one of the engineered Cas12i nucleases or functional variants thereof) or a polynucleotide encoding any of the engineered Cas12i effector proteins; and (2) crRNA or a polynucleotide encoding the crRNA, the crRNA comprising: (i) a spacer capable of hybridizing to a target sequence of a target DNA, and (ii) a direct repeat sequence linked to the spacer sequence capable of directing the engineered Cas12i effector protein to bind to the crRNA to form a CRISPR-Cas12i complex targeting the target sequence.

In some embodiments, a CRISPR-Cas12i system is provided, which comprises one or more vectors, the one or more vectors comprising: (1) a first regulatory element operably connected to the nucleotide sequence encoding any of the engineered Cas12i effector proteins (such as any of the engineered Cas12i nucleases or functional variants thereof); and (2) a second regulatory element operably linked to a polynucleotide encoding a crRNA comprising: (i) a spacer sequence capable of hybridizing to a target sequence of a target DNA, and (ii) a direct repeat sequence linked to the spacer sequence capable of directing the engineered Cas12i effector protein to bind to the crRNA to form a CRISPR-Cas12i complex targeting the target sequence; wherein the first regulatory element and the second regulatory element are located on the same or different vectors of the CRISPR-Cas12i system.

In certain embodiments, the first regulatory element and the second regulatory element are located on different vectors of the CRISPR-Cas12i system. In certain embodiments, the first regulatory element and the second regulatory element are located on the same vector of the CRISPR-Cas12i system. In certain embodiments, the first regulatory element and the nucleotide sequence encoding an engineered Cas 12i effector protein are upstream of the second regulatory element and the polynucleotide encoding crRNA. In certain embodiments, the first regulatory element and the nucleotide sequence encoding an engineered Cas12i effector protein are downstream of the second regulatory element and the polynucleotide encoding crRNA. In certain embodiments, the first regulatory element and the second regulatory element are the same. In certain embodiments, the first regulatory element and the second regulatory element are different.

In some embodiments, a CRISPR-Cas12i system is provided, comprising a vector comprising: (1) a polynucleotide encoding any of the engineered Cas12i effector proteins (such as any of the engineered Cas12i nuclease or functional variant thereof); (2) a polynucleotide encoding a crRNA the crRNA comprising: (i) a spacer sequence capable of hybridizing to a target sequence of a target DNA, and (ii) a direct repeat sequence linked to the spacer sequence capable of directing the engineered Cas12i effector protein to bind to the crRNA to form a CRISPR-Cas 12i complex targeting the target sequence; and (3) a regulatory element operably linked to the polynucleotide encoding the engineered Cas12i effector protein and the polynucleotide encoding the crRNA.

In certain embodiments, the vector comprises from 5' to 3' the regulatory element, a polynucleotide encoding the engineered Cas12i effector protein, and a polynucleotide encoding the crRNA. In certain embodiments, the vector comprises from 5' to 3' the regulatory element, a polynucleotide encoding the crRNA, and a polynucleotide encoding the engineered Cas12i effector protein. In certain embodiments, the polynucleotide encoding the crRNA and the polynucleotide encoding the engineered Cas12i effector protein are connected by a linker sequence, such as The polynucleotide sequence of encoding any one of P2A, T2A, E2A, F2A, BmCPV 2A, BmIFV 2A, (GS)n (SEQ ID NO.74), (GGGS)n (SEQ ID NO.75) and (GGGGS)n (SEQ ID NO.76) (wherein n is at least an integer of 1), or the polynucleotide sequence of encoding any one of IRES, SV40, CMV, UBC, EF1α, PGK and CAGG, or any combination thereof.

In some embodiments, components of the CRISPR-Cas12i system of the present invention can be delivered in various forms, such as DNA/RNA or RNA/RNA or protein RNA combinations. For example, an engineered Cas12i effector protein (such as any of the engineered Cas12i nucleases or functional variants thereof) can be delivered as a polynucleotide encoding DNA or a polynucleotide encoding RNA or as a protein. The guidance substance can be delivered as a DNA-encoded polynucleotide or RNA. Mixed delivery formats are available.

In some aspects, the present invention provides a method comprising delivering one or more polynucleotides, e.g., one or more vectors as described herein, one or more transcripts thereof, and/or one or more proteins transcribed therefrom, to a host cell.

### III. Method of use

The application provides a method for the detection of target nucleic acids or modifying nucleic acids in vitro, ex vivo or in vivo using any of the engineered Cas12i effector proteins described herein (such as any of the engineered Cas 12i nucleases or functional variants thereof) or the CRISPR-Cas 12i system, and a method for using the engineered Cas 12i effector protein or CRISPR-Cas 12i system for treatment (such as gene editing) or diagnosis. Also provided are uses of the engineered Cas12i effector proteins or CRISPR-Cas12i systems described herein for detecting or modifying nucleic acids in cells, and for treating or diagnosing diseases or conditions in a subject; and a use of the composition containing any one of the engineered Cas 12i effector proteins, or one or more components of the engineered CRISPR-Cas12i system in preparations of a medicament for detecting or modifying nucleic acids in cells and for treating or diagnosing diseases in a subject.

### Methods for detecting target nucleic acids in samples

The application also provides methods of detecting target nucleic acids using either the engineered Cas12i effector protein or the CRISPR-Cas12i system with improved activity. The use of Cas12i effector proteins as detection reagents exploits the finding that once activated by detection of target DNA, type V CRISPR/Cas proteins (e.g., Cas12i) can promiscuously cleave non-targeted single-stranded DNA (ssDNA or RNA, i.e., a single-stranded nucleic acid to which the guide sequence of guide RNA does not hybridize). Therefore, when target DNA (double-stranded or single-stranded) is present in the sample (e.g., in some cases above a threshold amount), the result is cleavage of single-stranded nucleic acids in the sample, which can be detected using any convenient detection method (for example, using tagged single-stranded detection nucleic acids such as DNA or RNA). Cas12i can cut ssDNA and ssRNA. For example, methods using Cas proteins as detection reagents are described in US 10253365 and WO2020/056924, which are incorporated herein by reference in their entirety.

In some embodiments, a method is provided for detecting target DNA (e.g., double-stranded or single-stranded) in a sample, comprising: (a) contacting the sample with: (i) any of the engineered Cas12i effector protein as described herein (such as any of the engineered Cas 12i nucleases or functional variants thereof); (ii) a guide RNA comprising a guide sequence that hybridizes to the target DNA; and (iii) a detection nucleic acid, which is single-stranded (i.e., a "single-stranded detection nucleic acid") and does not hybridize to the guide sequence of the guide RNA; and (b) measuring the detectable signal generated by cleaving the single stranded detection nucleic acid through the engineered Cas12i effector protein. In certain cases, the single-stranded detection nucleic acid comprises a fluorescent-emitting dye pair (e.g., the fluorescent-emitting dye pair is a fluorescence resonance energy transfer (FRET) pair, a quencher/fluorescent pair). In some cases, the target DNA is viral DNA (e.g., papillomavirus, hepatovirus, herpesvirus, adenovirus, poxvirus, parvovirus, etc.). In some embodiments, the single-stranded detection nucleic acid is DNA. In some embodiments, the single-stranded detection nucleic acid is RNA.

The disclosed method for detecting target DNA (single-stranded or double-stranded) in a sample can detect target DNA with high sensitivity. In some cases, methods of the present disclosure can be used to detect target DNA present in a sample comprising a plurality of DNAs (including the target DNA and a plurality of non-target DNAs), wherein the target DNA presents in one or more copies per 10⁷ non target DNAs (e.g., one or more copies per 10⁶ non-target DNAs, one or more copies per 10⁵ non-target DNAs, one or more copies per 10⁴ non-target DNAs, one or more copies per 10³ non-target DNAs, one or more copies per 10² non-target DNAs, one or more per 50 non-target DNAs copies, one or more copies per 20 non-target DNAs, one or more copies per 10 non-target DNAs, or one or more copies per 5 non-target DNAs). In some embodiments, the engineered Cas12i effector proteins described herein can detect target DNA with greater sensitivity than the reference Cas12i nuclease. In some embodiments, compared to the reference Cas 12i nuclease, the engineered Cas 12i effector protein can detect target DNA with 10%, 15%, 20%, 25%, 30%, 40%, 50%, 60%, 70%, 80%, 90%, 95%, or higher sensitivity.

### Modification method

In some embodiments, the present application provides a method of modifying a target nucleic acid comprising a target sequence, comprising contacting the target nucleic acid with any of the engineered CRISPR-Cas12i systems described herein. In some embodiments, the methods are performed in vitro. In some embodiments, the target nucleic acid is present in a cell. In some embodiments, the cell is a bacterial cell, yeast cell, mammalian cell, plant cell, or animal cell. In some embodiments, the methods are performed ex vivo. In some embodiments, the methods are performed in vivo. Target nucleic acid modifications include but are not limited to target nucleic acid single-stranded cleavage, double-stranded cleavage, base substitution, base insertion, base deletion, mutation (such as pathogenic mutation) sequence repair, etc.

In some embodiments, the target nucleic acid is cleaved or a target sequence in the target nucleic acid is altered by the engineered CRISPR-Cas12i system. In some embodiments, expression of the target nucleic acid is altered by the engineered CRISPR-Cas12i system. In some embodiments, the target nucleic acid is genomic DNA. In some embodiments, the target sequence is associated with a disease or condition, such as based on misexpression of the target sequence (e.g., overexpression, lack of expression, or expression of a disease-causing RNA or protein). In some embodiments, the engineered CRISPR-Cas12i system comprises an array of precursor guide RNAs encoding multiple crRNAs, wherein each crRNA comprises a different guide sequence.

In some embodiments, the present application provides a method of treating a disease or condition associated with a target nucleic acid in a cell of an individual, comprising modifying the target nucleic acid in the cell of the individual using any of the methods described herein, thereby treating the disease or condition. In some embodiments, the disease or condition is selected from the group consisting of cancer, cardiovascular disease, genetic disease (e.g., gene defective disease such as sickle cell anemia (SCD) or beta-thalassemia (TDT)), autoimmune diseases, metabolic diseases, neurodegenerative diseases, eye diseases, bacterial infections and viral infections.

The engineered CRISPR-Cas12i systems described herein can modify target nucleic acids in cells in a variety of ways, depending on the type of engineered Cas12i effector protein in the CRISPR-Cas12i system. In some embodiments, the methods induce site specific cleavage in the target nucleic acid. In some embodiments, the methods cleave genomic DNA in cells such as bacterial cells, plant cells, or animal cells (e.g., mammalian cells). In some embodiments, the method kills the cell by cleaving genomic DNA in the cell. In some embodiments the methods cleave viral nucleic acid in the cell.

In some embodiments, the method alters (e.g., increases or decreases) the expression level of the target nucleic acid in the cell. In some embodiments, the method uses an engineered Cas12i effector protein to increase the expression level of the target nucleic acid in the cell, for example, based on an enzymatically inactive Cas12i protein fused to a transactivation domain. In some embodiments, the method uses an engineered Cas12i effector protein to reduce the expression level of the target nucleic acid in the cell, for example, based on an enzymatically inactive Cas12i protein fused to a transcriptional repression domain. In some embodiments, the method uses an engineered Cas12i effector proteins to introduce epigenetic modifications into the target nucleic acid or target nucleic acids related proteins (e.g., proteins that bind to the target nucleic acid or its vicinity, such as transcription factors or histones) in the cell, for example, based on an enzymatically inactive Cas12i protein fused to an epigenetic modification domain. The engineered Cas12i systems described herein can be used to introduce additional modifications into the target nucleic acid, depending on the functional domains comprised by the engineered Cas12i effector protein.

In some embodiments, the method alters a target sequence in the target nucleic acid in the cell. In some embodiments, the methods introduce mutations into the target nucleic acid in the cell, such as mutating a disease-causing sequence to a non-pathogenic sequence (e.g., using any dCas12i of the present invention that is fused to a protein comprising an adenosine deaminase or a cytidine deaminase). In some embodiments, the methods use one or more endogenous DNA repair pathways, such as nonhomologous end joining (NHEJ) or homology-directed recombination (HDR), to repair double strand breaks induced in the target DNA in the cell, as a result of sequence-specific cleavage by the CRISPR complex. Exemplary mutations include, but are not limited to: insertions, deletions, substitutions, and frameshifts. In some embodiments, pegRNA is used to simultaneously cleave target sequences and provide DNA repair templates. In some embodiments, the method inserts donor DNA at the target site. In some embodiments, insertion of donor DNA results in the introduction of a selectable marker or reporter protein into the cell. In some embodiments, insertion of donor DNA results in knock-in of the gene. In some embodiments, insertion of donor DNA results in a knockout mutation. In some embodiments, insertion of donor DNA results in substitution mutations such as single nucleotide substitutions. In some embodiments, the methods induce phenotypic changes in cells.

In some embodiments, the engineered CRISPR-Cas12i system is used as part of a genetic circuit or to insert a genetic circuit into the genomic DNA of a cell. The inducer-controlled engineered split Cas12i effector proteins described herein are particularly useful as components of genetic circuits. Gene circuits can be used in gene therapy. Methods and techniques for designing and using genetic circuits are known in the art. Further reference may be made to, for example, Brophy, Jennifer AN, and Christopher A. Voigt. "Principles of genetic circuit design." Nature methods 11.5 (2014): 508.

The engineered CRISPR-Cas12i system described herein can be used to modify a variety of target nucleic acids. In some embodiments, the target nucleic acid is in a cell. In some embodiments, the target nucleic acid is genomic DNA. In some embodiments, the target nucleic acid is extrachromosomal DNA. In some embodiments, the target nucleic acid is exogenous to the cell. In some embodiments, the target nucleic acid is a viral nucleic acid such as viral DNA. In some embodiments, the target nucleic acid is a plasmid in a cell. In some embodiments, the target nucleic acid is a horizontally transferred plasmid. In some embodiments, the target nucleic acid is RNA.

In some embodiments, the target nucleic acid is an isolated nucleic acid such as isolated DNA. In some embodiments, the target nucleic acid is present in a cell-free environment. In some embodiments, the target nucleic acid is an isolated vector such as a plasmid. In some embodiments, the target nucleic acid is an isolated linear DNA fragment.

The methods described here are applicable to any suitable cell type. In some embodiments, the cell is a bacterial, yeast, fungal, algal, plant or animal cell (e.g., a mammalian cell, such as a human cell). In some embodiments, the cells are of natural origin such as cells isolated from tissue biopsy. In some embodiments, the cells are cells isolated from an in vitro cultured cell line. In some embodiments, the cells are from a primary cell line. In some embodiments, the cells are from an immortalized cell line. In some embodiments, the cells are genetically engineered cells.

In some embodiments, the cell is an animal cell of an organism selected from the group consisting of cattle, sheep, goat, horse, pig, deer, chicken, duck, goose, rabbit, and fish.

In some embodiments, the cell is a plant cell of an organism selected from the group consisting of corn, wheat, barley, oat, rice, soybean, oil palm, safflower, sesame, tobacco, flax, cotton, sunflower, pearl millet, corn, sorghum, oilseed rape, hemp, vegetable crops, feed crops, industrial crops, tree crops and biomass crops.

In some embodiments, the cells are mammalian cells. In some embodiments, the cells are human cells. In some embodiments, the human cell is a human embryonic kidney 293T (HEK293T or 293T) cell or a HeLa cell. In some embodiments, the cells are human embryonic kidney (HEK293T) cells. In some embodiments, the cells are mouse Hepa1-6 cells. In some embodiments, the mammalian cells are selected from the group consisting of immune cells, liver cells, tumor cells, stem cells, blood cells, neural cells, zygotes, muscle cells (e.g., cardiomyocytes), and skin cells.

In some embodiments, the cell is an immune cell selected from the group consisting of: cytotoxic T cells, helper T cells, natural killer (NK) T cells, iNK-T cells, NK-T-like cells, γδT cells, tumor infiltrating T cells and dendritic cells (DC) activated T cells. In some embodiments, the methods produce modified immune cells, such as CAR-T cells or TCR-T cells.

In some embodiments, the cell is an embryonic stem (ES) cell, an induced pluripotent stem (iPS) cell, a progenitor cell of a gamete, a gamete, a zygote, or a cell in an embryo.

The methods described herein can be used to modify target cells in vivo, ex vivo, or in vitro, and can be performed in a manner that alters the cells such that, once modified, descendants or cell lines of the modified cells retain the altered cell phenotype. The modified cells and progeny may be part of a multicellular organism, such as a plant or animal with ex vivo or in vivo applications such as genome editing and gene therapy.

In some embodiments, the methods are performed ex vivo. In some embodiments, after introducing the engineered CRISPR-Cas12i system into the cells, the modified cells (e.g., mammalian cells) are propagated ex vivo. In some embodiments, the modified cells are cultured to propagate for at least about any of 1 day, 2 days, 3 days, 4 days, 5 days, 6 days, 7 days, 10 days, 12 days, or 14 days . In some embodiments, the modified cells are cultured for no more than about any of 1 day, 2 days, 3 days, 4 days, 5 days, 6 days, 7 days, 10 days, 12 days, or 14 days. In some embodiments, the modified cells are further evaluated or screened to select cells with one or more desired phenotypes or properties.

In some embodiments, the target sequence is a sequence associated with a disease or condition. Exemplary diseases or conditions include, but are not limited to: cancer, cardiovascular disease, genetic diseases, autoimmune diseases, metabolic diseases, neurodegenerative diseases, eye diseases, bacterial infections, and viral infections. In some embodiments, the disease or condition is a genetic disease. In some embodiments, the disease or condition is a single gene disease or condition. In some embodiments, the disease or condition is a polygenic disease or condition.

In some embodiments, the target sequence has mutations compared to a wild-type sequence. In some embodiments, the target sequence has a single nucleotide polymorphism (SNP) associated with a disease or condition.

In some embodiments, the donor DNA inserted into the target nucleic acid encodes a biological product selected from the group consisting of: reporter proteins, antigen-specific receptors, therapeutic proteins, antibiotic resistance proteins, RNAi molecules, cytokines, kinases, antigens, antigen-specific receptors, cytokine receptors and suicide peptides. In some embodiments, the donor DNA encodes a therapeutic protein. In some embodiments, the donor DNA encodes a therapeutic protein useful in gene therapy. In some embodiments, the donor DNA encodes a therapeutic antibody. In some embodiments, the donor DNA encodes an engineered receptor, such as a chimeric antigen receptor (CAR) or an engineered TCR. In some embodiments, the donor DNA encodes a therapeutic RNA, such as a small RNA (e.g., siRNA, shRNA, or miRNA) or a long non-coding RNA (lincRNA).

The methods described herein can be used to perform multiplex gene editing or modulation at two or more (e.g., 2, 3, 4, 5, 6, 8, 10, or more) different target sites. In some embodiments, the methods detect or modify a plurality of target nucleic acids or target nucleic acid sequences. In some embodiments, the method comprises contacting the target nucleic acid with a guide RNA comprising multiple (e.g., 2, 3, 4, 5, 6, 8, 10, or more) crRNA sequences, wherein each crRNA comprises different target sequences.

Provided are also engineered cells comprising a modified target nucleic acid, which is generated using any of the methods described herein. The engineered cells can be used in cell therapy. Autologous or allogeneic cells can be used to prepare engineered cells using the methods described herein for cell therapy.

The methods described herein can also be used to generate isogenic lines of cells (e.g., mammalian cells) to study genetic variants.

There is also provided an engineered non-human animal comprising engineered cells as described herein. In some embodiments, the engineered non-human animal is a genome-edited non-human animal. The engineered non-human animals can be used as disease models.

Techniques for generating non-human genome-edited or transgenic animals are well known in the art and include, but are not limited to: pronuclear microinjection, viral infection, transformation of embryonic stem cells and induced pluripotent stem (iPS) cells. Detailed methods that can be used include, but are not limited to, the method described by Sundberg and Ichiki (2006, Genetically Engineered Mice Handbook, CRC Press) and the method described by Gibson (2004, A Primer of Genome Science 2nd ed. Sunderland, Mass.: Sinauer).

The engineered animal may be any suitable species, including but not limited to: cattle, horses, sheep, dogs, deer, felines, goats, pigs, primates, and less well understood mammals such as elephants , deer, zebra or camel.

### Treatment method

In some embodiments, there is provided the use of the aforementioned engineered CRISPR-Cas12i system in the preparation of a medicament for treating a disease or disorder associated with a target nucleic acid in a cell of an individual. In some embodiments, there is provided a method of using the aforementioned engineered CRISPR-Cas12i system to treat diseases or disorders associated with target nucleic acids in cells of an individual.

In some embodiments, the present invention provides a method of treating a disease in a subject (e.g., a human) in need thereof, comprising administering (e.g., intravenous injection or infusion)the CRISPR-Cas12i system of the present invention to the subject, which comprises: (1) any of the engineered Cas12i effector proteins (such as any of the engineered Cas12i nucleases or functional variants thereof), or a polynucleotide encoding the engineered Cas 12i effector proteins; and (2) crRNA, or a polynucleotide encoding said crRNA, the crRNA comprising: (i) a spacer sequence capable of hybridizing to a target sequence on a target nucleic acid associated with the disease, and (ii) a direct repeat sequence linked to the spacer sequence capable of directing the engineered Cas12i effector protein to bind to the crRNA to form a CRISPR-Cas 12i complex targeting the target sequence; wherein the spacer sequence and the hybridization of the target sequence mediates contact of the engineered Cas12i effector protein with the target sequence, causing the engineered Cas12i effector protein to modify (e.g., cleave or base edit) the target sequence, thereby treating the subject's disease. Wherein, any component of the CRISPR-Cas12i system can be delivered simultaneously, delivered sequentially, or delivered in any form of DNA/RNA, DNA/DNA, RNA/RNA, protein/RNA, or protein/DNA.

Further provided is a treatment method using any one of the methods for modifying target nucleic acids in cells as described in this article. In some embodiments, the present application provides a method of treating a disease or condition associated with a target nucleic acid in a cell of an individual, comprising contacting the target nucleic acid with any of the engineered CRISPR-Cas12i systems described herein, wherein the guide sequence of the guide RNA is complementary to the target sequence of the target nucleic acid, wherein the engineered Cas12i effector protein and the guide RNA associate with each other to bind to the target nucleic acid to modify (e.g., cleave or base substitute) the target nucleic acid, thereby allowing the disease or condition to be treated. In some embodiments, mutations (e.g., knock-out or knock-in mutations) are introduced into the target nucleic acid. In some embodiments, expression of the target nucleic acid is enhanced. In some embodiments, expression of the target nucleic acid is inhibited. In some embodiments, the present application provides a method of treating a disease or condition in an individual, comprising administering to the individual an effective amount of any of the engineered CRISPR-Cas12i systems described herein and a donor encoding a therapeutic agent DNA (e.g., a non-pathogenic native sequence used as a repair template), wherein the guide sequence of the guide RNA is complementary to the target sequence of the target nucleic acid of the individual, and wherein the engineered Cas12i effector protein and the guide RNA are complementary to each other to bind to the target nucleic acid and insert donor DNA into the target sequence, thereby allowing the disease or condition to be treated.

In some embodiments, the present application provides a method of treating a disease or condition in an individual, comprising administering to the individual an effective amount of an engineered cell comprising a modified target nucleic acid, wherein the engineered cell is prepared by contacting the cell with any one of the engineered CRISPR-Cas12i systems described herein, wherein the guide sequence of the guide RNA is complementary to the target sequence of the target nucleic acid, and wherein the engineered Cas12i effector protein and the guide RNA associate with each other to bind to the target nucleic acid to modify the target nucleic acid. In some embodiments, the engineered cells are immune cells. In some embodiments, the engineered cells are stem cells (e.g., hematopoietic stem cells, neural stem cells). In some embodiments, the engineered cells are neural cells. In some embodiments, the individual is a human. In some embodiments, the individual is an animal, such as a model animal such as a rodent, pet, or farm animal. In some embodiments, the individual is a mammal, such as a cat, dog, rabbit, hamster, etc.

In some embodiments, the disease or condition is selected from the group consisting of: cancer, cardiovascular disease, genetic disease, autoimmune disease, metabolic disease, neurodegenerative disease, eye disease, bacterial infection, and viral infection. In some embodiments, the target nucleic acid is PCSK9. In some embodiments, the disease or condition is cardiovascular disease. In some embodiments, the disease or condition is coronary artery disease. In some embodiments, the methods reduce cholesterol levels in an individual. In some embodiments, the methods treat diabetes in an individual.

Conditions or diseases that the CRISPR-Cas12i system of the present invention can be used to treat include, but are not limited to, cystic fibrosis, hereditary angioedema, diabetes, progressive duchenne muscular dystrophy, Becker muscular dystrophy, α-1-antitrypsin deficiency, Pompe disease, myotonic dystrophy, Huntington's disease, fragile X syndrome, Friedreich's ataxia, amyotrophic lateral sclerosis, frontotemporal dementia, hereditary chronic kidney disease, high lipidemia, hypercholesterolemia, Leber's congenital amaurosis, sickle cell disease or β-thalassemia, etc. In certain embodiments, the disorder or disease is transthyretin amyloidosis, such as transthyretin-related wild-type amyloidosis (ATTRwt), hereditary transthyretin amyloidosis transthyretin-related hereditary amyloidosis (ATTRm), familial amyloid polyneuropathy (FAP, ATTR-PN), or familial amyloid cardiomyopathy (FAC, ATTR-CM). In certain embodiments, the disorder or disease is transthyretin instability caused by mutation or abnormal expression (e.g., high expression) of the TTR gene. In certain embodiments, the disorder or disease is other disorders or diseases caused by mutation or abnormal expression (e.g., high expression) of the TTR gene, or a derivative disorder or disease.

### Delivery method

In some embodiments, the engineered CRISPR-Cas 12i systems described herein, or components thereof, nucleic acid molecules thereof, or nucleic acid molecules encoding or providing components thereof, can be delivered to host cells via a variety of delivery systems, such as plasmids or viruses (for example, any of the vectors described in the "Constructs and Vectors" section above). In some embodiments or methods, the engineered CRISPR-Cas12i system can be delivered by other methods, such as nuclear transfection or electroporation of a ribonucleoprotein complex composed of the engineered Cas12i effector protein and one or more homologous RNA guiding sequences.

In some embodiments, delivery is via nanoparticles or exosomes.

In some embodiments, paired Cas12i nickase complexes can be delivered directly using nanoparticles or other direct protein delivery methods such that complexes comprising two paired crRNA elements are co-delivered. Additionally, proteins can be delivered to cells via viral vectors or directly, followed by direct delivery of a CRISPR array comprising two paired spacers for double nicking. In certain cases, for direct RNA delivery, the RNA can be conjugated to at least one sugar moiety such as N-acetylgalactosamine (GalNAc) (particularly triantennary GalNAc).

In some embodiments or methods, the engineered CRISPR-Cas12i system can be delivered using any delivery method suitable for the disease being treated, such as delivery by intravenous injection or infusion, or local delivery at the diseased position (e.g., tumor internal delivery). Suitable routes of administration of the engineered CRISPR-Cas12i systems described herein include, but are not limited to: topical, subcutaneous, transdermal, intradermal, intralesional, intraarticular, intraperitoneal, intravesical, transmucosal, gingival, intradental, cochlear, intratympanic, intraorganal, epidural, intrathecal, intramuscular, intravenous, intravascular, intraosseous, periocular, intratumoral, intracerebral, and intracerebroventricular administration. In some embodiments, the engineered CRISPR-Cas12i systems described herein are administered to a subject by injection, through a catheter, through a suppository, or through an implant, the implant is a porous, non-porous, or gel-like material, including membranes such as sialic acid membranes, or fibers.

### IV. Kits and products

Compositions, kits, unit reagent, and product comprising one or more components of any of the engineered Cas12i nucleases or functional variants thereof, engineered Cas12i effector proteins, or engineered CRISPR-Cas 12i systems described herein are also provided.

In some embodiments, there is provided a kit, comprising: one or more AAV vectors encoding any of an engineered Cas12i nuclease or functional variant thereof, an engineered Cas12i effector protein, or an engineered CRISPR-Cas12i system as described herein. In some embodiments, the kit further comprises one or more guide RNAs (or DNA or vectors encoding them). In some embodiments, the kit further comprises donor DNA. In some embodiments, the kit further comprises cells such as human cells.

The kit may comprise one or more additional components, such as containers, reagents, media, cytokines, buffers, antibodies, etc., to allow propagation of the engineered cells. The kit may also comprise a device for administering the composition.

The kits may also comprise instructions for using the engineered CRISPR-Cas12i systems described herein, such as methods of detecting or modifying target nucleic acids. In some embodiments, the kit comprises instructions for treating or diagnosing a disease or condition. Instructions regarding the use of the kit components typically comprise information regarding the amounts, schedules, and routes of administration for the deliberate treatment. The container may be in unit dose form, bulk packaging (e.g., multi-dose packaging), or subunit dose form. For example, a kit comprising a sufficient dose of a composition disclosed herein may be provided to provide effective treatment of an individual over an extended period of time. Kits may also comprise multiple unit doses of the composition and instructions for use, packaged in quantities sufficient for storage and use in pharmacies (e.g., hospital pharmacies and compounding pharmacies).

The kit of the present invention is in suitable packaging. Suitable packaging includes, but is not limited to: vials, bottles, jars, flexible packaging (e.g., sealed mylar or plastic bags), etc. Kits may optionally provide additional components such as buffers and explanatory information. Accordingly, the present application also provides an article of manufacture including a vial (e.g., a sealed vial), a bottle, ajar, a flexible packaging, and the like.

The products may comprise a container and a label or package insert on or adhesive to the container. Suitable containers comprise, for example, bottles, vials, syringes, and the like. The container can be formed from a variety of materials, such as glass or plastic. Typically, the container comprises a composition effective to treat a disease or disorder described herein, and may have a sterile access port (e.g., the container may be an intravenous solution bag or a vial with a stopper pierceable by a hypodermic needle). The label or package insert indicates that the composition is used to treat a specific condition in an individual. The label or package insert will further comprise instructions for administering the composition to an individual.

Package insert refers to the instructions typically comprised in the commercial packaging of a therapeutic product that comprise information regarding the indications, usage, dosage, administration, contraindications, and/or warnings regarding the use of such therapeutic product.

Additionally, the products may comprise a second container comprising a pharmaceutically acceptable buffer, such as bacteriostatic water for injection (BWFI), phosphate buffered saline, Ringer's solution, and dextrose solution. From a commercial and user perspective, it can also comprise other materials including other buffers, diluents, filters, needles and syringes. If necessary, solubilizers and local anesthetics (e.g., lidocaine) may also be comprised to relieve pain at the injection position.

Typically, the ingredients are presented individually in unit dosage form or mixed together , for example, as a dry lyophilized powder or anhydrous concentrate in a sealed container such as an ampoule or sachet displaying the active dose. When the drug is administered by infusion, it may be dispensed from an infusion bottle filled with sterile pharmaceutical grade water or saline. When the pharmaceutical composition is administered by injection, ampoules of sterile water for injection or saline may be provided so that the ingredients may be mixed prior to administration.

### Examples

Specific Examples of the present invention will be described in more detail below with reference to the accompanying drawings. Although specific Examples of the present invention are shown in the accompanying drawings, it should be understood that the present invention can be implemented in various forms and should not be limited to the Examples set forth herein. Rather, these Examples are provided in order to have a more thorough understanding of the present invention and to fully convey the scope of the present invention to those skilled in the art.

### Example 1: Replace the amino acids interacting with the PAM in the reference Cas12i2 enzyme with positively charged amino acids, and verify its gene editing efficiency.

### Plasmid construction

The coding sequence of Cas12i2 was codon optimized (human) and synthesized. Variants of Cas proteins were produced by PCR-based site directed mutagenesis. The specific method was to divide the DNA sequence design of Cas12i2 protein into two parts centered on the mutation site, design two pairs of primers to amplify the two parts of the DNA sequence respectively, and introduce the sequence that needs to be mutated on the primers, and finally load the two fragments into pCAG-2A-eGFP vector (with penicillin resistance) by Gibson clone. The combination of mutants was constructed by splitting the DNA of Cas 12i2 protein into multiple segments and using PCR and Gibson clone. The position of the mutant was determined by analyzing the structural information of Cas 12i2 using protein structure visualization software commonly used in the art (for example, PyMol, Chimera and other software can be used). For the structural information of Cas12i2, please refer to PDB: 6LTU, 6LTR, 6LU0, 6LTP). Cas12i2 effector protein was expressed in human 293T cells through the pCAG-2A-eGFP vector. DNA encoding Cas12i2 protein was inserted between XmaI and NheI. A vector expressing the crRNA of Cas12i2 in 293T was constructed by ligating the annealed oligonucleotide comprising the target sequence into the BasI-digested pUC19-U6-i2-crRNA backbone. The nucleotide sequence encoding DR was SEQ ID NO.59.

### Cell culture, transfection, and fluorescence-activated cell sorting (FACS)

HEK293T cells were cultured in DMEM (Gibco) comprising 1% penicillin-streptomycin (Gibco) and 10% fetal calf serum (Gibco). Cells were seeded in 24-well cell culture dishes (Corning) for 16 hours until cell density reached 70%. By using Lipofectamine 3000 (Invitrogen), 600 ng of plasmid encoding Cas 12i2 protein and 300 ng of plasmid encoding crRNA were transfected into each 24-well cell culture dish. After approximately 68 hours of transfection, HEK293T cells to be subjected to fluorescence-activated cell sorting (FACS) were digested with trypsin-EDTA (0.05%) (Gibco). Cell sorting was performed using MoFlo XDP (Beckman Coulter) with GFP channel.

### Targeted deep sequencing analysis for genome modification

FACS-sorted GFP-positive HEK293T cells were lysed with buffer L and incubated at 55°C for 3 h and then at 95°C for 10 min. PCR amplification of dsDNA fragments comprising target sites in different genomic sites was performed using corresponding primers. For targeted deep sequencing, cell lysate was directly used as a template to directly amplify the target site through barcoded PCR. PCR products were purified and pooled into several libraries for high-throughput sequencing. The frequency (%) of indels was analyzed using CRISPResso2 software by calculating the ratio of reads comprising indels. In this application, the index of indel frequency (%) was uniformly used to compare and analyze gene editing efficiency. Reads with less than 0.05% of complete reads are discarded.

### Example 1-A Select Four Engineered Cas12i2 with Single Amino Acid Substitution

Engineered Cas12i2 enzymes with a single mutation in the amino acid sequence were expressed according to the method described in Example 1. The preferred amino acid substitution methods and their corresponding gene editing efficiencies are shown in Fig. 1a, Fig. 1b and Table 1. We firstly selected 10 amino acids within 9 Å of Cas12i2 from the PAM DNA: E176, E178, Y226, A227, N229, E237, K238, K264, T447, E563, and conducted point mutation testing of arginine (R). As shown in Fig. 1a and Table 1, by comparing the gene editing efficiency of these Cas12i mutants with wild-type Cas12i2 (SEQ ID NO.1) in 293T cells at two genomic sites: CCRS-3 and RNF2-7, the results showed that Cas12i mutants with the following amino acid substitutions: E176R, K238R, T447R and E563R can effectively improve gene editing efficiency (Fig. 1a showed that the four single amino acid substitutions of E176R, K238R, T447R and E563R all obtained indel rates higher than about 10% (this was the gene editing efficiency of the reference enzyme for CCR5-3) and higher than about 12% (this was the gene editing efficiency of the reference enzyme for RNF2-7), which was significantly better than other single amino acid substitutions scheme), the remaining six mutants did not help improve gene editing efficiency, or even reduced it. The coding sequences of the crRNA spacer sequences for CCR5-3 and RNF2-7 used here were shown in SEQ ID NO.60 and 61, respectively.

### Example 1-B Comparison of engineered Cas12i2 with multiple preferred amino acid substitutions simultaneously

The engineered Cas 12i2 enzymes whose amino acid sequences have two or more preferred amino acid substitutions were expressed according to the method described in Example 1. The comparison of their combination methods and their gene editing efficiencies was shown in Table 1 and Fig. 1b. We combined the point mutations in the four mutants E176R, K238R, T447R and E563R screened in Example 1-A that can improve efficiency. As shown in Fig. 1b and Table 1, by comparing the gene editing efficiency of these mutants with wild-type Cas12i2 in 293T cells at 3 genomic sites: CCR5-3, CCR5-5, and RNF2-7, we found point mutations after combination, mutants with further improved efficiency can be obtained. Especially when four mutations were combined together (E176R+K238R+T447R+E563R), a combination of mutations with optimal efficiency can be obtained. The coding sequences of the crRNA spacer sequences for CCR5-3, RNF2-7 and CCR5-5 used here are shown in SEQ ID NO.60, 61 and 62 respectively.

**Table 1 Summary of results (gene editing efficiency) of Example 1**

| Single amino acid substitution and combined substitutions | Gene editing efficiency (indel rate at CCR5-3) | Gene editing efficiency (indel rate at RNF2-7) |
|---|---|---|
| Reference enzyme (Cas12i2; SEQ ID NO.1) | 10.44 | 11.70 |
| E178R | 0.15 | 0.00 |
| Y226R | 0.19 | 0.00 |
| A227R | 0.35 | 0.29 |
| N229R | 8.68 | 5.37 |
| E237R | 0.26 | 0.07 |
| K264R | 15.83 | 19.35 |
| **E176R** | 24.07 | 29.16 |
| **K238R** | 15.83 | 19.35 |
| **T447R** | 28.61 | 43.71 |
| **E563R** | 55.49 | 68.30 |
| E176R+K238R | 28.76 | 34.76 |
| E176R+T447R | 49.47 | 59.64 |
| E176R+E563R | 74.78 | 75.94 |
| K238R+T447R | 37.52 | 50.41 |
| K238R+E563R | 64.08 | 73.38 |
| E176R+K238R+T447R | 56.42 | 63.16 |
| E176R+K238R+E563R | 73.17 | 74.50 |
| E176R+T447R+E563R | 68.39 | 73.89 |
| E176R+K238R+T447R+E563R | 73.45 | 86.29 |

### Example 2: Replace the amino acids involved in opening the double-stranded DNA in the reference Cas12i2 enzyme with amino acids with an aromatic ring, and verify their gene editing efficiencies respectively.

### Plasmid construction

Variants of Cas proteins were produced by PCR-based site directed mutagenesis. The specific method was to divide the DNA sequence design of Cas12i2 protein into two parts centered on the mutation site, design two pairs of primers to amplify the two parts of the DNA sequence respectively, and introduce the sequence that needs to be mutated on the primers, and finally load the two fragments into pCAG-2A-eGFP vector by Gibson clone. The position of the amino acid substitution was determined by analyzing the structural information of Cas12i2 using protein structure visualization software commonly used (for example, PyMol, Chimera and other software can be used). For the structural information of Cas 12i2, please refer to PDB: 6LTU, 6LTR, 6LU0, 6LTP). Cas12i2 effector protein was expressed in human 293T cells through the pCAG-2A-eGFP vector. DNA encoding Cas12i2 protein was inserted between XmaI and NheI. A vector expressing the crRNA of Cas 12i2 in 293T was constructed by ligating the annealed oligonucleotide comprising the target sequence into the BasI-digested pUC19-U6-i2-crRNA backbone. The nucleotide sequence encoding DR was SEQ ID NO.59.

### Cell culture, transfection, and fluorescence-activated cell sorting (FACS)

HEK293T cells were cultured in DMEM (Gibco) comprising 1% penicillin-streptomycin (Gibco) and 10% fetal calf serum (Gibco). Cells were seeded in 24-well cell culture dishes (Corning) for 16 hours until cell density reached 70%. By using Lipofectamine 3000 (Invitrogen), 600 ng of plasmid encoding Cas 12i2 protein and 300 ng of plasmid encoding crRNA were transfected into each 24-well cell culture dish. After approximately 68 hours of transfection, HEK293T cells to be subjected to fluorescence-activated cell sorting (FACS) were digested with trypsin-EDTA (0.05%) (Gibco). Cell sorting was performed using MoFlo XDP (Beckman Coulter) with GFP channel.

### Targeted deep sequencing analysis for genome modification

FACS-sorted GFP-positive HEK293T cells were lysed with buffer L and incubated at 55°C for 3 h and then at 95°C for 10 min. PCR amplification of dsDNA fragments comprising target sites in different genomic sites was performed using corresponding primers. For targeted deep sequencing, cell lysate was directly used as a template to directly amplify the target site through barcoded PCR. PCR products were purified and pooled into several libraries for high-throughput sequencing. The frequency (%) of indels was analyzed using CRISPResso2 software by calculating the ratio of reads comprising indels. Reads with less than 0.05% of complete reads are discarded.

Firstly, we selected the amino acids involved in opening the double-stranded DNA in the Cas12i2 enzyme: Q163 and N164 to conduct point mutation testing of amino acids with an aromatic ring (Y, F, W). As can be seen from Fig. 2 and Table 2, by comparing the gene editing efficiency of these mutants with wild-type Cas12i2 in 293T cells at 3 genomic sites: CCRS-3, CCR5-5, RNF2-7, we found that, five mutants: Q163W, Q163Y, Q163F, N164Y, and N164F can effectively improve gene editing efficiency (at least at one genomic site). In particular, N164Y and N164F demonstrated excellent gene editing efficiency at three genomic sites. N164W has no effect on improving gene editing efficiency compared to the reference enzyme.

**Table 2 Summary of results (gene editing efficiency) of Example 2**

| Single amino acid substitution scheme | Gene editing efficiency (%) (indel rate at CCR5-3) | Gene editing efficiency (%) (indel rate at RNF2-7) | Gene editing efficiency (%) (indel rate at CCR5-5) |
|---|---|---|---|
| **Q163W** | 22.07 | 16.97 | 10.64 |
| **Q163Y** | 12.51 | 9.70 | 5.47 |
| **Q163F** | 17.42 | 12.22 | 7.09 |
| N164W | 8.36 | 7.45 | 3.97 |
| **N164Y** | 29.72 | 43.77 | 21.66 |
| **N164F** | 28.89 | 45.48 | 22.99 |
| Reference enzyme (Cas12i2) | 10.44 | 11.70 | 9.79 |

### Example 3: Replace the amino acids located in the RuvC domain and interacting with the single-stranded DNA substrate in the reference Cas12i nuclease with positively charged amino acids, and verify its gene editing efficiency

### Plasmid construction

Variants of Cas proteins were produced by PCR-based site directed mutagenesis. The specific method was to divide the DNA sequence design of Cas12i2 protein into two parts centered on the mutation site, design two pairs of primers to amplify the two parts of the DNA sequence respectively, and introduce the sequence that needs to be mutated on the primers, and finally load the two fragments into pCAG-2A-eGFP vector by Gibson clone. The combination of mutants was constructed by splitting the DNA of Cas 12i2 protein into multiple segments and using PCR and Gibson clone. The position of the mutant was determined by analyzing the structural information of Cas12i2 using protein structure visualization software commonly used (for example, PyMol, Chimera and other software can be used). For the structural information of Cas12i2, please refer to PDB: 6LTU, 6LTR, 6LU0, 6LTP). The ssDNA substrate displayed in these Cas12i2 structures was only 5nt. In order to get the information about the interaction between longer ssDNA and Cas12i2, we made homologous alignment between the structure of Cas12i1 (PDB id: 6w5c, 6w62 and 6w64; Zhang h. et al. Nature Structural & Molecular Biology 27, 1069-1076 (2020)) and the structure of cas12i2, so that the ssDNA substrate (9nT) in the structure of Cas12i1 was placed into the ruvc catalytic pocket of Cas12i2, and further looked for the amino acids within 9Å through this model. Cas12i2 effector protein was expressed in human 293T cells through the pCAG-2A-eGFP vector. DNA encoding Cas12i2 protein was inserted between XmaI and NheI. A vector expressing the crRNA of Cas12i2 in 293T was constructed by ligating the annealed oligonucleotide comprising the target sequence into the BasI-digested pUC19-U6-i2-crRNA backbone. The nucleotide sequence encoding DR was SEQ ID NO.59. The coding sequences of the crRNA spacer sequences for CCR5-3 and RNF2-7 are shown in SEQ ID NOs. 60 and 61, respectively.

### Cell culture, transfection, and fluorescence-activated cell sorting (FACS)

HEK293T cells were cultured in DMEM (Gibco) comprising 1% penicillin-streptomycin (Gibco) and 10% fetal calf serum (Gibco). Cells were seeded in 24-well cell culture dishes (Corning) for 16 hours until cell density reached 70%. By using Lipofectamine 3000 (Invitrogen), 600 ng of plasmid encoding Cas 12i2 protein and 300 ng of plasmid encoding crRNA were transfected into each 24-well cell culture dish. After approximately 68 hours of transfection, HEK293T cells to be subjected to fluorescence-activated cell sorting (FACS) were digested with trypsin-EDTA (0.05%) (Gibco). Cell sorting was performed using MoFlo XDP (Beckman Coulter) with GFP channel.

### Targeted deep sequencing analysis for genome modification

FACS-sorted GFP-positive HEK293T cells were lysed with buffer L and incubated at 55°C for 3 h and then at 95°C for 10 min. PCR amplification of dsDNA fragments comprising target sites in different genomic sites was performed using corresponding primers. For targeted deep sequencing, cell lysate was directly used as a template to directly amplify the target site through barcoded PCR. PCR products were purified and pooled into several libraries for high-throughput sequencing. The frequency (%) of indels was analyzed using CRISPResso2 software by calculating the ratio of reads comprising indels. Reads with less than 0.05% of complete reads are discarded.

We replaced the amino acids located in the RuvC domain and interacting with the single-stranded DNA substrate in the reference Cas12i2 enzyme with positively charged amino acids. The gene editing efficiencies of these mutants and wild-type Cas12i2 at CCR5-3 and/or RNF2-7 genomic sites were compared in 293T cells. As shown in Fig. 3a and Table 3, N391R, I926R, and G929R can effectively improve gene editing efficiency (at least at one genomic site). Wherein, I926R demonstrated excellent gene editing efficiency at both genomic sites. As shown in Figs. 3b, 3c and Table 3, many mutants were able to effectively increase gene editing efficiency (at least at one genomic site). Wherein, the ranking of single amino acid substitution schemes with improved gene editing efficiency is: D362R>E323R>Q425R>N925R>other mutants with improved efficiency.

We combined the point mutations in the four mutants E323R, D362R, Q425R and I926R screened in Figs. 3a, 3b, and 3c that can improve efficiency. As shown in Fig. 3d and Table 3, by comparing the gene editing efficiency of these mutants with wild-type Cas12i2 in 293T cells at 2 genomic sites: CCR5-3, RNF2-7, we found that after the combination of point mutations, we were able to obtain mutants (at least in one site) with further improved gene editing efficiency.

We combined the point mutations or combinations in some of the mutants that can improve efficiency screened in Figs. 3a, 3b, 3c, and 3d with the flexible region mutations I926G and L439 (L+GG). As shown in Fig. 3e and Table 3, by comparing the gene editing efficiency of these mutants with wild-type Cas12i2 at the RNF2-7 site in 293T cells, we found that after the combination of point mutations, we were able to obtain mutants with further improved efficiency, such as N925R+I926G, I926R+L439(L+GG). It can be seen that further introducing flexible region mutations can increase the gene editing efficiency of Cas12i mutants.

**Table 3 Summary of results (gene editing efficiency) of Example 3**

| Single amino acid substitution scheme and the combination substitution scheme | Gene editing efficiency (%) (indel rate at CCR5-3) | Gene editing efficiency (%) (indel rate at RNF2-7) |
|---|---|---|
| Reference enzyme (Cas12i2) | 10.44 | 11.70 |
| N390R | 1.64 | 9.13 |
| **N391R** | **16.92** | **28.91** |
| F392R | 0.20 | 0.71 |
| L751R | 0.08 | 0.74 |
| E755R | 1.95 | 6.84 |
| N840R | 8.29 | 19.68 |
| N848R | 0.09 | 0.13 |
| S851R | 0.75 | 9.40 |
| A856R | 0.10 | 0.86 |
| Q885R | 0.13 | 0.48 |
| M897R | 0.26 | 1.15 |
| **I926R** | **66.51** | **73.60** |
| **G929R** | **32.35** | **40.67** |
| Y932R | 0.06 | 0.44 |
| **E323R** | **46.44** | **55.50** |
| L327R | 28.57 | 40.21 |
| V355R | 21.62 | 29.88 |
| G359R | 15.50 | 25.12 |
| G360R | 19.92 | 29.03 |
| K361R | 16.79 | 15.15 |
| **D362R** | **53.15** | **62.88** |
| Q414R | 14.77 | 22.41 |
| K421R | 12.37 | 13.41 |
| **Q425R** | **32.51** | **45.73** |
| S650R | 1.48 | 3.81 |
| E652R | 2.05 | 3.80 |
| K705R | 15.11 | 16.78 |
| K708R | 9.98 | 15.64 |
| E709R | 0.65 | 5.68 |
| S752R | 4.26 | 6.64 |
| **N925R** | **30.39** | **42.01** |
| T928R | 3.38 | 3.46 |
| E323R+D362R | 73.83 | 84.37 |
| E323R+Q425R | 45.80 | 64.08 |
| E323R+I926R | 80.58 | 86.10 |
| Q425R+I926R | 70.05 | 81.84 |
| E323R+D362R+Q425R | 60.76 | 74.79 |
| E323R+D362R+I926R | 78.08 | 83.50 |
| E323R+Q425R+I926R | 73.44 | 83.19 |
| E323R+D362R+Q425R+I926R | 80.51 | 86.62 |
| **N925R** | | **33.13** |
| I926G | | 43.68 |
| L439(L+GG)* | | 31.22 |
| **D362R+I926R** | | 63.87 |
| **N925R+I926R** | | 49.84 |
| **N925R**+I926G | | 62.42 |
| **D362R+N925R+I926R** | | 72.99 |
| **D362R+N925R**+I926G | | 55.06 |
| **I926R**+L439(L+GG) | | 80.53 |
| **E323R+D362R**+I926G | | 81.93 |

| | | |
|---|---|---|
| *L439 (L+GG) means that two glycines are inserted after amino acid No. 439. | | |

### Example 4: Replace the amino acids interacting with the DNA-RNA double helix in the reference Cas12i2 enzyme with positively charged amino acids and verify its gene editing efficiency

### Plasmid construction

Variants of Cas proteins were produced by PCR-based site directed mutagenesis. The specific method was to divide the DNA sequence design of Cas12i2 protein into two parts centered on the mutation site, design two pairs of primers to amplify the two parts of the DNA sequence respectively, and introduce the sequence that needs to be mutated on the primers, and finally load the two fragments into pCAG-2A-eGFP vector by Gibson clone. The combination of mutants was constructed by splitting the DNA of Cas 12i2 protein into multiple segments and using PCR and Gibson clone. The position of the mutant was determined by analyzing the structural information of Cas12i2 using protein structure visualization software commonly used (for example, PyMol, Chimera and other software can be used). For the structural information of Cas12i2, please refer to PDB: 6LTU, 6LTR, 6LU0, 6LTP). Cas12i2 effector protein was expressed in human 293T cells through the pCAG-2A-eGFP vector. DNA encoding Cas12i2 protein was inserted between XmaI and NheI. A vector expressing the crRNA of Cas12i2 in 293T was constructed by ligating the annealed oligonucleotide comprising the target sequence into the BasI-digested pUC19-U6-i2-crRNA backbone. The nucleotide sequence encoding DR was SEQ ID NO.59. The coding sequences of the crRNA spacer sequences for CCR5-3 and RNF2-7 are shown in SEQ ID NOs. 60 and 61, respectively.

### Cell culture, transfection, and fluorescence-activated cell sorting (FACS)

HEK293T cells were cultured in DMEM (Gibco) comprising 1% penicillin-streptomycin (Gibco) and 10% fetal calf serum (Gibco). Cells were seeded in 24-well cell culture dishes (Corning) for 16 hours until cell density reached 70%. By using Lipofectamine 3000 (Invitrogen), 600 ng of plasmid encoding Cas 12i2 protein and 300 ng of plasmid encoding crRNA were transfected into each 24-well cell culture dish. After approximately 68 hours of transfection, HEK293T cells to be subjected to fluorescence-activated cell sorting (FACS) were digested with trypsin-EDTA (0.05%) (Gibco). Cell sorting was performed using MoFlo XDP (Beckman Coulter) with GFP channel.

### Targeted deep sequencing analysis for genome modification

FACS-sorted GFP-positive HEK293T cells were lysed with buffer L and incubated at 55°C for 3 h and then at 95°C for 10 min. PCR amplification of dsDNA fragments comprising target sites in different genomic sites was performed using corresponding primers. For targeted deep sequencing, cell lysate was directly used as a template to directly amplify the target site through barcoded PCR. PCR products were purified and pooled into several libraries for high-throughput sequencing. The frequency (%) of indels was analyzed using CRISPResso2 software by calculating the ratio of reads comprising indels. Reads with less than 0.05% of complete reads are discarded.

Fig. 4 and Table 4 summarized the comparison of the gene editing efficiencies of the Cas12i2 mutant in this example with wild-type Cas12i2 at two genomic sites: CCRS-3 and RNF2-7 in 293T cells. We found that 7 mutants: G116R, E117R, T159R, S161R, E319R, E343R, and D958R can effectively improve gene editing efficiency (at least at one genomic site). Wherein, D958R demonstrated excellent gene editing efficiency at both genomic sites.

**Table 4 Summary of results (gene editing efficiency) of Example 4**

| Single amino acid substitution scheme and the combination substitution scheme | Gene editing efficiency (%) (indel rate at CCR5-3) | Gene editing efficiency (%) (indel rate at RNF2-7) |
|---|---|---|
| Reference enzyme (Cas12i2) | 10.44 | 11.70 |
| **G116R** | **19.14** | **23.95** |
| **E117R** | **19.79** | **25.01** |
| A156R | 6.15 | 3.64 |
| **T159R** | **22.59** | **21.89** |
| **S161R** | **21.81** | **23.64** |
| T301R | 0.75 | 0.18 |
| I305R | 0.84 | 0.45 |
| K306R | 9.35 | 4.30 |
| T308R | 11.23 | 6.48 |
| N312R | 12.93 | 11.03 |
| F313R | 19.45 | 12.58 |
| D427R | 14.11 | 16.28 |
| K433R | 12.72 | 11.85 |
| V438R | 10.81 | 7.03 |
| N441R | 12.75 | 11.96 |
| Q442R | 5.22 | 1.34 |
| M852R | 1.33 | 1.77 |
| L855R | 2.03 | 1.70 |
| N861R | 5.82 | 12.65 |
| Q865R | 9.23 | 6.63 |
| E160R | 13.15 | 17.70 |
| Q316R | 12.70 | 15.86 |
| **E319R** | **24.62** | - |
| **Q320R** | **18.85** | **20.11** |
| E247R | 8.56 | 11.63 |
| **E343R** | **18.20** | **24.24** |
| **E348R** | **14.83** | **20.37** |
| E349R | 7.71 | 11.39 |
| N679R | 8.98 | 12.43 |
| E683R | 16.67 | 18.17 |
| E691R | 10.93 | 14.98 |
| D782R | 9.67 | 21.61 |
| E783R | 9.78 | 13.25 |
| E797R | 12.27 | 20.91 |
| E800R | 0.71 | 1.53 |
| D853R | 0.50 | 0.00 |
| S957R | 13.41 | 17.61 |
| **D958R** | **29.00** | **36.82** |
| G293R | 14.32 | 19.40 |
| E294R | 13.07 | 16.98 |
| N297R | 9.02 | 10.90 |

### Example 5: Combine some of the Cas12i2 engineered amino acid mutations screened in Examples 1-4 to improve gene editing efficiency, and verify their gene editing efficiency.

### Plasmid construction

The combination of mutants was constructed by splitting the DNA of Cas12i2 protein into multiple segments and using PCR and Gibson clone. The position of the mutant was determined by analyzing the structural information of Cas 12i2 using protein structure visualization software commonly used (for example, PyMol, Chimera and other software can be used). For the structural information of Cas12i2, please refer to PDB: 6LTU, 6LTR, 6LU0, 6LTP). Cas 12i2 effector protein was expressed in human 293T cells through the pCAG-2A-eGFP vector. DNA encoding Cas12i2 protein was inserted between XmaI and NheI. A vector expressing the crRNA of Cas12i2 in 293T was constructed by ligating the annealed oligonucleotide comprising the target sequence into the BasI-digested pUC19-U6-i2-crRNA backbone. The nucleotide sequence encoding DR was SEQ ID NO.59.

### Cell culture, transfection, and fluorescence-activated cell sorting (FACS)

HEK293T cells were cultured in DMEM (Gibco) comprising 1% penicillin-streptomycin (Gibco) and 10% fetal calf serum (Gibco). Cells were seeded in 24-well cell culture dishes (Corning) for 16 hours until cell density reached 70%. By using Lipofectamine 3000 (Invitrogen), 600 ng of plasmid encoding Cas 12i2 protein and 300 ng of plasmid encoding crRNA were transfected into each 24-well cell culture dish. After approximately 68 hours of transfection, HEK293T cells to be subjected to fluorescence-activated cell sorting (FACS) were digested with trypsin-EDTA (0.05%) (Gibco). Cell sorting was performed using MoFlo XDP (Beckman Coulter) with GFP channel.

### Targeted deep sequencing analysis for genome modification

FACS-sorted GFP-positive HEK293T cells were lysed with buffer L and incubated at 55°C for 3 h and then at 95°C for 10 min. PCR amplification of dsDNA fragments comprising target sites in different genomic sites was performed using corresponding primers. For targeted deep sequencing, cell lysate was directly used as a template to directly amplify the target site through barcoded PCR. PCR products were purified and pooled into several libraries for high-throughput sequencing. The frequency (%) of indels was analyzed using CRISPResso2 software by calculating the ratio of reads comprising indels. Reads with less than 0.05% of complete reads are discarded.

We selected the amino acid mutations or amino acid mutation combinations screened in Examples 1-4: E176R+K238R+T447R+E563R, N164Y, E323R+D362R, I926R, E323R+D362R+I926R, E323R+D362R+I926G, E323R+D362R+I926G+L439(L+G), E323R+D362R+I926G+L439(L+GG) for further combinations. As shown in Fig. 5 and Table 5, by comparing the gene editing efficiency of these mutants with wild-type Cas12i2 in 293T cells at 5 genomic sites: CCR5-3, CCR5-5, CD34-8, CD34-9, RNF2-14, we found that after combination of different kinds of point mutations, we were able to obtain mutants with further improved efficiency. At the same time, we named the mutant (E176R+K238R+T447R+E563R+N164Y+E323R+D362R) with the best efficiency as CasXX. Here, the coding sequences of the crRNA spacer sequences used for CD34-8, CD34-9 and RNF2-14 are shown in SEQ ID NOs.: 63, 64 and 65 respectively.

**Table 5 Summary of the results (gene editing efficiency) of Example 5 (only the gene editing efficiency data reflected at RNF2-14 is taken as an example)**

| Type of mutation (the number is shown in the section "engineered Cas12i nuclease") | the combination substitution scheme | Amino acid sequence number SEQ ID NO. | Gene editing efficiency (%) (indel rate at RNF2-14) |
|---|---|---|---|
| / | Reference enzyme (Cas12i2) | 1 | 1.06 |
| (1) | **E176R+K238R+T447R+E563R** | | 33.73 |
| (2) | N164Y | | 10.08 |
| (3)/(4) | I926R | | 12.07 |
| (3)/(4) | E323R+D362R | | 14.48 |
| (1)+(2) | **E176R+K238R+T447R+E563R**+N164Y | 2 | 70.28 |
| (1)+(3)/(4) | **E176R+K238R+T447R+E563R+**I926R | 3 | 74.31 |
| (1)+(3)/(4) | **E176R+K238R+T447R+E563R**+E323R +D362R | 4 | 81.65 |
| (2)+(3)/(4) | N164Y+I926R | 5 | 46.89 |
| (2)+(3)/(4) | N164Y+E323R+D362R | 6 | 49.15 |
| (1)+(2)+(3)/(4) | **E176R+K238R+T447R+E563R**+N164Y +I926R | 7 | 86.96 |
| (1)+(2)+(3)/(4) | **E176R+K238R+T447R+E563R**+N164Y +E323R+D362R (named as **CasXX**) | 8 | **81.07** |
| (1)+(2)+(3)/(4) | **E176R+K238R+T447R+E563R**+N164Y +I926R+E323R+D362R | 9 | 76.54 |
| (1)+(2)+(3)/(4)+(6) | **E176R+K238R+T447R+E563R**+N164Y +E323R+D362R+I926G | 10 | 86.74 |
| (1)+(2)+(3)/(4)+(6) | **E176R+K238R+T447R+E563R**+N164Y +E3 23R+D3 62R+I926G+L43 9(L+GG) | 11 | 83.76 |
| (1)+(2)+(3)/(4)+(6) | **E176R+K238R+T447R+E563R**+N164Y +E323R+D362R+I926G+L439(L+G)* | 12 | 84.85 |

| | | | |
|---|---|---|---|
| *L439 (L+G) means that a glycine is inserted after amino acid No. 439; in the context of this specification and the accompany drawings, it is sometimes represented as 439G. | | | |

In addition, we constructed the following mutation combinations:
E176R+K238R+T447R+E563R+N164Y+D958R;
E176R+K238R+T447R+E563R+I926R+D958R;
E176R+K238R+T447R+E563R+E323R+D362R+D958R;
N164Y+I926R+D958R; N164Y+E323R+D362R+D958R;
E176R+K238R+T447R+E563R+N164Y+I926R+D958R;
E176R+K238R+T447R+E563R+N164Y+E323R+D362R+D958R;
E176R+K238R+T447R+E563R+N164Y+I926R+E323R+D362R+D958R;
E176R+K238R+T447R+E563R+N164Y+E323R+D362R+I926G+D958R;
E176R+K238R+T447R+E563R+N164Y+E323R+D362R+I926G+L439(L+GG)+D958R; and
E 176R+K238R+T447R+E563R+N 164Y+E323R+D362R+I926G+L439(L+G)+D958R.

Its gene editing efficiency can be detected through T7 endonuclease 1 (T7E1) assay and targeted deep sequencing.

### Example 6: Comparison of CasXX and conventional gene editing tools to verify its gene editing efficiency.

### Plasmid construction

To compare the gene editing activities of CasXX and other Cas, the coding sequences of AsCas 12a, BhCas12b v4, SpCas9, SaCas9, and SaCas9-KKH were codon optimized (human) and synthesized. Cas effector protein was expressed in human 293T cells through the pCAG-2A-eGFP vector. DNA encoding Cas protein was inserted between XmaI and NheI. A vector expressing the sgRNA or crRNA of AsCas12a, BhCas12b v4, SpCas9, SaCas9, SaCas9-KKH and Cas12i2 in 293T was constructed by ligating the annealed oligonucleotide comprising the target sequence into the BasI-digested pUC19-U6-i2-crRNA backbone. The nucleotide sequence encoding DR of CasXX was SEQ ID NO.59.

### Cell culture, transfection, and fluorescence-activated cell sorting (FACS)

HEK293T cells were cultured in DMEM (Gibco) comprising 1% penicillin-streptomycin (Gibco) and 10% fetal calf serum (Gibco). Cells were seeded in 24-well cell culture dishes (Corning) for 16 hours until cell density reached 70%. By using Lipofectamine 3000 (Invitrogen), 600 ng of plasmid encoding Cas 12i2 protein and 300 ng of plasmid encoding crRNA were transfected into each 24-well cell culture dish. After approximately 68 hours of transfection, HEK293T cells to be subjected to fluorescence-activated cell sorting (FACS) were digested with trypsin-EDTA (0.05%) (Gibco). Cell sorting was performed using MoFlo XDP (Beckman Coulter) with GFP channel.

### Targeted deep sequencing analysis for genome modification

FACS-sorted GFP-positive HEK293T cells were lysed with buffer L and incubated at 55°C for 3 h and then at 95°C for 10 min. PCR amplification of dsDNA fragments comprising target sites in different genomic sites was performed using corresponding primers. For targeted deep sequencing, cell lysate was directly used as a template to directly amplify the target site through barcoded PCR. PCR products were purified and pooled into several libraries for high-throughput sequencing. The frequency (%) of indels was analyzed using CRISPResso2 software by calculating the ratio of reads comprising indels. Reads with less than 0.05% of complete reads are discarded.

We first tested the gene editing efficiency of CasXX at 62 human genomic sites comprising different the PAM sequences. The designed spacer sequence was 20 nucleotides. As shown in Fig. 6a, CasXX demonstrated extremely powerful gene editing capabilities, with an average gene editing efficiency exceeding 60%, and gene editing efficiencies exceeding 50% at almost all sites tested. Moreover, it showed high gene editing efficiency for any NTTN PAM (N=A, T, G, C).

To further demonstrate the gene editing capabilities of engineered CasXX, we compared CasXX with AsCas12a at the TTTN PAM site and with BhCas12b v4 at the TTN PAM site. As shown in Fig. 6b, CasXX exhibited higher average gene editing efficiency at target sites with both PAMs.

We also compared CasXX with SpCas9, SaCas9, and SaCas9-KKH at the same site. As shown in Fig. 6c, CasXX demonstrated higher average gene editing efficiency at all test sites.

To test the gene editing activity of CasXX in vivo, the pCAG-2A-eGFP vector encoding CasXX and the pUC19-U6-i2-crRNA vector encoding crRNA were transfected into the mouse Hepa1-6 liver cancer cell line using lipofectamine transfection. Wherein, corresponding crRNAs were designed for 65 endogenous gene sites. The designed spacer sequence is 20 nucleotides. The frequency of indels is obtained by PCR amplification and sequencing, which is similar to the exogenous gene editing analysis method.

As shown in Fig. 6d, CasXX demonstrated powerful gene editing capabilities on 65 endogenous gene sites of the mouse Hepa1-6 cell line, with an average gene editing efficiency exceeding 60%.

### Example 7: Gene editing at genomic sites comprising different PAMs using CasXX.

### Plasmid construction

The DNA sequence encoding CasXX was loaded into the pCAG-2A-EGFP plasmid to construct a plasmid expressing CasXX. A vector expressing crRNA in HEK293T was constructed by ligating annealed oligonucleotides comprising the target sequence into the BasI-digested pUC19-U6-crRNA backbone. Wherein, 64 different crRNAs were designed to target 64 human endogenous sites. The 5' end of the endogenous target nucleic acid has different PAMs 5'-NNNN-3' (N=A, T, G or C) as shown in Fig. 7 to detect the recognition ability of CasXX to different PAMs. Wherein, the PAM sequences contained in these 64 sites cover all NNNN combinations: NTTN, NTAN, NTCN, NTGN, NATN, NAAN, NACN, NAGN, NCTN, NCAN, NCCN, NCGN, NGTN, NGAN, NGCN, NGGN. The nucleotide sequence encoding DR is SEQ ID NO.59. The designed spacer sequence has 20 nucleotides.

### Cell culture, transfection, and fluorescence-activated cell sorting (FACS)

HEK293T cells were cultured in DMEM (Gibco) comprising 1% penicillin-streptomycin (Gibco) and 10% fetal calf serum (Gibco). Cells were seeded in 24-well cell culture dishes (Corning) for 16 hours until cell density reached 70%. By using Lipofectamine 3000 (Invitrogen), 600 ng of plasmid encoding Cas protein and 300 ng of plasmid encoding crRNA were transfected into each 24-well cell culture dish. After approximately 72 hours of transfection, the cells were digested with trypsin-EDTA (0.05%) (Gibco), and subjected to fluorescence-activated cell sorting (FACS).

### Targeted deep sequencing analysis for genome modification

FACS-sorted GFP-positive HEK293T cells were lysed with buffer L and incubated at 55°C for 3 h and then at 95°C for 10 min. PCR amplification of dsDNA fragments comprising target sites in different genomic sites was performed using corresponding primers. For targeted deep sequencing, cell lysate was directly used as a template to directly amplify the target site through barcoded PCR. PCR products were purified and pooled into several libraries for high-throughput sequencing. The frequency (%) of indels was analyzed using CRISPResso2 software by calculating the ratio of reads comprising indels. Reads with less than 0.05% of complete reads are discarded.

As shown in Fig. 7, at the target sites with PAM such as NTTN, NTAN, NTCN, NTGN, NATN, NAAN, NCTN, NCAN, and NGTN at the 5' end, CasXX has shown high gene editing efficiency. The average gene editing efficiencies at these sites exceeds 40%.

### Example 8: In vitro cleavage of double-stranded DNA comprising different PAMs using CasXX

### Construct the expression plasmids for the mutants

We loaded the DNA sequences encoding wild-type Cas12i2 (SEQ ID NO.1) and CasXX (SEQ ID NO.8) into the BPK2014 plasmid (with chloramphenicol resistance) to construct plasmids with prokaryotic expression of Cas12i2 and CasXX proteins.

### Protein purification

The BPK2014 prokaryotic expression plasmid was transformed into E. coli strain BL21 (λDE3) (TransGen Biotech), and the transformed bacterial liquid was spread on solid LB comprising chloramphenicol. Three clones were picked into 5 ml liquid LB and cultured overnight. Then the bacterial liquid was transferred to 3L liquid LB and continued culturing until OD600 reached 0.6-0.8, then induced with IPTG (0.5mM) at 16°C for 20 hours. Cas12i-expressing bacteria were harvested by ultracentrifugation, resuspended in lysis buffer (50mM Tris-HCl, pH7.5, 300mM NaCl) and disrupted by sonication. After centrifugation, the Cas12i protein in the supernatant was first purified using a Ni column. Briefly, after incubation with the supernatant, the Ni column was washed sequentially with lysis buffer supplemented with 0, 20, and 50 mM imidazole. Then, The Cas 12i protein was eluted with lysis buffer supplemented with 500mM imidazole. The collected samples were then loaded into an ion exchange column (CM Sepharose Fast Flow, GE). Wild-type Cas 12i2 and CasXX proteins were eluted with storage buffer (20mM Tris-HCl, 300mM NaCl, 1mM TCEP, 10% glycerol, pH 7.5). Proteins were sterilized with filter and stored at -80°C.

### In vitro transcription of crRNA

The nucleotide sequence encoding DR was SEQ ID NO.59. Oligonucleotides comprising the T7 promoter sequence (named as T7-F) and oligonucleotides comprising crRNA and T7 promoter complementary sequences (named as T7-12i-crRNA-R) were synthesized and annealed in 1xNEBufferTM2 (NEB). The sequences of these oligonucleotides were listed in Table 6. The annealed product was used as a template to produce crRNA using HiScribeTM T7 Quick High Yield RNA Synthesis Kit (NEB). Transcribed crRNA was purified using Monarch^{®} RNA Cleanup Kit (NEB).

**Table 6 crRNA primer sequences**

| | | |
|---|---|---|
| SEQ ID NO.41 | T7-F | TAATACGACTCACTATAGG |
| SEQ ID NO.42 | T7-12i-crRNA-R | AAAAATTTGTTTGGATATGTTGAACCGTCAATGA AAGACGGATTTCTCCCTATAGTGAGTCGTATTA |

### In vitro enzymatic cleavage of Cas12i protein

To prepare linear dsDNA substrates for in vitro cleavage, targets comprising the same protospacer and different PAMs were first cloned into pUC19 (with penicillin resistance) treated with EcoR1 and HindIII. Target sequences with the PAM at 5' are listed in Table 7. The target-carrying pUC19 plasmid was then linearized by SacI and purified using DNA Clean & Concentrator (Zymo Research). For in vitro cleavage experiments, 400 nM Cas12i protein was first incubated with 2 µM crRNA for 15 min at 37°C. Next, in a 10 µl reaction system comprising 1x NEBufferTM3.1 (NEB), Cas12i-crRNA RNP was reacted with 150 ng of linearized target DNA at 37°C for 40 minutes. Then, the reaction was stopped with 50mM EDTA, and the RNA was digested with RNase cocktail (Invitrogen) at 37°C for 15 minutes. Finally, the samples were treated with protease (NEB) at 37°C for 15 minutes. Reaction products were separated by electrophoresis on a 1.2% agarose gel.

As shown in Fig. 8, the wild-type Cas12i2 protein only had partial cleavage efficiency for double-stranded DNA comprising 5'-NTTN-3' PAM, but had almost no cleavage activity for the remaining PAMs. However, the CasXX protein exhibited high cleavage efficiency for double-stranded DNA comprising NTTN, NTAN, NTCN, NTGN, NATN, NAAN, NACN, NCTN, NCAN, NGTN, and NGAN PAM. CasXX can almost completely cleave double-stranded DNA comprising NTTN, NTAN, NTCN, NATN, NAAN, NACN, NCTN, NCAN, and NGTN PAM. Therefore, the engineered Cas12i nuclease provided by the present invention can be used for a wider range of gene editing or treatment.

**Table 7: 5' PAM-target sequence**

| | | |
|---|---|---|
| SEQ ID NO.43 | NTTN-target | **CTTC**TTCAACATATCCAAACAAAT |
| SEQ ID NO.44 | NTAN-target | **CTAC**TTCAACATATCCAAACAAAT |
| SEQ ID NO.45 | NTCN-target | **CTCC**TTCAACATATCCAAACAAAT |
| SEQ ID NO.46 | NTGN-target | **CTCC**TTCAACATATCCAAACAAAT |
| SEQ ID NO.47 | NATN-target | **CATC**TTCAACATATCCAAACAAAT |
| SEQ ID NO.48 | NAAN-target | **CAAC**TTCAACATATCCAAACAAAT |
| SEQ ID NO.49 | NACN-target | **CACC**TTCAACATATCCAAACAAAT |
| SEQ ID NO.50 | NAGN-target | **CACC**TTCAACATATCCAAACAAAT |
| SEQ ID NO.51 | NCTN-target | **CCTC**TTCAACATATCCAAACAAAT |
| SEQ ID NO.52 | NCAN-target | **CCAC**TTCAACATATCCAAACAAAT |
| SEQ ID NO.53 | NCCN-target | **CCCC**TTCAACATATCCAAACAAAT |
| SEQ ID NO.54 | NCGN-target | **CCGC**TTCAACATATCCAAACAAAT |
| SEQ ID NO.55 | NGTN-target | **CGTC**TTCAACATATCCAAACAAAT |
| SEQ ID NO.56 | NGAN-target | **CGAC**TTCAACATATCCAAACAAAT |
| SEQ ID NO.57 | NGCN-target | **CGCC**TTCAACATATCCAAACAAAT |
| SEQ ID NO.58 | NGGN-target | **CGGC**TTCAACATATCCAAACAAAT |

### Example 9: Using GUIDE-Seq to detect off-target effects of CasXX

### Plasmid construction

We loaded the DNA sequence encoding CasXX into the pCAG-2A-EGFP plasmid to construct a plasmid expressing CasXX. A vector expressing Cas protein crRNA in 293T (human kidney epithelial cell line) was constructed by ligating annealed oligonucleotides comprising the target sequence into the BasI-digested pUC19-U6-crRNA backbone. Wherein, the crRNA comprised a spacer sequence (spacer; SEQ ID NO.77) that can target the endogenous site of EMX1-7. The nucleotide sequence encoding DR is SEQ ID NO.59.

### Cell culture, transfection, and fluorescence-activated cell sorting (FACS)

HEK293T cells were cultured in DMEM (Gibco) comprising 1% penicillin-streptomycin (Gibco) and 10% fetal calf serum (Gibco). Cells were seeded in 24-cell culture dishes (Corning) for 16 hours until cell density reached 70%. By using Lipofectamine 3000 (Invitrogen), 600 ng of plasmid encoding Cas protein, 300 ng of plasmid encoding crRNA and 10 pmol of annealed double-stranded DNA tag (see Table 8 for the sequence) were transfected into cells cultured in a 24-well cell culture dish. After 72 hours of transfection, the cells were digested with trypsin-EDTA (0.05%) (Gibco), and then subjected to GFP fluorescence-activated cell sorting (FACS). The cells that successfully expressed Cas enzyme were sorted.

**Table 8 Primer sequences of double-stranded DNA tags (P represents phosphate modification, * represents phosphorothioate diester bond modification)**

| | | |
|---|---|---|
| SEQ ID NO.25 | GUIDE-F1 | 5'-P-G*T*TTAATTGAGTTGTCATATGTTAATAACGGT*A*T-3' |
| SEQ ID NO.26 | GUIDE-R1 | 5'-P-A*T*ACCGTTATTAACATATGACAACTCAATTAA*A*C-3' |

### Detecting the off target effect of CasXX on a genome-wide scale

Genomes for FACS-sorted GFP-positive 293T cells were extracted using the E.Z.N.A.^{®} MicroElute Genomic DNA Kit (Omega). The purified genome was quantified using Qubit. Use the Covaris S220 instrument and follow the instrument's recommended procedures to fragment the genome to about 500bp. Then use the VAHTS Universal Pro DNA Library Prep Kit for Illumina (Vazyme) to construct the DNA library. For the library construction process, please refer to the reference literature (Tsai, SQ et al. "GUIDE-seq enables genome-wide profiling of off-target cleavage by CRISPR-Cas nucleases," Nat Biotechnol. 2015;33(2):187-197; the contents of which are incorporated herein by reference in their entirety). The library construction products were subjected to high-throughput sequencing. Sequencing results were analyzed to look for potential off-target sites. We conducted a systematic analysis of the off-target effects of CasXX at the EMX1-7 site. The specific analysis results were shown in Fig. 10. The first row of sequences was the reference target sequence, and the following sequences represented the target sequence and off-target sequence respectively, as well as the number of reads enriched by double-stranded DNA tags in the target sequence and off-target sequence.

It can be seen from the relatively high number of off-targets that the editing specificity of CasXX enzyme in mammalian cells was not ideal and needed further optimization.

### Example 10: Introduce new amino acid mutations based on the CasXX sequence (SEQ ID NO.8), and test the characteristics of improved specificity of the mutant (two target positions were selected for mutant screening experiments, namely EMX1-7 site and RNF2-1 site)

### Preparation for screening experiments: construct the expression plasmids for the mutants

On the basis of CasXX (comprising the N164Y+E176R+K238R+E323R+D362R+T447R+E563R mutation based on SEQ ID NO. 1), we planned to further introduce new amino acid point mutations to improve the specificity of CasXX gene editing and reduce off-target effect. Based on the sequence of CasXX (SEQ ID NO.8), we used primers with mutated bases to perform PCR on the DNA sequence of CasXX, and used the NEBuilder^{®} HiFi DNA Assembly Master Mix (NEB) kit to load the purified PCR product into pCAG -2A-EGFP plasmid, thereby constructing a CasXX plasmid expressing the relevant point mutations. We obtained a total of 26 mutants based on CasXX sequences and comprising single amino acid mutations, named CasXX-HF-1 to HF-26. The specific mutation methods were shown in Table 9. Wherein, CasXX-HF-26 restored the N164Y point mutation in CasXX to the original amino acid N of wild-type Cas12i2, namely Y164N (that is, the N164Y mutation was deleted).

**Table 9: CasXX-HF mutants**

| Perform point mutation on the basis of reference enzyme CasXX (SEQ ID NO.8): | | |
|---|---|---|
| CasXX-HF Mutant number | Mutation scheme | SEQ ID NO. |
| HF-1 | S565A | |
| HF-2 | N297A | |
| HF-3 | Q865A | |
| HF-4 | T308A | |
| HF-5 | R309A | |
| HF-6 | N312A | |
| HF-7 | N441A | |
| HF-8 | R857A | 14 |
| HF-9 | N861A | 15 |
| HF-10 | Y119F | |
| HF-11 | K433A | |
| HF-12 | K807A | 16 |
| HF-13 | N841A | |
| HF-14 | N848A | 17 |
| HF-15 | K845A | |
| HF-16 | D782A | |
| HF-17 | R715A | 18 |
| HF-18 | R719A | 19 |
| HF-19 | S766A | |
| HF-20 | K394A | 20 |
| HF-21 | H357A | 21 |
| HF-22 | K844A | 22 |
| HF-23 | E395A | |
| HF-24 | S346A | |
| HF-25 | R402A | |
| HF-26 | Y164N | |

### Construction of expression plasmid for crRNA targeting EMX1-7 site

A vector expressing Cas protein crRNA in 293T was constructed by ligating the annealed oligonucleotide comprising the target sequence into the pUC19-U6-crRNA backbone digested with BasI (NEB) using T4ligase (NEB). The crRNA was designed to target the EMX1-7 locus. See Table 10 for specific sequences. The nucleotide sequence encoding DR is SEQ ID NO.59.

**Table 10: DNA sequences encoding spacers targeting EMX1-7**

| Sequence number | Name of crRNA target site | Encoding sequence of spacer |
|---|---|---|
| SEQ ID NO.27 | EMX1-7 | TGGTTGCCCACCCTAGTCAT |

### Cell culture, transfection, and fluorescence-activated cell sorting (FACS)

HEK293T cells were cultured in DMEM (Gibco) comprising 1% penicillin-streptomycin (Gibco) and 10% fetal calf serum (Gibco). Cells were seeded in 24-cell culture dishes (Corning) for 16 hours until cell density reached 70%. By using Lipofectamine 3000 (Invitrogen), 600 ng of plasmid encoding Cas protein, 300 ng of plasmid encoding crRNA were transfected into cells cultured in a 24-well cell culture dish. After 72 hours of transfection, the cells were digested with trypsin-EDTA (0.05%) (Gibco), and then subjected to GFP fluorescence-activated cell sorting (FACS).

### Use T7E1 restriction method to detect the editing efficiency of Cas protein at EMX1-7 target sites and off-target sites

FACS-sorted GFP-positive HEK293T cells were lysed with 40 µL of buffer L (bimake), incubated at 55°C for 3 hours, and then at 95°C for 10 minutes. PCR amplification was performed on dsDNA fragments containing target or off target sites in different genomic sites using primer pairs targeting EMX1-7 target site, EMX1-7 off-target site 1, EMX1-7 off-target site 2, and EMX1-7 off-target site 3 (as shown in Fig. 10). The sequences of the target or off target sites were shown in Table 11. Afterwards, using 10 µL of PCR product, a re-annealing procedure was performed to form heteroduplex dsDNA. The mixture was then treated with 1/10 volume of NEBuffer^{™} 2.1 and 0.2 µL of T7 endonuclease I (NEB) for 50 min at 37°C. Digestion products were analyzed by -2.5% agarose gel electrophoresis. The indel rate (Indel, %) was calculated based on the gray value of the band. The indel rate of each Cas12i mutant at the target site or off-target site was shown in Table 12, corresponding to Fig. 11. Experimental results showed that R857A, N861A, K807A, N848A, R715A, R719A, K394A, H357A, and K844A, these single point mutants based on the CasXX sequence (SEQ ID NO: 8) (corresponding to the engineered Cas12i nuclease of SEQ ID NO: 14-22, as shown in Table 9) can effectively reduce the indel rate at the off target sites EMX1-7-OT-1, EMX1-7-OT-2, and EMX1-7-OT-3, with higher specificity. In the experimental results, we defined the reduction in the indel rate at the off-target site and the basically unchanged (or increased) editing efficiency at the target site as an improvement in editing specificity.

**Table 11 Sequences of EXM1-7 target site and off-target site**

| SEQ ID NO. | name | abbreviation | sequence |
|---|---|---|---|
| 28 | target site | EMX1-7 | TGGTTGCCCACCCTAGTCAT (same as SEQ ID NO.27) |
| 29 | off-target site 1 | EMX1-7-OT-1 | TGTTTGCCCACCCTAGTCCT |
| 30 | off-target site 2 | EMX1-7-OT-2 | TGATTGCCCACCCTACTCCT |
| 31 | off-target site 3 | EMX1-7-OT-3 | TGGTCACCCACCCTAGTCTG |

**Table 12 Comparison of editing efficiency of CasXX-HF1 with CasXX-HF-26 in EMX1-7 target sequence and off-target sequence**

| CasXX-HF Mutant number | Mutation scheme | The indel rate at EMX1-7 | The indel rate at EMX1-7-OT-1 | The indel rate at EMX1-7-OT-2 | The indel rate at EMX1-7-OT-3 |
|---|---|---|---|---|---|
| HF-1 | S565A | 35.89 | 18.06 | 13.24 | 13.55 |
| HF-2 | N297A | 39.41 | 19.88 | 16.24 | 11.69 |
| HF-3 | Q865A | 42.77 | 23.78 | 10.50 | 12.67 |
| HF-4 | T308A | 40.61 | 17.94 | 0.00 | 9.27 |
| HF-5 | R309A | 43.83 | 27.64 | 18.95 | 14.92 |
| HF-6 | N312A | 42.11 | 9.50 | 18.94 | 0.00 |
| HF-7 | N441A | 36.82 | 14.41 | 10.99 | 9.03 |
| **HF-8** | **R857A** | **30.08** | **1.11** | **0.00** | **0.00** |
| **HF-9** | **N861A** | **34.97** | **10.35** | **0.00** | **1.52** |
| HF-10 | Y119F | 38.00 | 22.47 | 13.99 | 14.17 |
| HF-11 | K433A | 41.34 | 19.06 | 0.00 | 10.86 |
| HF-12 | K807A | 5.20 | 18.89 | 0.00 | 1.68 |
| HF-13 | N841A | 39.46 | 18.37 | 13.08 | 12.86 |
| **HF-14** | **N848A** | **30.98** | **0.00** | **0.00** | **0.00** |
| HF-15 | K845A | 39.17 | 19.51 | 0.00 | 13.42 |
| HF-16 | D782A | 37.33 | 11.45 | 0.00 | 5.19 |
| **HF-17** | **R715A** | **35.07** | **5.21** | **0.00** | **1.45** |
| **HF-18** | **R719A** | **41.08** | **18.94** | **0.00** | **8.16** |
| HF-19 | S766A | 38.50 | 22.90 | 0.00 | 8.32 |
| **HF-20** | **K394A** | **37.30** | **0.00** | **0.00** | **0.00** |
| **HF-21** | **H357A** | **39.24** | **7.70** | **0.00** | **2.39** |
| **HF-22** | **K844A** | **39.22** | **3.89** | **0.00** | **0.00** |
| HF-23 | E395A | 35.70 | 19.60 | 0.00 | 8.15 |
| HF-24 | S346A | 37.03 | 18.33 | 13.00 | 9.37 |
| HF-25 | R402A | 34.83 | 15.83 | 0.00 | 7.72 |
| HF-26 | Y164N | 39.14 | 12.56 | 4.81 | 11.95 |
| **CasXX** | **reference** | **42.05** | **16.41** | **11.66** | **9.76** |

### Construction of expression plasmid for crRNA targeting RNF2-1 site

A vector expressing Cas protein crRNA in HEK293T was constructed by ligating the annealed oligonucleotide comprising the target sequence into the pUC19-U6-crRNA backbone digested with BasI (NEB) using T4 ligase (NEB). The final crRNAs all targeted the same RNF2-1 site, but the spacer sequences were different. The specific sequences encoding these spacers were shown in Table 13. Wherein, RNF2-1-FM represented that the spacer sequence in crRNA completely matches the RNF2-1 site. RNF2-1-Mis-1/2 represented that the spacer sequence in crRNA did not match the RNF2-1 site at the 1st and 2nd base positions, but matched at the remaining positions. RNF2-1-Mis-5/6 represented that the spacer sequence in crRNA did not match the RNF2-1 site at the 5th and 6th base positions, but matched at the remaining positions. RNF2-1-Mis-17/1 represented that the spacer sequence in crRNA did not match the RNF2-1 site at the 17th and 18th base positions, but matched at the remaining positions. RNF2-1-Mis-19/20 represented that the spacer sequence in crRNA did not match the RNF2-1 site at the 19th and 20th base positions, but matched at the remaining positions. Here, the purpose of setting base mismatch was to simulate off-target effects. The nucleotide sequence encoding DR was SEQ ID NO.59.

**Table 13 DNA coding sequences of spacer sequences targeting RNF2-1**

| SEQ ID NO. | crRNA name | spacer encoding sequence |
|---|---|---|
| 32 | crRNA-RNF2-1-FM | TACAGGAGGCAATAACAGAT |
| 33 | crRNA-RNF2-1 -Mis-1/2 | **GC**CAGGAGGCAATAACAGAT |
| 34 | crRNA-RNF2-1-Mis-5/6 | TACA**TT**AGGCAATAACAGAT |
| 35 | crRNA-RNF2-1-Mis-17/18 | TACAGGAGGCAATAAC**CT**AT |
| 36 | crRNA-RNF2-1-Mis-19/20 | TACAGGAGGCAATAACAG**CG** |

### Cell culture, transfection, and fluorescence-activated cell sorting (FACS)

HEK293T cells were cultured in DMEM (Gibco) comprising 1% penicillin-streptomycin (Gibco) and 10% fetal calf serum (Gibco). Cells were seeded in 24-cell culture dishes (Corning) for 16 hours until cell density reached 70%. By using Lipofectamine 3000 (Invitrogen), 600 ng of plasmid encoding Cas protein, 300 ng of plasmid encoding crRNA were transfected into cells cultured in a 24-well cell culture dish. After 72 hours of transfection, the cells were digested with trypsin-EDTA (0.05%) (Gibco), and then subjected to GFP fluorescence-activated cell sorting (FACS). The cells that successfully expressed Cas enzyme were sorted.

### Using T7E1 enzyme digestion method to detect the editing efficiency of Cas12i mutants at RNF2-1 and off-target sites

FACS-sorted GFP-positive HEK293T cells were lysed with 40 µL of buffer L (bimake), incubated at 55°C for 3 hours, and then at 95°C for 10 minutes. PCR amplification was performed on dsDNA fragments of RNF2-1 site using RNF2-1 primer pairs. Afterwards, using 10 µL of PCR product, a re-annealing procedure was performed to form heteroduplex dsDNA. The mixture was then treated with 1/10 volume of NEBuffer^{™} 2.1 and 0.2 µL of T7 endonuclease I (NEB) for 50 min at 37°C. Digestion products were analyzed by -2.5% agarose gel electrophoresis. The indel rate (Indel, %) was calculated based on the gray value of the band. The indel rate of at the target site for each Cas12i mutant using different crRNAs was shown in Table 14, corresponding to Fig. 12. Experimental results showed that R857A, N861A, K807A, N848A, R715A, R719A, K394A, H357A, and K844A, these single point mutants based on the CasXX sequence (corresponding to the engineered Cas 12i nuclease of SEQ ID NO: 14-22, as shown in Table 9) can effectively reduce the indel rate even when using simulated off target crRNA-RNF2-1-Mis-1/2, crRNA-RNF2-1-Mis-5/6, crRNA-RNF2-1-Mis- 17/18 and crRNA-RNF2-1-Mis-19/20. In the experimental results, we defined the reduction in the indel rate at the off-target site and the basically unchanged (or increased) editing efficiency at the target site as an improvement in editing specificity.

**Table 14: Editing efficiency of different CasXX-HF mutants at the RNF2-1 position using crRNA targeting the target sequence and simulating the off-target spacer sequence**

| CasXX-HF mutant number | Mutation scheme | On-target spacer (%) | Off-target spacer-1 (%) | Off-target spacer-2 (%) | Off-target spacer-3 (%) | Off-target spacer-4 (%) |
|---|---|---|---|---|---|---|
| HF-1 | S565A | 33.68 | 26.30 | 18.91 | 39.35 | 42.04 |
| HF-2 | N297A | 41.56 | 29.55 | 21.42 | 42.23 | 43.42 |
| HF-3 | Q865A | 43.64 | 28.30 | 21.79 | 43.02 | 43.62 |
| HF-4 | T308A | 29.13 | 0.00 | 0.00 | 35.76 | 39.49 |
| HF-5 | R309A | 32.22 | 30.67 | 0.00 | 26.92 | 38.92 |
| HF-6 | N312A | 32.69 | 0.00 | 0.00 | 42.18 | 43.69 |
| HF-7 | N441A | 38.05 | 12.86 | 10.40 | 17.67 | 31.53 |
| **HF-8** | **R857A** | **39.15** | **3.18** | **0.00** | **0.00** | **3.89** |
| **HF-9** | **N861A** | **44.45** | **11.79** | **3.42** | **3.41** | **41.61** |
| HF-10 | Y119F | 35.25 | 11.46 | 9.31 | 15.33 | 32.11 |
| HF-11 | K433A | 31.50 | 9.80 | 0.00 | 16.26 | 33.46 |
| **HF-12** | **K807A** | **30.46** | **12.48** | **0.00** | **0.00** | **33.29** |
| HF-13 | N841A | 37.30 | 17.92 | 13.45 | 23.58 | 43.25 |
| **HF-14** | **N848A** | **23.74** | **0.00** | **0.00** | **0.00** | **32.51** |
| HF-15 | K845A | 38.94 | 12.87 | 5.20 | 4.17 | 40.51 |
| HF-16 | D782A | 41.08 | 12.28 | 12.86 | 4.56 | 29.88 |
| **HF-17** | **R715A** | **39.99** | **5.00** | **0.00** | **0.00** | **23.18** |
| **HF-18** | **R719A** | **39.31** | **5.07** | **0.00** | **0.00** | **2.81** |
| HF-19 | S766A | 39.49 | 18.53 | 9.09 | 7.07 | 38.14 |
| **HF-20** | **K394A** | **35.86** | **0.00** | **0.00** | **0.00** | **3.20** |
| **HF-21** | **H357A** | **35.84** | **5.96** | **0.00** | **0.00** | **19.12** |
| **HF-22** | **K844A** | **37.23** | **2.25** | **0.00** | **0.00** | **15.07** |
| HF-23 | E395A | 37.68 | 12.80 | 5.46 | 0.00 | 33.70 |
| HF-24 | S346A | 34.71 | 15.28 | 8.65 | 8.03 | 40.87 |
| HF-25 | R402A | 37.54 | 12.97 | 3.39 | 0.00 | 32.88 |
| HF-26 | Y164N | 32.33 | 10.00 | 9.50 | 11.73 | 36.56 |
| **CasXX** | 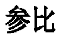 | **39.67** | **17.02** | **10.42** | **12.10** | **37.56** |

### Example 11: Mutants introducing new amino acid mutations based on the CasXX sequence

For mutants introducing new amino acid mutations based on the CasXX sequence, those mutants with improved specificity were screened in a fluorescent reporter system. The difference between this Example and Example 10 was that the analysis method that reflects the editing efficiency was changed from agarose gel electrophoresis to fluorescence.

### Construction of expression plasmid for crRNA targeting mCherrygene

A vector expressing Cas protein crRNA in HEK293T was constructed by ligating the annealed oligonucleotide comprising the target sequence into the pUC19-U6-crRNA backbone digested with BasI (NEB) using T4 ligase (NEB). The final crRNAs all targeted the same mCherry site, but the spacer sequences were different. The specific sequence of encoding spacer was shown in Table 15. mCherry-FM represented that the spacer sequence in crRNA completely matched the mCherry site. crRNA-mCherry-Mis-1/2 represented that the spacer sequence in crRNA did not match the crRNA-mCherry site at the 1st and 2nd base positions, but matched at the remaining positions. crRNA-mCherry-Mis-5/6 represented that the spacer sequence in crRNA did not match the crRNA-mCherry site at the 5th and 6th base positions, but matched at the remaining positions. crRNA-mCherry-Mis-19/20 represented that the spacer sequence in crRNA did not match the crRNA-mCherry site at the 19th and 20th base positions, but matched at the remaining positions. The nucleotide sequence encoding DR is SEQ ID NO.59.

**Table 15: Spacer encoding sequences targeting mCherry and simulating off target**

| SEQ ID NO. | Name of crRNA target site | Spacer encoding sequence |
|---|---|---|
| 37 | crRNA-mCherry-FM | ACCTTGTAGATGAACTCGCC |
| 38 | crRNA-mCherry-Mis-1/2 | CACTTGTAGATGAACTCGCC |
| 39 | crRNA-mCherry-Mi s-5/6 | ACCT**GT**TAGATGAACTCGCC |
| 40 | crRNA-mCherry-Mis-19/20 | ACCTTGTAGATGAACTCG**AA** |

### Cell culture and transfection

HEK293T cells were cultured in DMEM (Gibco) comprising 1% penicillin-streptomycin (Gibco) and 10% fetal calf serum (Gibco). Cells were seeded in 24-well cell culture dishes (Corning) for 16 hours until cell density reached 70%. By using Lipofectamine 3000 (Invitrogen), 600 ng of plasmid encoding Cas protein, 300 ng of plasmid encoding crRNA, and 100 ng of plasmid encoding mCherry were transfected into cells cultured in 24-well cell culture dishes.

### Using flow cytometry to detect the editing efficiency of Cas protein at the mCherry site

72h after transfection, the cells were digested with trypsin-EDTA (0.05%) (Gibco), and then analyzed by flow cytometry to calculate the ratio of GFP and mCherry fluorescence. The lower the ratio of mCherry, the higher the efficiency of Cas protein in editing the mCherry gene. The process diagram and mCherry editing efficiency formula were shown in Fig. 13.

Experimental results showed that for R857A, R719A, K394A, and K844A (corresponding to SEQ ID NO.14, 19, 20, and 22 respectively), these single point mutants based on CasXX sequences, when using simulated off-target crRNA-mCherry-Mis-1/2, crRNA-mCherry-Mis-5/6 and crRNA- mCherry-Mis-19/20, the efficiency of editing the mCherry gene was significantly reduced, as shown in Fig. 14 (the higher the mCherry% at the off-target site, the lower the off-target efficiency). Here, we defined the reduction in the indel rate at the off-target site and the basically unchanged (or increased) editing efficiency at the target site as an improvement in editing specificity.

### Example 12: Introducing amino acid combination mutations based on the CasXX sequence, and screening mutants with improved specificity at endogenous gene (combination) sites

### Preparation for screening experiments: construct the expression plasmids for the mutants

Based on Examples 10 and 11, we selected point mutations R857A, R719A, K394A, and K844A for further combinatorial exploration. Based on the sequence of CasXX, we used primers with mutated bases to perform PCR on the DNA sequence of CasXX. We used the NEBuilder^{®} HiFi DNAAssembly Master Mix (NEB) kit to load the purified PCR product into the pCAG-2A-EGFP plasmid, thereby constructing a CasXX plasmid with combinatorial mutations. We obtained a total of 11 mutant names based on the CasXX sequence (SEQ ID NO.8).The specific mutation schemes comprising combinatorial mutations were shown in Table 16.

**Table 16: CasXX-HF mutants**

| Perform point mutation on the basis of reference enzyme CasXX (SEQ ID NO.8) : | | |
|---|---|---|
| CasXX-HF mutant number | Further Mutation scheme | SEQ ID NO. |
| HF-8 | R857A | 14 |
| HF-18 | R719A | 19 |
| HF-20 | K394A | 20 |
| HF-22 | K844A | 22 |
| HF-18+20 | R719A/K394A | |
| HF-20+22 | K394A/K844A | |
| HF-8+20 | R857A/K394A | |
| HF-18+22 | R719A/K844A | 23 |
| HF-8+18 | R857A/R719A | |
| HF-8+22 | R857A/K844A | 24 |
| HF-18+20+22 | R719A/K394A/K844A | |
| HF-8+18+20 | R857A/R719A/K394A | |
| HF-8+20+22 | R857A/K394A/K844A | |
| HF-8+18+22 | R857A/R719A/K844A | |
| HF-8+18+20+22 | R857A/R719A/K394A/K844A | |

### Construction of expression plasmids of crRNA targeting EMX1-7 and RNF2-1 positions

A vector expressing Cas protein crRNA in 293T was constructed by ligating the annealed oligonucleotide comprising the target sequence into the pUC19-U6-crRNA backbone digested with BasI (NEB) using T4 ligase (NEB). crRNA was designed as described in Example 10, targeting EMX1-7 or RNF2-1, or simulating RNF2-1 off-target. The Spacer coding sequences were shown in Tables 10 and 13.

### Cell culture, transfection, and fluorescence-activated cell sorting (FACS)

HEK293T cells were cultured in DMEM (Gibco) comprising 1% penicillin-streptomycin (Gibco) and 10% fetal calf serum (Gibco). Cells were seeded in 24-cell culture dishes (Corning) for 16 hours until cell density reached 70%. By using Lipofectamine 3000 (Invitrogen), 600 ng of plasmid encoding Cas protein, 300 ng of plasmid encoding crRNA were transfected into cells cultured in a 24-well cell culture dish. After 72 hours of transfection, the cells were digested with trypsin-EDTA (0.05%) (Gibco), and then subjected to GFP fluorescence-activated cell sorting (FACS). The cells that successfully expressed Cas enzyme were sorted.

### Using T7E1 enzyme digestion method to detect the editing efficiency of Cas protein at target sites and off-target sites

FACS-sorted GFP-positive HEK293T cells were lysed with 40 µL of buffer L (bimake), incubated at 55°C for 3 hours, and then at 95°C for 10 minutes. PCR amplification was performed on dsDNA fragments comprising target sites or off-target sites in different genomic sites using the corresponding primers described in Example 10. The sequences of the target positions or off-target positions were shown in Tables 11 and 13. Afterwards, using 10 µL of PCR product, a re-annealing procedure was performed to form heteroduplex dsDNA. The mixture was then treated with 1/10 volume of NEBuffer^{™} 2.1 and 0.2 µL of T7 endonuclease I (NEB) for 50 min at 37°C. Digestion products were analyzed by -2.5% agarose gel electrophoresis. The indel rate (Indel, %) was calculated based on the gray value of the band. Experimental results showed that R857A, R719A, K394A, K844A, R719A/K844A (SEQ ID NO: 23) and R857A/K844A (SEQ ID NO: 24), these single point mutants based on the CasXX sequence can effectively reduce the indel rate at off target sites, EMX1-7-OT-1, EMX1-7-OT-2, and EMX1-7-OT-3, without sacrificing the editing efficiency at the target site, see Table 17, corresponding to Fig. 15. At the same time, R857A, R719A, K394A, K844A, R719A/K844A and R857A/K844A, these mutants based on the CasXX sequence can effectively reduce the indel rate without sacrificing the efficiency at the target site, when using simulated off-target crRNA-RNF2-1-Mis-1/2, crRNA-RNF2-1-Mis-5/6, crRNA-RNF2-1-Mis-17/18 and crRNA-RNF2-1-Mis-19/20, see Table 18, corresponding Fig. 16. Here, we define the reduction in the indel rate at the off-target site and the basically unchanged (or increased) editing efficiency at the target site as an improvement in editing specificity.

**Table 17: On-target and off-target editing efficiencies of different CasXX-HF mutants on EMX1-7**

| CasXX-HF mutant | Mutation scheme | on-target gene editing efficiency on EMX 1-7 | off-target-1 gene editing efficiency on EMX1-7 | off-target-2 gene editing efficiency on EMX 1-7 | off-target-3 gene editing efficiency on EMX1-7 |
|---|---|---|---|---|---|
| **HF-8** | **R857A** | **30.24** | **1.00** | **0.00** | **1.73** |
| **HF-18** | **R719A** | **36.78** | **5.71** | **0.00** | **3.22** |
| **HF-20** | **K394A** | **32.58** | **0.00** | **0.00** | **0.00** |
| **HF-22** | **K844A** | **32.34** | **2.91** | **0.00** | **0.00** |
| HF-18+20 | R719A/K394A | 25.05 | 0.00 | 0.00 | 0.00 |
| HF-20+22 | K394A/K844A | 18.64 | 0.00 | 0.00 | 0.00 |
| HF-8+20 | R857A/K394A | 18.42 | 0.00 | 0.00 | 0.00 |
| **HF-18+22** | **R719A/K844A** | **32.71** | **0.00** | **0.00** | **0.00** |
| HF-8+18 | R857A/R719A | 30.33 | 0.00 | 0.00 | 0.00 |
| **HF-8+22** | **R857A/K844A** | **22.81** | **0.00** | **0.00** | **0.00** |
| HF-18+20+22 | R719A/K394A/ K844A | 0.00 | 0.00 | 0.00 | 0.00 |
| HF-8+18+20 | R857A/R719A/ K394A | 0.00 | 0.00 | 0.00 | 0.00 |
| HF-8+20+22 | R857/K394A/K 844A | 12.61 | 0.00 | 0.00 | 0.00 |
| HF-8+18+22 | R857A/R719A/ K844A | 0.00 | 0.00 | 0.00 | 0.00 |
| HF-8+18+20+22 | R857A/R719A/ K394A/K844A | 0.00 | 0.00 | 0.00 | 0.00 |
| **CasXX** | **reference** | **35.73** | **14.54** | **9.91** | **16.29** |

**Table 18: On-target and off-target editing efficiencies of different CasXX-HF mutants on RNF2-1**

| CasXX-HF mutant | Mutation scheme | RNF2-1 on-target gene editing efficiency on RNF2-1 | Mismatch-1,2 | Mismatch-5,6 | Mismatch -17,18 | Mismatch -19,20 |
|---|---|---|---|---|---|---|
| **HF-8** | **R857A** | **40.45** | **8.50** | **0.00** | **0.00** | **29.50** |
| **HF-18** | **R719A** | **42.46** | **12.62** | **0.00** | **0.00** | **41.69** |
| **HF-20** | **K394A** | **40.26** | **0.00** | **0.00** | **0.00** | **7.22** |
| **HF-22** | **K844A** | **40.34** | **0.00** | **0.00** | **0.00** | **26.86** |
| HF-18+20 | R719A/K394A | 27.46 | 0.00 | 0.00 | 0.00 | 2.40 |
| HF-20+22 | K394A/K844A | 16.79 | 0.00 | 0.00 | 0.00 | 10.00 |
| HF-8+20 | R857A/K394A | 6.93 | 0.00 | 0.00 | 0.00 | 0.00 |
| **HF-18+22** | **R719A/K844A** | **42.26** | **0.00** | **0.00** | **0.00** | **19.31** |
| HF-8+18 | R857A/R719A | 32.49 | 0.00 | 0.00 | 0.00 | 19.08 |
| **HF-8+22** | **R857A/K844A** | **23.54** | **0.00** | **0.00** | **0.00** | **0.00** |
| HF-18+20+22 | R719A/K394A/ K844A | 0.00 | 0.00 | 0.00 | 0.00 | 0.00 |
| HF-8+18+20 | R857A/R719A/ K394A | 0.00 | 0.00 | 0.00 | 0.00 | 0.00 |
| HF-8+20+22 | R857A/K394A/ K844A | 7.51 | 0.00 | 0.00 | 0.00 | 0.00 |
| HF-8+18+22 | R857A/R719A/ K844A | 0.00 | 0.00 | 0.00 | 0.00 | 0.00 |
| HF-8+18+20+22 | R857A/R719A/ K394A/K844A | 0.00 | 0.00 | 0.00 | 0.00 | 0.00 |
| **CasXX** | **reference** | **41.50** | **25.08** | **23.71** | **24.80** | **45.69** |

The mutants shown in SEQ ID NO.23 and SEQ ID NO.24 in this specification (corresponding to the mutants with sequence numbers in Table 16) had significantly higher specificity.

### Example 13: Using GUIDE-Seq to detect off-target effects of CasXX and CasXX+K394A mutants.

### Plasmid construction

The DNA sequences encoding CasXX (SEQ ID NO.8) and CasXX+K394A mutant (SEQ ID NO.20) were loaded into the pCAG-2A-EGFP plasmid, thereby constructing plasmids expressing CasXX or (CasXX+K394A). A vector expressing Cas12i protein crRNA in HEK293T was constructed by ligating the annealed oligonucleotide comprising the target sequence into the BasI-digested pUC19-U6-crRNA backbone. The crRNA was designed to comprise a spacer sequence that can target the endogenous site of CD34-7. The nucleotide sequence encoding DR was SEQ ID NO.59. The nucleotide sequence encoding spacer comprised SEQ ID NO.78.

### Cell culture, transfection, and fluorescence-activated cell sorting (FACS)

HEK293T cells were cultured in DMEM (Gibco) comprising 1% penicillin-streptomycin (Gibco) and 10% fetal calf serum (Gibco). Cells were seeded in 24-cell culture dishes (Corning) for 16 hours until cell density reached 70%. By using Lipofectamine 3000 (Invitrogen), 600 ng of plasmid encoding CasXX or (CasXX + K394A) protein, 300 ng of plasmid encoding crRNA and 10 pmol of annealed double stranded DNA tags (sequence shown in Table 8) were transfected into cells cultured in a 24-well cell culture dish. After 72 hours of transfection, the cells were digested with trypsin-EDTA (0.05%) (Gibco), and then subjected to GFP fluorescence-activated cell sorting (FACS).

### Genome-wide detection of off-target effects of Cas12i2 mutants

Genomes from FACS-sorted GFP-positive HEK293T cells were extracted using the E.Z.N.A.^{®} MicroElute Genomic DNA Kit (Omega). The purified genome was quantified using Qubit. the genome was fragmented to about 500bp by using the Covaris S220 instrument and following the instrument's recommended procedures. Then the DNA library was constructed by using the VAHTS Universal Pro DNA Library Prep Kit for Illumina (Vazyme). For the library construction process, please refer to the reference literature (Tsai, SQ et al. "GUIDE-seq enables genome-wide profiling of offtarget cleavage by CRISPR-Cas nucleases," Nat Biotechnol. 2015;33(2): 187-197). The library construction products were subjected to high-throughput sequencing. Sequencing results were analyzed to look for potential off-target sites. We conducted a systematic analysis of the off-target effects of CasXX and (CasXX+K394A) at the endogenous site of CD34-7. The specific analysis results were shown in Fig. 17. The first row of sequences was the reference target sequence, and the following sequences represented the target sequence and off-target sequence respectively, as well as the number of reads enriched by double-stranded DNA tags in the target sequence and off-target sequence. Fig. 17 illustrated that CasXX had an off-target effect at the CD34-7 site, but the CasXX+K394A mutant had no off-target effect at this site. Therefore, the additional introduction of the K394A point mutation on the basis of the CasXX sequence can greatly improve the fidelity of CasXX, making CasXX highly specific.

Although the embodiments of the present invention have been described above in conjunction with the accompanying drawings, the present invention is not limited to the above-mentioned specific embodiments and application fields. The above-mentioned specific embodiments are only illustrative and instructive, rather than restrictive. Under the guidance of this specification and without departing from the scope of protection of the claims of the present invention, those of ordinary skill in the art can also make many forms, which are all comprised in the protection of the present invention.

### SEQUENCE LISTING

| Number | Amino acid sequence |
|---|---|
| SEQ ID NO.1 | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQNNSIISVLFGTG EKEDRSVKLRITKKILEAISNLKEIPKNVAPIQEIILNVAKATKETFRQVYAGNLGAPSTL EKFIAKDGQKEFDLKKLQTDLKKVIRGKSKERDWCCQEELRSYVEQNTIQYDLWAW GEMFNKAHTALKIKSTRNYNFAKQRLEQFKEIQSLNNLLVVKKLNDFFDSEFFSGEET YTICVHHLGGKDLSKLYKAWEDDPADPENAIVVLCDDLKNNFKKEPIRNILRYIFTIRQ ECSAQDILAAAKYNQQLDRYKSQKANPSVLGNQGFTWTNAVILPEKAQRNDRPNSL DLRIWLYLKLRHPDGRWKKHHIPFYDTRFFQEIYAAGNSPVDTCQFRTPRFGYHLPKL TDQTAIRVNKKHVKAAKTEARIRLAIQQGTLPVSNLKITEISATINSKGQVRIPVKFDV GRQKGTLQIGDRFCGYDQNQTASHAYSLWEVVKEGQYHKELGCFVRFISSGDIVPOSI TIONNRGNQFDQLSYEGLAYPQYADWRKKASKFVSLWQITKKNKKKEIVTVEAKEKF DAICKYQPRLYKFNKEYAYLLRDIVRGKSLVELQQIRQEIFRFIEQDCGVTRLGSLSLST LETVKAVKGIIYSYFSTALNASKNNPISDEQRKEFDPELFALLEKLELIRTRKKKQKVE RIANSLIQTCLENNIKFIRGEGDLSTTNNATKKKANSRSMDWLARGVFNKIRQLAPMH NITLFGCGSLYTSHQDPLVHRNPDKAMKCRWAAIPVKDIGDWVLRKLSQNLRAKNIG TGEYYHQGVKEFLSHYELQDLEEELLKWRSDRKSNIPCWVLQNRLAEKLGNKEAVV YIPVRGGRIYFATHKVATGAVSIVFDQKQVWVCNADHVAAANIALTVKGIGEQSSDEE NPDGSRIKLQLTS |
| SEQ ID NO.2 | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPSTL ERFIAKDGQKEFDLKKLQTDLKKVIRGKSKERDWCCQEELRSYVEQNTIQYDLWAW GEMFNKAHTALKIKSTRNYNFAKQRLEQFKEIQSLNNLLVVKKLNDFFDSEFFSGEET YTICVHHLGGKDLSKLYKAWEDDPADPENAIVVLCDDLKNNFKKEPIRNILRYIFTIRQ |
| | ECSAQDILAAAKYNQQLDRYKSQKANPSVLGNQGFTWRNAVILPEKAQRNDRPNSL DLRIWLYLKLRHPDGRWKKHHIPFYDTRFFQEIYAAGNSPVDTCQFRTPRFGYHLPKL TDQTAIRVNKKHVKAAKTEARIRLAIQQGTLPVSNLKITRISATINSKGQVRIPVKFDV GRQKGTLQIGDRFCGYDQNQTASHAYSLWEVVKEGQYHKELGCFVRFISSGDIVPOSI TIONNRGNQFDQLSYEGLAYPQYADWRKKASKFVSLWQITKKNKKKEIVTVEAKEKF DAICKYQPRLYKFNKEYAYLLRDIVRGKSLVELQQIRQEIFRFIEQDCGVTRLGSLSLST LETVKAVKGIIYSYFSTALNASKNNPISDEQRKEFDPELFALLEKLELIRTRKKKQKVE RIANSLIQTCLENNIKFIRGEGDLSTTNNATKKKANSRSMDWLARGVFNKIRQLAPMH NITLFGCGSLYTSHQDPLVHRNPDKAMKCRWAAIPVKDIGDWVLRKLSQNLRAKNIG TGEYYHQGVKEFLSHYELQDLEEELLKWRSDRKSNIPCWVLQNRLAEKLGNKEAVV YIPVRGGRIYFATHKVATGAVSIVFDQKQVWVCNADHVAAANIALTVKGIGEQSSDEE NPDGSRIKLQLTS |
| SEQ ID NO.3 | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQNNSIISVLFGTG RKEDRSVKLRITKKILEAISNLKEIPKNVAPIQEIILNVAKATKETFRQVYAGNLGAPSTL ERFIAKDGQKEFDLKKLQTDLKKVIRGKSKERDWCCQEELRSYVEQNTIQYDLWAW GEMFNKAHTALKIKSTRNYNFAKQRLEQFKEIQSLNNLLVVKKLNDFFDSEFFSGEET YTICVHHLGGKDLSKLYKAWEDDPADPENAIVVLCDDLKNNFKKEPIRNILRYIFTIRQ ECSAQDILAAAKYNQQLDRYKSQKANPSVLGNQGFTWRNAVILPEKAQRNDRPNSL DLRIWLYLKLRHPDGRWKKHHIPFYDTRFFQEIYAAGNSPVDTCQFRTPRFGYHLPKL TDQTAIRVNKKHVKAAKTEARIRLAIQQGTLPVSNLKITRISATINSKGQVRIPVKFDV GRQKGTLQIGDRFCGYDQNQTASHAYSLWEVVKEGQYHKELGCFVRFISSGDIVPOSI TIONNRGNQFDQLSYEGLAYPQYADWRKKASKFVSLWQITKKNKKKEIVTVEAKEKF DAICKYQPRLYKFNKEYAYLLRDIVRGKSLVELQQIRQEIFRFIEQDCGVTRLGSLSLST LETVKAVKGIIYSYFSTALNASKNNPISDEQRKEFDPELFALLEKLELIRTRKKKQKVE RIANSLIQTCLENNIKFIRGEGDLSTTNNATKKKANSRSMDWLARGVFNKIRQLAPMH NITLFGCGSLYTSHQDPLVHRNPDKAMKCRWAAIPVKDIGDWVLRKLSQNLRAKNR |
| | GTGEYYHQGVKEFLSHYELQDLEEELLKWRSDRKSNIPCWVLQNRLAEKLGNKEAV VYIPVRGGRIYFATHKVATGAVSIVFDQKQVWVCNADHVAAANIALTVKGIGEQSSDE ENPDGSRIKLQLTS |
| SEQ ID NO.4 | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQNNSIISVLFGTG RKEDRSVKLRITKKILEAISNLKEIPKNVAPIQEIILNVAKATKETFRQVYAGNLGAPSTL ERFIAKDGQKEFDLKKLQTDLKKVIRGKSKERDWCCQEELRSYVEQNTIQYDLWAW GEMFNKAHTALKIKSTRNYNFAKQRLEQFKRIQSLNNLLVVKKLNDFFDSEFFSGEET YTICVHHLGGKRLSKLYKAWEDDPADPENAIVVLCDDLKNNFKKEPIRNILRYIFTIRQ ECSAQDILAAAKYNQQLDRYKSQKANPSVLGNQGFTWRNAVILPEKAQRNDRPNSL DLRIWLYLKLRHPDGRWKKHHIPFYDTRFFQEIYAAGNSPVDTCQFRTPRFGYHLPKL TDQTAIRVNKKHVKAAKTEARIRLAIQQGTLPVSNLKITRISATINSKGQVRIPVKFDV GRQKGTLQIGDRFCGYDQNQTASHAYSLWEVVKEGQYHKELGCFVRFISSGDIVPOSI TIONNRGNQFDQLSYEGLAYPQYADWRKKASKFVSLWQITKKNKKKEIVTVEAKEKF DAICKYQPRLYKFNKEYAYLLRDIVRGKSLVELQQIRQEIFRFIEQDCGVTRLGSLSLST LETVKAVKGIIYSYFSTALNASKNNPISDEQRKEFDPELFALLEKLELIRTRKKKQKVE RIANSLIQTCLENNIKFIRGEGDLSTTNNATKKKANSRSMDWLARGVFNKIRQLAPMH NITLFGCGSLYTSHQDPLVHRNPDKAMKCRWAAIPVKDIGDWVLRKLSQNLRAKNIG TGEYYHQGVKEFLSHYELQDLEEELLKWRSDRKSNIPCWVLQNRLAEKLGNKEAVV YIPVRGGRIYFATHKVATGAVSIVFDQKQVWVCNADHVAAANIALTVKGIGEQSSDEE NPDGSRIKLQLTS |
| SEQ ID NO. 5 | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG EKEDRSVKLRITKKILEAISNLKEIPKNVAPIQEIILNVAKATKETFRQVYAGNLGAPSTL EKFIAKDGQKEFDLKKLQTDLKKVIRGKSKERDWCCQEELRSYVEQNTIQYDLWAW GEMFNKAHTALKIKSTRNYNFAKQRLEQFKEIQSLNNLLVVKKLNDFFDSEFFSGEET |
| | YTICVHHLGGKDLSKLYKAWEDDPADPENAIVVLCDDLKNNFKKEPIRNILRYIFTIRQ ECSAQDILAAAKYNQQLDRYKSQKANPSVLGNQGFTWTNAVILPEKAQRNDRPNSL DLRIWLYLKLRHPDGRWKKHHIPFYDTRFFQEIYAAGNSPVDTCQFRTPRFGYHLPKL TDQTAIRVNKKHVKAAKTEARIRLAIQQGTLPVSNLKITEISATINSKGQVRIPVKFDV GRQKGTLQIGDRFCGYDQNQTASHAYSLWEVVKEGQYHKELGCFVRFISSGDIVPOSI TIONNRGNQFDQLSYEGLAYPQYADWRKKASKFVSLWQITKKNKKKEIVTVEAKEKF DAICKYQPRLYKFNKEYAYLLRDIVRGKSLVELQQIRQEIFRFIEQDCGVTRLGSLSLST LETVKAVKGIIYSYFSTALNASKNNPISDEQRKEFDPELFALLEKLELIRTRKKKQKVE RIANSLIQTCLENNIKFIRGEGDLSTTNNATKKKANSRSMDWLARGVFNKIRQLAPMH NITLFGCGSLYTSHQDPLVHRNPDKAMKCRWAAIPVKDIGDWVLRKLSQNLRAKNR GTGEYYHQGVKEFLSHYELQDLEEELLKWRSDRKSNIPCWVLQNRLAEKLGNKEAV VYIPVRGGRIYFATHKVATGAVSIVFDQKQVWVCNADHVAAANIALTVKGIGEQSSDE ENPDGSRIKLQLTS |
| SEQ ID NO. 6 | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG EKEDRSVKLRITKKILEAISNLKEIPKNVAPIQEIILNVAKATKETFRQVYAGNLGAPSTL EKFIAKDGQKEFDLKKLQTDLKKVIRGKSKERDWCCQEELRSYVEQNTIQYDLWAW GEMFNKAHTALKIKSTRNYNFAKQRLEQFKRIQSLNNLLVVKKLNDFFDSEFFSGEET YTICVHHLGGKRLSKLYKAWEDDPADPENAIVVLCDDLKNNFKKEPIRNILRYIFTIRQ ECSAQDILAAAKYNQQLDRYKSQKANPSVLGNQGFTWTNAVILPEKAQRNDRPNSL DLRIWLYLKLRHPDGRWKKHHIPFYDTRFFQEIYAAGNSPVDTCQFRTPRFGYHLPKL TDQTAIRVNKKHVKAAKTEARIRLAIQQGTLPVSNLKITEISATINSKGQVRIPVKFDV GRQKGTLQIGDRFCGYDQNQTASHAYSLWEVVKEGQYHKELGCFVRFISSGDIVPOSI TIONNRGNQFDQLSYEGLAYPQYADWRKKASKFVSLWQITKKNKKKEIVTVEAKEKF DAICKYQPRLYKFNKEYAYLLRDIVRGKSLVELQQIRQEIFRFIEQDCGVTRLGSLSLST LETVKAVKGIIYSYFSTALNASKNNPISDEQRKEFDPELFALLEKLELIRTRKKKQKVE RIANSLIQTCLENNIKFIRGEGDLSTTNNATKKKANSRSMDWLARGVFNKIRQLAPMH |
| | NITLFGCGSLYTSHQDPLVHRNPDKAMKCRWAAIPVKDIGDWVLRKLSQNLRAKNIG TGEYYHQGVKEFLSHYELQDLEEELLKWRSDRKSNIPCWVLQNRLAEKLGNKEAVV YIPVRGGRIYFATHKVATGAVSIVFDQKQVWVCNADHVAAANIALTVKGIGEQSSDEE NPDGSRIKLQLTS |
| SEQ ID NO. 7 | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPSTL ERFIAKDGQKEFDLKKLQTDLKKVIRGKSKERDWCCQEELRSYVEQNTIQYDLWAW GEMFNKAHTALKIKSTRNYNFAKQRLEQFKEIQSLNNLLVVKKLNDFFDSEFFSGEET YTICVHHLGGKDLSKLYKAWEDDPADPENAIVVLCDDLKNNFKKEPIRNILRYIFTIRQ ECSAQDILAAAKYNQQLDRYKSQKANPSVLGNQGFTWRNAVILPEKAQRNDRPNSL DLRIWLYLKLRHPDGRWKKHHIPFYDTRFFQEIYAAGNSPVDTCQFRTPRFGYHLPKL TDQTAIRVNKKHVKAAKTEARIRLAIQQGTLPVSNLKITRISATINSKGQVRIPVKFDV GRQKGTLQIGDRFCGYDQNQTASHAYSLWEVVKEGQYHKELGCFVRFISSGDIVPOSI TIONNRGNQFDQLSYEGLAYPQYADWRKKASKFVSLWQITKKNKKKEIVTVEAKEKF DAICKYQPRLYKFNKEYAYLLRDIVRGKSLVELQQIRQEIFRFIEQDCGVTRLGSLSLST LETVKAVKGIIYSYFSTALNASKNNPISDEQRKEFDPELFALLEKLELIRTRKKKQKVE RIANSLIQTCLENNIKFIRGEGDLSTTNNATKKKANSRSMDWLARGVFNKIRQLAPMH NITLFGCGSLYTSHQDPLVHRNPDKAMKCRWAAIPVKDIGDWVLRKLSQNLRAKNR GTGEYYHQGVKEFLSHYELQDLEEELLKWRSDRKSNIPCWVLQNRLAEKLGNKEAV VYIPVRGGRIYFATHKVATGAVSIVFDQKQVWVCNADHVAAANIALTVKGIGEQSSDE ENPDGSRIKLQLTS |
| SEQ ID NO.8 | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPSTL ERFIAKDGQKEFDLKKLQTDLKKVIRGKSKERDWCCQEELRSYVEQNTIQYDLWAW |
| | GEMFNKAHTALKIKSTRNYNFAKQRLEQFKRIQSLNNLLVVKKLNDFFDSEFFSGEET YTICVHHLGGKRLSKLYKAWEDDPADPENAIVVLCDDLKNNFKKEPIRNILRYIFTIRQ ECSAQDILAAAKYNQQLDRYKSQKANPSVLGNQGFTWRNAVILPEKAQRNDRPNSL DLRIWLYLKLRHPDGRWKKHHIPFYDTRFFQEIYAAGNSPVDTCQFRTPRFGYHLPKL TDQTAIRVNKKHVKAAKTEARIRLAIQQGTLPVSNLKITRISATINSKGQVRIPVKFDV GRQKGTLQIGDRFCGYDQNQTASHAYSLWEVVKEGQYHKELGCFVRFISSGDIVPOSI TIONNRGNQFDQLSYEGLAYPQYADWRKKASKFVSLWQITKKNKKKEIVTVEAKEKF DAICKYQPRLYKFNKEYAYLLRDIVRGKSLVELQQIRQEIFRFIEQDCGVTRLGSLSLST LETVKAVKGIIYSYFSTALNASKNNPISDEQRKEFDPELFALLEKLELIRTRKKKQKVE RIANSLIQTCLENNIKFIRGEGDLSTTNNATKKKANSRSMDWLARGVFNKIRQLAPMH NITLFGCGSLYTSHQDPLVHRNPDKAMKCRWAAIPVKDIGDWVLRKLSQNLRAKNIG TGEYYHQGVKEFLSHYELQDLEEELLKWRSDRKSNIPCWVLQNRLAEKLGNKEAVV YIPVRGGRIYFATHKVATGAVSIVFDQKQVWVCNADHVAAANIALTVKGIGEQSSDEE NPDGSRIKLQLTS |
| SEQ ID NO. 9 | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPSTL ERFIAKDGQKEFDLKKLQTDLKKVIRGKSKERDWCCQEELRSYVEQNTIQYDLWAW GEMFNKAHTALKIKSTRNYNFAKQRLEQFKRIQSLNNLLVVKKLNDFFDSEFFSGEET YTICVHHLGGKRLSKLYKAWEDDPADPENAIVVLCDDLKNNFKKEPIRNILRYIFTIRQ ECSAQDILAAAKYNQQLDRYKSQKANPSVLGNQGFTWRNAVILPEKAQRNDRPNSL DLRIWLYLKLRHPDGRWKKHHIPFYDTRFFQEIYAAGNSPVDTCQFRTPRFGYHLPKL TDQTAIRVNKKHVKAAKTEARIRLAIQQGTLPVSNLKITRISATINSKGQVRIPVKFDV GRQKGTLQIGDRFCGYDQNQTASHAYSLWEVVKEGQYHKELGCFVRFISSGDIVPOSI TIONNRGNQFDQLSYEGLAYPQYADWRKKASKFVSLWQITKKNKKKEIVTVEAKEKF DAICKYQPRLYKFNKEYAYLLRDIVRGKSLVELQQIRQEIFRFIEQDCGVTRLGSLSLST LETVKAVKGIIYSYFSTALNASKNNPISDEQRKEFDPELFALLEKLELIRTRKKKQKVE |
| | RIANSLIQTCLENNIKFIRGEGDLSTTNNATKKKANSRSMDWLARGVFNKIRQLAPMH NITLFGCGSLYTSHQDPLVHRNPDKAMKCRWAAIPVKDIGDWVLRKLSQNLRAKNR GTGEYYHQGVKEFLSHYELQDLEEELLKWRSDRKSNIPCWVLQNRLAEKLGNKEAV VYIPVRGGRIYFATHKVATGAVSIVFDQKQVWVCNADHVAAANIALTVKGIGEQSSDE ENPDGSRIKLQLTS |
| SEQ ID NO.10 | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPSTL ERFIAKDGQKEFDLKKLQTDLKKVIRGKSKERDWCCQEELRSYVEQNTIQYDLWAW GEMFNKAHTALKIKSTRNYNFAKQRLEQFKRIQSLNNLLVVKKLNDFFDSEFFSGEET YTICVHHLGGKRLSKLYKAWEDDPADPENAIVVLCDDLKNNFKKEPIRNILRYIFTIRQ ECSAQDILAAAKYNQQLDRYKSQKANPSVLGNQGFTWRNAVILPEKAQRNDRPNSL DLRIWLYLKLRHPDGRWKKHHIPFYDTRFFQEIYAAGNSPVDTCQFRTPRFGYHLPKL TDQTAIRVNKKHVKAAKTEARIRLAIQQGTLPVSNLKITRISATINSKGQVRIPVKFDV GRQKGTLQIGDRFCGYDQNQTASHAYSLWEVVKEGQYHKELGCFVRFISSGDIVPOSI TIONNRGNQFDQLSYEGLAYPQYADWRKKASKFVSLWQITKKNKKKEIVTVEAKEKF DAICKYQPRLYKFNKEYAYLLRDIVRGKSLVELQQIRQEIFRFIEQDCGVTRLGSLSLST LETVKAVKGIIYSYFSTALNASKNNPISDEQRKEFDPELFALLEKLELIRTRKKKQKVE RIANSLIQTCLENNIKFIRGEGDLSTTNNATKKKANSRSMDWLARGVFNKIRQLAPMH NITLFGCGSLYTSHQDPLVHRNPDKAMKCRWAAIPVKDIGDWVLRKLSQNLRAKNG GTGEYYHQGVKEFLSHYELQDLEEELLKWRSDRKSNIPCWVLQNRLAEKLGNKEAV VYIPVRGGRIYFATHKVATGAVSIVFDQKQVWVCNADHVAAANIALTVKGIGEQSSDE ENPDGSRIKLQLTS |
| SEQ ID NO. 11 | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPSTL |
| | ERFIAKDGQKEFDLKKLQTDLKKVIRGKSKERDWCCQEELRSYVEQNTIQYDLWAW GEMFNKAHTALKIKSTRNYNFAKQRLEQFKRIQSLNNLLVVKKLNDFFDSEFFSGEET YTICVHHLGGKRLSKLYKAWEDDPADPENAIVVLCDDLKNNFKKEPIRNILRYIFTIRQ ECSAQDILAAAKYNQQLDRYKSQKANPSVLGGGNQGFTWRNAVILPEKAQRNDRPN SLDLRIWLYLKLRHPDGRWKKHHIPFYDTRFFQEIYAAGNSPVDTCQFRTPRFGYHLP KLTDQTAIRVNKKHVKAAKTEARIRLAIQQGTLPVSNLKITRISATINSKGQVRIPVKFD VGRQKGTLQIGDRFCGYDQNQTASHAYSLWEVVKEGQYHKELGCFVRFISSGDIVPO SITIONNRGNQFDQLSYEGLAYPQYADWRKKASKFVSLWQITKKNKKKEIVTVEAKE KFDAICKYQPRLYKFNKEYAYLLRDIVRGKSLVELQQIRQEIFRFIEQDCGVTRLGSLSL STLETVKAVKGIIYSYFSTALNASKNNPISDEQRKEFDPELFALLEKLELIRTRKKKQKV ERIANSLIQTCLENNIKFIRGEGDLSTTNNATKKKANSRSMDWLARGVFNKIRQLAPM HNITLFGCGSLYTSHQDPLVHRNPDKAMKCRWAAIPVKDIGDWVLRKLSQNLRAKN GGTGEYYHQGVKEFLSHYELQDLEEELLKWRSDRKSNIPCWVLQNRLAEKLGNKEA VVYIPVRGGRIYFATHKVATGAVSIVFDQKQVWVCNADHVAAANIALTVKGIGEQSSD EENPDGSRIKLQLTS |
| SEQ ID NO.12 | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPSTL ERFIAKDGQKEFDLKKLQTDLKKVIRGKSKERDWCCQEELRSYVEQNTIQYDLWAW GEMFNKAHTALKIKSTRNYNFAKQRLEQFKRIQSLNNLLVVKKLNDFFDSEFFSGEET YTICVHHLGGKRLSKLYKAWEDDPADPENAIVVLCDDLKNNFKKEPIRNILRYIFTIRQ ECSAQDILAAAKYNQQLDRYKSQKANPSVLGGNQGFTWRNAVILPEKAQRNDRPNS LDLRIWLYLKLRHPDGRWKKHHIPFYDTRFFQEIYAAGNSPVDTCQFRTPRFGYHLPK LTDQTAIRVNKKHVKAAKTEARIRLAIQQGTLPVSNLKITRISATINSKGQVRIPVKFDV GRQKGTLQIGDRFCGYDQNQTASHAYSLWEVVKEGQYHKELGCFVRFISSGDIVPOSI TIONNRGNQFDQLSYEGLAYPQYADWRKKASKFVSLWQITKKNKKEIVTVEAKEKF DAICKYQPRLYKFNKEYAYLLRDIVRGKSLVELQQIRQEIFRFIEQDCGVTRLGSLSLST |
| | LETVKAVKGIIYSYFSTALNASKNNPISDEQRKEFDPELFALLEKLELIRTRKKKQKVE RIANSLIQTCLENNIKFIRGEGDLSTTNNATKKKANSRSMDWLARGVFNKIRQLAPMH NITLFGCGSLYTSHQDPLVHRNPDKAMKCRWAAIPVKDIGDWVLRKLSQNLRAKNG GTGEYYHQGVKEFLSHYELQDLEEELLKWRSDRKSNIPCWVLQNRLAEKLGNKEAV VYIPVRGGRIYFATHKVATGAVSIVFDQKQVWVCNADHVAAANIALTVKGIGEQSSDE ENPDGSRIKLQLTS |
| SEQ ID NO. 13 | MSNKEKNASETRKAYTTKMIPRSHDRMKLLGNFMDYLMDGTPIFFELWNQFGGGID RDIISGTANKDKISDDLLLAVNWFKVMPINSKPQGVSPSNLANLFQQYSGSEPDIQAQ EYFASNFDTEKHQWKDMRVEYERLLAELQLSRSDMHHDLKLMYKEKCIGLSLSTAH YITSVMFGTGAKNNRQTKHQFYSKVIQLLEESTQINSVEQLASIILKAGDCDSYRKLRI RCSRKGATPSILKIVQDYELGTNHDDEVNVPSLIANLKEKLGRFEYECEWKCMEKIK AFLASKVGPYYLGSYSAMLENALSPIKGMTTKNCKFVLKQIDAKNDIKYENEPFGKI VEGFFDSPYFESDTNVKWVLHPHHIGESNIKTLWEDLNAIHSKYEEDIASLSEDKKEK RIKVYQGDVCQTINTYCEEVGKEAKTPLVQLLRYLYSRKDDIAVDKIIDGITFLSKKH KVEKQKINPVIQKYPSFNFGNNSKLLGKIISPKDKLKHNLKCNRNQVDNYIWIEIKVL NTKTMRWEKHHYALSSTRFLEEVYYPATSENPPDALAARFRTKTNGYEGKPALSAE QIEQIRSAPVGLRKVKKRQMRLEAARQQNLLPRYTWGKDFNINICKRGNNFEVTLAT KVKKKKEKNYKVVLGYDANIVRKNTYAAIEAHANGDGVIDYNDLPVKPIESGFVTV ESQVRDKSYDQLSYNGVKLLYCKPHVESRRSFLEKYRNGTMKDNRGNNIQIDFMKD FEAIADDETSLYYFNMKYCKLLQSSIRNHSSQAKEYREEIFELLRDGKLSVLKLSSLSN LSFVMFKVAKSLIGTYFGHLLKKPKNSKSDVKAPPITDEDKQKADPEMFALRLALEE KRLNKVKSKKEVIANKIVAKALELRDKYGPVLIKGENISDTTKKGKKSSTNSFLMDW LARGVANKVKEMVMMHQGLEFVEVNPNFTSHQDPFVHKNPENTFRARYSRCTPSEL TEKNRKEILSFLSDKPSKRPTNAYYNEGAMAFLATYGLKKNDVLGVSLEKFKQIMAN ILHQRSEDQLLFPSRGGMFYLATYKLDADATSVNWNGKQFWVCNADLVAAYNVGL VDIQKDFKKK |
| SEQ ID NO. 14 CasXX - R857A | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE |
| | SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPST LERFIAKDGQKEFDLKKLQTDLKKVIRGKSKERDWCCQEELRSYVEQNTIQYDLWA WGEMFNKAHTALKIKSTRNYNFAKQRLEQFKRIQSLNNLLVVKKLNDFFDSEFFSGE ETYTICVHHLGGKRLSKLYKAWEDDPADPENAIVVLCDDLKNNFKKEPIRNILRYIFTI RQECSAQDILAAAKYNQQLDRYKSQKANPSVLGNQGFTWRNAVILPEKAQRNDRPN SLDLRIWLYLKLRHPDGRWKKHHIPFYDTRFFQEIYAAGNSPVDTCQFRTPRFGYHLP KLTDQTAIRVNKKHVKAAKTEARIRLAIQQGTLPVSNLKITRISATINSKGQVRIPVKF DVGRQKGTLQIGDRFCGYDQNQTASHAYSLWEVVKEGQYHKELGCFVRFISSGDIVP OSITIONNRGNQFDQLSYEGLAYPQYADWRKKASKFVSLWQITKKNKKKEIVTVEAK EKFDAICKYQPRLYKFNKEYAYLLRDIVRGKSLVELQQIRQEIFRFIEQDCGVTRLGSLS LSTLETVKAVKGIIYSYFSTALNASKNNPISDEQRKEFDPELFALLEKLELIRTRKKKQK VERIANSLIQTCLENNIKFIRGEGDLSTTNNATKKKANSRSMDWLAAGVFNKIRQLAP MHNITLFGCGSLYTSHQDPLVHRNPDKAMKCRWAAIPVKDIGDWVLRKLSQNLRAK NIGTGEYYHQGVKEFLSHYELQDLEEELLKWRSDRKSNIPCWVLQNRLAEKLGNKE AVVYIPVRGGRIYFATHKVATGAVSIVFDQKQVWVCNADHVAAANIALTVKGIGEQSS DEENPDGSRIKLQLTS |
| SEQ ID NO. 15 CasXX - N861A | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPST LERFIAKDGQKEFDLKKLQTDLKKVIRGKSKERDWCCQEELRSYVEQNTIQYDLWA WGEMFNKAHTALKIKSTRNYNFAKQRLEQFKRIQSLNNLLVVKKLNDFFDSEFFSGE ETYTICVHHLGGKRLSKLYKAWEDDPADPENAIVVLCDDLKNNFKKEPIRNILRYIFTI RQECSAQDILAAAKYNQQLDRYKSQKANPSVLGNQGFTWRNAVILPEKAQRNDRPN SLDLRIWLYLKLRHPDGRWKKHHIPFYDTRFFQEIYAAGNSPVDTCQFRTPRFGYHLP KLTDQTAIRVNKKHVKAAKTEARIRLAIQQGTLPVSNLKITRISATINSKGQVRIPVKF DVGRQKGTLQIGDRFCGYDQNQTASHAYSLWEVVKEGQYHKELGCFVRFISSGDIVP |
| | OSITIONNRGNQFDQLSYEGLAYPQYADWRKKASKFVSLWQITKKNKKKEIVTVEAK EKFDAICKYQPRLYKFNKEYAYLLRDIVRGKSLVELQQIRQEIFRFIEQDCGVTRLGSLS LSTLETVKAVKGIIYSYFSTALNASKNNPISDEQRKEFDPELFALLEKLELIRTRKKKQK VERIANSLIQTCLENNIKFIRGEGDLSTTNNATKKKANSRSMDWLARGVFAKIRQLAP MHNITLFGCGSLYTSHQDPLVHRNPDKAMKCRWAAIPVKDIGDWVLRKLSQNLRAK NIGTGEYYHQGVKEFLSHYELQDLEEELLKWRSDRKSNIPCWVLQNRLAEKLGNKE AVVYIPVRGGRIYFATHKVATGAVSIVFDQKQVWVCNADHVAAANIALTVKGIGEQSS DEENPDGSRIKLQLTS |
| SEQ ID NO. 16 CasXX - K807A | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPST LERFIAKDGQKEFDLKKLQTDLKKVIRGKSKERDWCCQEELRSYVEQNTIQYDLWA WGEMFNKAHTALKIKSTRNYNFAKQRLEQFKRIQSLNNLLVVKKLNDFFDSEFFSGE ETYTICVHHLGGKRLSKLYKAWEDDPADPENAIVVLCDDLKNNFKKEPIRNILRYIFTI RQECSAQDILAAAKYNQQLDRYKSQKANPSVLGNQGFTWRNAVILPEKAQRNDRPN SLDLRIWLYLKLRHPDGRWKKHHIPFYDTRFFQEIYAAGNSPVDTCQFRTPRFGYHLP KLTDQTAIRVNKKHVKAAKTEARIRLAIQQGTLPVSNLKITRISATINSKGQVRIPVKF DVGRQKGTLQIGDRFCGYDQNQTASHAYSLWEVVKEGQYHKELGCFVRFISSGDIVP OSITIONNRGNQFDQLSYEGLAYPQYADWRKKASKFVSLWQITKKNKKKEIVTVEAK EKFDAICKYQPRLYKFNKEYAYLLRDIVRGKSLVELQQIRQEIFRFIEQDCGVTRLGSLS LSTLETVKAVKGIIYSYFSTALNASKNNPISDEQRKEFDPELFALLEKLELIRTRKAKQK VERIANSLIQTCLENNIKFIRGEGDLSTTNNATKKKANSRSMDWLARGVFNKIRQLAP MHNITLFGCGSLYTSHQDPLVHRNPDKAMKCRWAAIPVKDIGDWVLRKLSQNLRAK NIGTGEYYHQGVKEFLSHYELQDLEEELLKWRSDRKSNIPCWVLQNRLAEKLGNKE AVVYIPVRGGRIYFATHKVATGAVSIVFDQKQVWVCNADHVAAANIALTVKGIGEQSS DEENPDGSRIKLQLTS |
| SEQ ID NO. 17 CasXX - N848A | MSSAIKSYKSVLRPNERKNQLLKSTIQCLEDGSAFFFKMLQGLFGGITPEIVRFSTEQE |
| | KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPST LERFIAKDGQKEFDLKKLQTDLKKVIRGKSKERDWCCQEELRSYVEQNTIQYDLWA WGEMFNKAHTALKIKSTRNYNFAKQRLEQFKRIQSLNNLLVVKKLNDFFDSEFFSGE ETYTICVHHLGGKRLSKLYKAWEDDPADPENAIVVLCDDLKNNFKKEPIRNILRYIFTI RQECSAQDILAAAKYNQQLDRYKSQKANPSVLGNQGFTWRNAVILPEKAQRNDRPN SLDLRIWLYLKLRHPDGRWKKHHIPFYDTRFFQEIYAAGNSPVDTCQFRTPRFGYHLP KLTDQTAIRVNKKHVKAAKTEARIRLAIQQGTLPVSNLKITRISATINSKGQVRIPVKF DVGRQKGTLQIGDRFCGYDQNQTASHAYSLWEVVKEGQYHKELGCFVRFISSGDIVP OSITIONNRGNQFDQLSYEGLAYPQYADWRKKASKFVSLWQITKKNKKKEIVTVEAK EKFDAICKYQPRLYKFNKEYAYLLRDIVRGKSLVELQQIRQEIFRFIEQDCGVTRLGSLS LSTLETVKAVKGIIYSYFSTALNASKNNPISDEQRKEFDPELFALLEKLELIRTRKKKQK VERIANSLIQTCLENNIKFIRGEGDLSTTNNATKKKAASRSMDWLARGVFNKIRQLAP MHNITLFGCGSLYTSHQDPLVHRNPDKAMKCRWAAIPVKDIGDWVLRKLSQNLRAK NIGTGEYYHQGVKEFLSHYELQDLEEELLKWRSDRKSNIPCWVLQNRLAEKLGNKE AVVYIPVRGGRIYFATHKVATGAVSIVFDQKQVWVCNADHVAAANIALTVKGIGEQSS DEENPDGSRIKLQLTS |
| SEQ ID NO. 18 CasXX - R715A | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPST LERFIAKDGQKEFDLKKLQTDLKKVIRGKSKERDWCCQEELRSYVEQNTIQYDLWA WGEMFNKAHTALKIKSTRNYNFAKQRLEQFKRIQSLNNLLVVKKLNDFFDSEFFSGE ETYTICVHHLGGKRLSKLYKAWEDDPADPENAIVVLCDDLKNNFKKEPIRNILRYIFTI RQECSAQDILAAAKYNQQLDRYKSQKANPSVLGNQGFTWRNAVILPEKAQRNDRPN SLDLRIWLYLKLRHPDGRWKKHHIPFYDTRFFQEIYAAGNSPVDTCQFRTPRFGYHLP KLTDQTAIRVNKKHVKAAKTEARIRLAIQQGTLPVSNLKITRISATINSKGQVRIPVKF |
| | DVGRQKGTLQIGDRFCGYDQNQTASHAYSLWEVVKEGQYHKELGCFVRFISSGDIVP OSITIONNRGNQFDQLSYEGLAYPQYADWRKKASKFVSLWQITKKNKKKEIVTVEAK EKFDAICKYQPRLYKFNKEYAYLLADIVRGKSLVELQQIRQEIFRFIEQDCGVTRLGSL SLSTLETVKAVKGIIYSYFSTALNASKNNPISDEQRKEFDPELFALLEKLELIRTRKKKQ KVERIANSLIQTCLENNIKFIRGEGDLSTTNNATKKKANSRSMDWLARGVFNKIRQLA PMHNITLFGCGSLYTSHQDPLVHRNPDKAMKCRWAAIPVKDIGDWVLRKLSQNLRA KNIGTGEYYHQGVKEFLSHYELQDLEEELLKWRSDRKSNIPCWVLQNRLAEKLGNK EAVVYIPVRGGRIYFATHKVATGAVSIVFDQKQVWVCNADHVAAANIALTVKGIGEQS SDEENPDGSRIKLQLTS |
| SEQ ID NO. 19 CasXX - R719A | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPST LERFIAKDGQKEFDLKKLQTDLKKVIRGKSKERDWCCQEELRSYVEQNTIQYDLWA WGEMFNKAHTALKIKSTRNYNFAKQRLEQFKRIQSLNNLLVVKKLNDFFDSEFFSGE ETYTICVHHLGGKRLSKLYKAWEDDPADPENAIVVLCDDLKNNFKKEPIRNILRYIFTI RQECSAQDILAAAKYNQQLDRYKSQKANPSVLGNQGFTWRNAVILPEKAQRNDRPN SLDLRIWLYLKLRHPDGRWKKHHIPFYDTRFFQEIYAAGNSPVDTCQFRTPRFGYHLP KLTDQTAIRVNKKHVKAAKTEARIRLAIQQGTLPVSNLKITRISATINSKGQVRIPVKF DVGRQKGTLQIGDRFCGYDQNQTASHAYSLWEVVKEGQYHKELGCFVRFISSGDIVP OSITIONNRGNQFDQLSYEGLAYPQYADWRKKASKFVSLWQITKKNKKKEIVTVEAK EKFDAICKYQPRLYKFNKEYAYLLRDIVAGKSLVELQQIRQEIFRFIEQDCGVTRLGSL SLSTLETVKAVKGIIYSYFSTALNASKNNPISDEQRKEFDPELFALLEKLELIRTRKKKQ KVERIANSLIQTCLENNIKFIRGEGDLSTTNNATKKKANSRSMDWLARGVFNKIRQLA PMHNITLFGCGSLYTSHQDPLVHRNPDKAMKCRWAAIPVKDIGDWVLRKLSQNLRA KNIGTGEYYHQGVKEFLSHYELQDLEEELLKWRSDRKSNIPCWVLQNRLAEKLGNK EAVVYIPVRGGRIYFATHKVATGAVSIVFDQKQVWVCNADHVAAANIALTVKGIGEQS SDEENPDGSRIKLQLTS |
| SEQ ID NO. 20 CasXX - K394A | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPST LERFIAKDGQKEFDLKKLQTDLKKVIRGKSKERDWCCQEELRSYVEQNTIQYDLWA WGEMFNKAHTALKIKSTRNYNFAKQRLEQFKRIQSLNNLLVVKKLNDFFDSEFFSGE ETYTICVHHLGGKRLSKLYKAWEDDPADPENAIVVLCDDLKNNFKAEPIRNILRYIFTI RQECSAQDILAAAKYNQQLDRYKSQKANPSVLGNQGFTWRNAVILPEKAQRNDRPN SLDLRIWLYLKLRHPDGRWKKHHIPFYDTRFFQEIYAAGNSPVDTCQFRTPRFGYHLP KLTDQTAIRVNKKHVKAAKTEARIRLAIQQGTLPVSNLKITRISATINSKGQVRIPVKF DVGRQKGTLQIGDRFCGYDQNQTASHAYSLWEVVKEGQYHKELGCFVRFISSGDIVP OSITIONNRGNQFDQLSYEGLAYPQYADWRKKASKFVSLWQITKKNKKKEIVTVEAK EKFDAICKYQPRLYKFNKEYAYLLRDIVRGKSLVELQQIRQEIFRFIEQDCGVTRLGSLS LSTLETVKAVKGIIYSYFSTALNASKNNPISDEQRKEFDPELFALLEKLELIRTRKKKQK VERIANSLIQTCLENNIKFIRGEGDLSTTNNATKKKANSRSMDWLARGVFNKIRQLAP MHNITLFGCGSLYTSHQDPLVHRNPDKAMKCRWAAIPVKDIGDWVLRKLSQNLRAK NIGTGEYYHQGVKEFLSHYELQDLEEELLKWRSDRKSNIPCWVLQNRLAEKLGNKE AVVYIPVRGGRIYFATHKVATGAVSIVFDQKQVWVCNADHVAAANIALTVKGIGEQSS DEENPDGSRIKLQLTS |
| SEQ ID NO. 21 CasXX - H357A | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPST LERFIAKDGQKEFDLKKLQTDLKKVIRGKSKERDWCCQEELRSYVEQNTIQYDLWA WGEMFNKAHTALKIKSTRNYNFAKQRLEQFKRIQSLNNLLVVKKLNDFFDSEFFSGE ETYTICVHALGGKRLSKLYKAWEDDPADPENAIVVLCDDLKNNFKKEPIRNILRYIFTI RQECSAQDILAAAKYNQQLDRYKSQKANPSVLGNQGFTWRNAVILPEKAQRNDRPN SLDLRIWLYLKLRHPDGRWKKHHIPFYDTRFFQEIYAAGNSPVDTCQFRTPRFGYHLP |
| | KLTDQTAIRVNKKHVKAAKTEARIRLAIQQGTLPVSNLKITRISATINSKGQVRIPVKF DVGRQKGTLQIGDRFCGYDQNQTASHAYSLWEVVKEGQYHKELGCFVRFISSGDIVP OSITIONNRGNQFDQLSYEGLAYPQYADWRKKASKFVSLWQITKKNKKKEIVTVEAK EKFDAICKYQPRLYKFNKEYAYLLRDIVRGKSLVELQQIRQEIFRFIEQDCGVTRLGSLS LSTLETVKAVKGIIYSYFSTALNASKNNPISDEQRKEFDPELFALLEKLELIRTRKKKQK VERIANSLIQTCLENNIKFIRGEGDLSTTNNATKKKANSRSMDWLARGVFNKIRQLAP MHNITLFGCGSLYTSHQDPLVHRNPDKAMKCRWAAIPVKDIGDWVLRKLSQNLRAK NIGTGEYYHQGVKEFLSHYELQDLEEELLKWRSDRKSNIPCWVLQNRLAEKLGNKE AVVYIPVRGGRIYFATHKVATGAVSIVFDQKQVWVCNADHVAAANIALTVKGIGEQSS DEENPDGSRIKLQLTS |
| SEQ ID NO. 22 CasXX - K844A | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPST LERFIAKDGQKEFDLKKLQTDLKKVIRGKSKERDWCCQEELRSYVEQNTIQYDLWA WGEMFNKAHTALKIKSTRNYNFAKQRLEQFKRIQSLNNLLVVKKLNDFFDSEFFSGE ETYTICVHHLGGKRLSKLYKAWEDDPADPENAIVVLCDDLKNNFKKEPIRNILRYIFTI RQECSAQDILAAAKYNQQLDRYKSQKANPSVLGNQGFTWRNAVILPEKAQRNDRPN SLDLRIWLYLKLRHPDGRWKKHHIPFYDTRFFQEIYAAGNSPVDTCQFRTPRFGYHLP KLTDQTAIRVNKKHVKAAKTEARIRLAIQQGTLPVSNLKITRISATINSKGQVRIPVKF DVGRQKGTLQIGDRFCGYDQNQTASHAYSLWEVVKEGQYHKELGCFVRFISSGDIVP OSITIONNRGNQFDQLSYEGLAYPQYADWRKKASKFVSLWQITKKNKKKEIVTVEAK EKFDAICKYQPRLYKFNKEYAYLLRDIVRGKSLVELQQIRQEIFRFIEQDCGVTRLGSLS LSTLETVKAVKGIIYSYFSTALNASKNNPISDEQRKEFDPELFALLEKLELIRTRKKKQK VERIANSLIQTCLENNIKFIRGEGDLSTTNNATAKKANSRSMDWLARGVFNKIRQLAP MHNITLFGCGSLYTSHQDPLVHRNPDKAMKCRWAAIPVKDIGDWVLRKLSQNLRAK NIGTGEYYHQGVKEFLSHYELQDLEEELLKWRSDRKSNIPCWVLQNRLAEKLGNKE AVVYIPVRGGRIYFATHKVATGAVSIVFDQKQVWVCNADHVAAANIALTVKGIGEQSS |
| | DEENPDGSRIKLQLTS |
| SEQ ID NO. 23 CasXX - R719A + K844A | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPST LERFIAKDGQKEFDLKKLQTDLKKVIRGKSKERDWCCQEELRSYVEQNTIQYDLWA WGEMFNKAHTALKIKSTRNYNFAKQRLEQFKRIQSLNNLLVVKKLNDFFDSEFFSGE ETYTICVHHLGGKRLSKLYKAWEDDPADPENAIVVLCDDLKNNFKKEPIRNILRYIFTI RQECSAQDILAAAKYNQQLDRYKSQKANPSVLGNQGFTWRNAVILPEKAQRNDRPN SLDLRIWLYLKLRHPDGRWKKHHIPFYDTRFFQEIYAAGNSPVDTCQFRTPRFGYHLP KLTDQTAIRVNKKHVKAAKTEARIRLAIQQGTLPVSNLKITRISATINSKGQVRIPVKF DVGRQKGTLQIGDRFCGYDQNQTASHAYSLWEVVKEGQYHKELGCFVRFISSGDIVP OSITIONNRGNQFDQLSYEGLAYPQYADWRKKASKFVSLWQITKKNKKKEIVTVEAK EKFDAICKYQPRLYKFNKEYAYLLRDIVAGKSLVELQQIRQEIFRFIEQDCGVTRLGSLS LSTLETVKAVKGIIYSYFSTALNASKNNPISDEQRKEFDPELFALLEKLELIRTRKKKQK VERIANSLIQTCLENNIKFIRGEGDLSTTNNATAKKANSRSMDWLARGVFNKIRQLAP MHNITLFGCGSLYTSHQDPLVHRNPDKAMKCRWAAIPVKDIGDWVLRKLSQNLRAK NIGTGEYYHQGVKEFLSHYELQDLEEELLKWRSDRKSNIPCWVLQNRLAEKLGNKE AVVYIPVRGGRIYFATHKVATGAVSIVFDQKQVWVCNADHVAAANIALTVKGIGEQSS DEENPDGSRIKLQLTS |
| SEQ ID NO.24 CasXX - R857A + K844A | MSSAIKSYKSVLRPNERKNQLLKSTIQCLEDGSAFFFKMLQGLFGGITPEIVRFSTEQE KQQQDIALWCAVNWFRPVSQDSLTHTIASDNLVEKFEEYYGGTASDAIKQYFSASIGE SYYWNDCRQQYYDLCRELGVEVSDLTHDLEILCREKCLAVATESNQYNSIISVLFGTG RKEDRSVKLRITKKILEAISNLKEIPKNVAPIQEIILNVAKATKETFRQVYAGNLGAPST LERFIAKDGQKEFDLKKLQTDLKKVIRGKSKERDWCCQEELRSYVEQNTIQYDLWA WGEMFNKAHTALKIKSTRNYNFAKQRLEQFKRIQSLNNLLVVKKLNDFFDSEFFSGE ETYTICVHHLGGKRLSKLYKAWEDDPADPENAIVVLCDDLKNNFKKEPIRNILRYIFTI RQECSAQDILAAAKYNQQLDRYKSQKANPSVLGNQGFTWRNAVILPEKAQRNDRPN |
| | SLDLRIWLYLKLRHPDGRWKKHHIPFYDTRFFQEIYAAGNSPVDTCQFRTPRFGYHLP KLTDQTAIRVNKKHVKAAKTEARIRLAIQQGTLPVSNLKITRISATINSKGQVRIPVKFD VGRQKGTLQIGDRFCGYDQNQTASHAYSLWEVVKEGQYHKELGCFVRFISSGDIVPO SITIONNRGNQFDQLSYEGLAYPQYADWRKKASKFVSLWQITKKNKKKEIVTVEAKE KFDAICKYQPRLYKFNKEYAYLLRDIVRGKSLVELQQIRQEIFRFIEQDCGVTRLGSLSL STLETVKAVKGIIYSYFSTALNASKNNPISDEQRKEFDPELFALLEKLELIRTRKKKQKV ERIANSLIQTCLENNIKFIRGEGDLSTTNNATAKKANSRSMDWLAAGVFNKIRQLAPM HNITLFGCGSLYTSHQDPLVHRNPDKAMKCRWAAIPVKDIGDWVLRKLSQNLRAKNI GTGEYYHQGVKEFLSHYELQDLEEELLKWRSDRKSNIPCWVLQNRLAEKLGNKEAV VYIPVRGGRIYFATHKVATGAVSIVFDQKQVWVCNADHVAAANIALTVKGIGEQSSDE ENPDGSRIKLQLTS |

## Claims

1. An engineered Cas 12i nuclease; comprising one or more mutations based on a reference Cas 12i nuclease, wherein the mutations are selected from:
(1) replacing one or more amino acids interacting with PAM in the reference Cas12i nuclease with positively charged amino acids;
(2) replacing one or more amino acids involved in opening a double-stranded DNA in the reference Cas12i nuclease with amino acids with an aromatic ring;
(3) replacing one or more amino acids located in the RuvC domain and interacting with a single-stranded DNA substrate in the reference Cas12i nuclease with positively charged amino acids;
(4) replacing one or more amino acids interacting with a DNA-RNA double helix in the reference Cas12i nuclease with positively charged amino acids; and
(5) replacing one or more polar or positively charged amino acids interacting with the DNA-RNA double helix in the reference Cas12i nuclease with hydrophobic amino acids;
preferably, the reference Cas12i nuclease is a wild-type Cas12i2 nuclease with an amino acid sequence shown in SEQ ID NO.1.

2. The engineered Cas12i nuclease according to claim 1, wherein the one or more amino acids interacting with the PAM are amino acids located within a distance of 9 angstroms from the PAM in three-dimensional structure, preferably, the one or more amino acids interacting with the PAM are one or more amino acids at the following positions: 176, 178, 226, 227, 229, 237, 238, 264, 447 and 563,
further preferably, the one or more amino acids interacting with the PAM are one or more of the following amino acids: E176, E178, Y226, A227, N229, E237, K238, K264, T447 and E563,
further preferably, the one or more amino acids interacting with the PAM are one or more of the following amino acids: E176, K238, T447 and E563,
wherein the amino acid position numbers are defined by the corresponding amino acid positions shown in SEQ ID NO. 1.

3. The engineered Cas12i nuclease according to claim 1 or 2, wherein the positively charged amino acid is R, K or H.

4. The engineered Cas12i nuclease according to any one of claims 1 to 3, wherein the replacement of one or more amino acids interacting with the PAM in the reference Cas12i nuclease with positively charged amino acids is one or more of the following replacements: E176R, K238R, T447R, and E563R;
preferably, the engineered Cas12i nuclease comprises any one of the following mutations or mutation combinations: (1) E563R; (2) E176R, T447R, E176R and E563R; (3) K238R and E563R; (4) E176R, K238R and T447R; (5) E176R, K238R, and E563R; (6) E176R, T447R, and E563R; and (7) E176R, K238R, T447R, and E563R;
wherein the amino acid positions are defined by the corresponding amino acid positions shown in SEQ ID NO.1.

5. The engineered Cas12i nuclease according to any one of claims 1 to 4, wherein the one or more amino acids involved in opening the double-stranded DNA are the amino acids interacting with the last base pair at the 3' end in PAM relative to the target strand;
preferably, the one or more amino acids involved in opening the double-stranded DNA are one or more amino acids at the following positions: 163 and 164;
further preferably, the one or more amino acids involved in opening the double-stranded DNA are one or more of the following amino acids: Q163 and N164;
further preferably, the amino acid involved in opening the double-stranded DNA is N164;
wherein the amino acid position numbers are defined by the corresponding amino acid positions shown in SEQ ID NO. 1.

6. The engineered Cas12i nuclease according to any one of claims 1 to 5, wherein the one or more amino acids involved in opening the double-stranded DNA are replaced with amino acids with an aromatic ring, and the amino acid with an aromatic ring is F, Y or W, preferably, the amino acid with an aromatic ring is F or Y.

7. The engineered Cas12i nuclease according to any one of claims 1 to 6, wherein the replacement of one or more amino acids involved in opening the double-stranded DNA in the reference Cas12i nuclease with amino acids with an aromatic ring comprises one or more of the following replacements: Q163F, Q163Y, Q163W, N164Y and N164F; preferably, the Cas12i nuclease comprises N164Y or N164F mutation; further preferably, the engineered Cas12i nuclease comprises N164Y.

8. The engineered Cas12i nuclease according to any one of claims 1 to 7, wherein the one or more amino acids located in the RuvC domain and interacting with the single-stranded DNA substrate are amino acids located within a distance of 9 angstroms from the single-stranded DNA substrate in three-dimensional structure;
preferably, the one or more amino acids located in the RuvC domain and interacting with the single-stranded DNA substrate are one or more amino acids at the following positions: 323, 327, 355, 359, 360, 361, 362, 388, 390, 391, 392, 393, 414, 417, 418, 421, 424, 425, 650, 652, 653, 696, 705, 708, 709, 751, 752, 755, 840, 848, 851, 856, 885, 897, 925, 926, 928, 929, 932, and 1022;
further preferably, the one or more amino acids located in the RuvC domain and interacting with the single-stranded DNA substrate are one or more of the following amino acids: E323, L327, V355, G359, G360, K361, D362, L388, N390 , N391, F392, K393, Q414, L417, L418, K421, Q424, Q425, S650, E652, G653, 1696, K705, K708, E709, L751, S752, E755, N840, N848, S851, A856, Q885 M897 , N925, I926, T928, G929, Y932, and A1022;
further preferably the one or more amino acids located in the RuvC domain and interacting with the single-stranded DNA substrate are one or more of the following amino acids: E323, D362, Q425, N925, I926, N391, Q424 and G929;
wherein the amino acid position numbers are defined by the corresponding amino acid positions shown in SEQ ID NO.1.

9. The engineered Cas12i nuclease according to any one of claims 1 to 8, wherein the replacement of one or more amino acids located in the RuvC domain and interacting with the single-stranded DNA substrate in the reference Cas12i nuclease with positively charged amino acids comprises replacement with R or K, preferably, the positively charged amino acid is R.

10. The engineered Cas12i nuclease according to any one of claims 1 to 9, wherein the replacements of one or more amino acids located in the RuvC domain and interacting with the single-stranded DNA substrate in the reference Cas12i nuclease with positively charged amino acids comprise one or more of the following replacements: E323R, D362R, N391R, Q424R, Q425R, N925R, I926R and G929R;
preferably, the engineered Cas12i nuclease comprises any one of the following mutations or mutation combinations: (1) E323R; (2) D362R; (3) Q425R; (4) N925R; (5) I926R; (6) E323R and D362R; (7) E323R and Q425R; (8) E323R and I926R; (9) Q425R and I926R; (10) D362R and I926R; (11) N925R and I926R; (12) E323R, D362R and Q425R; (13) E323R, D362R and I926R; (14) E323R, Q425R and I926R; (15) D362R, N925R and I926R; and (16) E323R, D362R, Q425R and I926R;
wherein the amino acid positions are defined by the corresponding amino acid positions shown in SEQ ID NO.1.

11. The engineered Cas12i nuclease according to any one of claims 1 to 10, wherein the one or more amino acids interacting with the DNA-RNA double helix are amino acids located within a distance of 9 angstroms from the DNA-RNA double helix in three-dimensional structure;
preferably, the one or more amino acids interacting with the DNA-RNA double helix are one or more amino acids at the following positions: 116, 117, 156, 159, 160, 161, 247, 293, 294, 297, 301, 305, 306, 308, 312, 313, 316, 319, 320, 343, 348, 349, 427, 433, 438, 441, 442, 679, 683, 691, 782, 783, 797, 800, 852, 853, 855, 861, 865, 957 and 958;
further preferably, the one or more amino acids interacting with the DNA-RNA double helix are one or more of the following amino acids: G116, E117, A156, T159, E160, S161, E247, G293, E294, N297, T301, I305, K306, T308, N312, F313, Q316, E319, Q320, E343, E348, E349, D427, K433, V438, N441, Q442, N679, E683, E691, D782, E783, E797, E800, M852, D853, L855, N861, Q865, S957 and D958;
further preferably, the one or more amino acids interacting with the DNA-RNA double helix are one or more of the following amino acids: G116, E117, T159, S161, E319, E343 and D958; wherein the amino acid positions are defined by the corresponding amino acid positions shown in SEQ ID NO:1.

12. The engineered Cas12i nuclease according to any one of claims 1 to 11, wherein the replacement of one or more amino acids interacting with the DNA-RNA double helix in the reference Cas12i nuclease with positively charged amino acids comprises replacement with R or K, preferably, the positively charged amino acid is R.

13. The engineered Cas12i nuclease according to any one of claims 1 to 12, wherein the replacements of one or more amino acids interacting with the DNA-RNA double helix in the reference Cas12i nuclease with positively charged amino acids comprise one or more of the following replacements: G116R, E117R, T159R, S161R, E319R, E343R and D958R;
preferably, the engineered Cas12i nuclease comprises D958R replacement;
wherein the amino acid positions are defined by the corresponding amino acid positions shown in SEQ ID NO.1.

14. The engineered Cas12i nuclease according to any one of claims 1 to 13, the one or more polar or positively charged amino acids interacting with the DNA-RNA double helix are amino acids selected from one or more of the following positions: 357, 394, 715, 719, 807, 844, 848, 857 and 861, that is, one or more of the following amino acids: H357, K394, R715, R719, K807, K844, N848, R857 and R861;
wherein the amino acid positions are defined by the corresponding amino acid positions shown in SEQ ID NO.1.

15. The engineered Cas12i nuclease according to any one of claims 1 to 14, wherein the replacement of one or more polar or positively charged amino acids interacting with the DNA-RNA double helix in the reference Cas12i nuclease with hydrophobic amino acids comprises replacement with alanine (A).

16. The engineered Cas12i nuclease according to any one of claims 1 to 15, comprising one or more mutations selected from the group consisting of: H357A, K394A, R715A, R719A, K807A, K844A, N848A, R857A and R861A;
preferably, comprising one or more mutations selected from the group consisting of: K394A, R719A, K844A and R857A;
wherein the amino acid positions are defined by the corresponding amino acid positions shown in SEQ ID NO.1.

17. The engineered Cas12i nuclease according to any one of claims 1 to 16, comprising R719A and K844A amino acid substitutions, or R857A and K844A amino acid substitutions; wherein the amino acid positions are defined by the corresponding amino acid positions shown in SEQ ID NO.1.

18. The engineered Cas12i nuclease according to any one of claims 1 to 17, further comprising one or more flexible region mutations, the mutations increase the flexibility of the flexible region in the reference Cas12i nuclease, wherein the flexible region is selected from amino acid residues 439-443 or amino acid residues 925-929;
preferably, the flexible region mutation is located at sites 439 and/or 926;
further preferably, the flexible region mutation is a mutation of L439 and/or I926;
wherein the amino acid positions are defined by the corresponding amino acid positions shown in SEQ ID NO.1.

19. The engineered Cas12i nuclease according to claim 18, wherein the one or more flexible region mutations are: replacing the amino acid in the flexible region with G, and/or inserting one or two Gs thereafter;
preferably, the one or more flexible region mutations comprise I926G, L439(L+G) or L439(L+GG);
further preferably, the one or more flexible region mutations comprise L439(L+G) or L439(L+GG);
wherein the amino acid position numbers are defined by the corresponding amino acid positions shown in SEQ ID NO.1.

20. An engineered Cas12i nuclease; comprising any one or more of the following mutations or mutation combinations:
(1) E563R; (2) E176R and T447R; (3) E176R and E563R; (4) K238R and E563R; (5) E176R, K238R and T447R; (6) E176R, T447R and E563R; (7) E176R, K238R and E563R; (8) E176R, K238R, T447R and E563R; (9) N164Y; (10) N164F; (11) E323R; (12) D362R; (13) Q425R; (14) N925R; (15) I926R; (16) D958R; (17) E323R and D362R; (18) E323R and Q425R; (19) E323R and I926R; (20) Q425R and I926R; (21) D362R and I926R; (22) N925R and I926R; (23) E323R, D362R and Q425R; (24) E323R, D362R and I926R; (25) E323R, Q425R and I926R; (26) D362R, N925R and I926R; (27) E323R, D362R, Q425R and I926R; (28) D362R and I926 G; (29) N925R and I926G; (30) D362R, N925R and I926G; (31) I926R and L439(L+G); (32) I926R and L439(L+GG); (33) E323R, D362R and I926G; ( 34) R719A and K844A; and (35) R857A and K844A;
preferably, comprising any one or more of following mutations or mutation combinations: (1) E176R, K238R, T447R and E563R; (2) N164Y; (3) I926R; (4) E323R and D362R; (4) I926G; (5) I926R and L439 (L+G); (6) I926R and L439 (L+GG); and (7) D958R;
wherein the amino acid positions are defined by the corresponding amino acid positions shown in SEQ ID NO.1.

21. An engineered Cas12i nuclease, comprising any one of the following mutation combinations: (1) E176R, K238R, T447R, E563R and N164Y; (2) E176R, K238R, T447R, E563R and I926R; (3) N164Y, E323R and D362R; (4) E176R, K238R, T447R, E563R, E323R and D362R; (5) N164Y and I926R; (6) E176R, K238R, T447R, E563R, N164Y and I926R; (7)E176R, K238R, T447R, E563R, N164Y, E323R and D362R; (8) E176R, K238R, T447R, E563R, N164Y, I926R, E323R and D362R; (9) E176R, K238R, T447R, E563R, N164Y, E323R, D362R and I926G; (10) E176R, K238R, T447R, E563R, N164Y, E323R, D362R, I926G and L439(L+GG); (11) E176R, K238R, T447R, E563R, N164Y, E323R, D362R, I926G and L439(L+G); (12) E176R, K238R, T447R, E563R, N164Y and D958R; (13) E176R, K238R, T447R, E563R, I926R and D958R; (14) E176R, K238R, T447R, E563R, E323R, D362R and D958R; (15) N164Y, I926R and D958R; (16) N164Y, E323R, D362R and D958R; (17) E176R, K238R, T447R, E563R, N164Y, I926R and D958R; (18) E176R, K238R, T447R, E563R, N164Y, E323R, D362R and D958R; (19) E176R, K238R, T447R, E563R, N164Y, I926R, E323R, D362R and D958R; (20) E176R, K238R, T447R, E563R, N164Y, E323R, D362R, I926G and D958R; (21) E176R, K238R, T447R, E563R, N164Y, E323R, D362R, I926G, L439(L+GG) and D958R; (22)E176R, K238R, T447R, E563R, N164Y, E323R, D362R, I926G, L439(L+G) and D958R; (23) E176R, K238R, T447R, E563R, N164Y, E323R, D362R and R857A; (24) E176R, K238R, T447R, E563R, N164Y, E323R, D362R and N861A; (25) E176R, K238R, T447R, E563R, N164Y, E323R, D362R and K807A; (26) E176R, K238R, T447R, E563R, N164Y, E323R, D362R and N848A; (27) E176R, K238R, T447R, E563R, N164Y, E323R, D362R and R715A; (28)E176R, K238R, T447R, E563R, N164Y, E323R, D362R and R719A; (29) E176R, K238R, T447R, E563R, N164Y, E323R, D362R and K394A; (30) E176R, K238R, T447R, E563R, N164Y, E323R, D362R and H357A; (31) E176R, K238R, T447R, E563R, N164Y, E323R, D362R and K844A; (32) E176R, K238R, T447R, E563R, N164Y, E323R, D362R, R719A and K844A; or (33) E176R, K238R, T447R, E563R, N164Y, E323R, D362R, R857A and K844A; wherein the amino acid positions are defined by the corresponding amino acid positions shown in SEQ ID NO.1.

22. The engineered Cas12i nuclease according to any one of claims 1 to 21, comprising the engineered Cas12i nuclease with an amino acid sequence shown in any one of SEQ ID NOs. 2 to 24, or an amino acid sequence having at least 80% identity with any amino acid sequence shown in SEQ ID NOs. 2-24.

23. An engineered Cas12i effector protein, comprising the engineered Cas12i nuclease according to any one of claims 1 to 22 or the functional derivative thereof;
optionally the engineered Cas12i nuclease or the functional derivative thereof has enzymatic activity, or the engineered Cas12i nuclease or the functional derivative thereof is an enzyme inactive mutant.

24. The engineered Cas12i effector protein according to claim 23, wherein the Cas12i effector protein is capable of inducing double-strand breaks or single-strand breaks in DNA molecules.

25. The engineered Cas12i effector protein according to claim 23, wherein the engineered Cas12i nuclease or a functional derivative thereof is an enzyme inactive mutant comprising one or more of the following mutations: D599A, E833A, S883A, H884A, R900A and D1019A; wherein the amino acid positions are defined by the corresponding amino acid positions shown in SEQ ID NO.1.

26. The engineered Cas12i effector protein according to any one of claims 23 to 25, further comprising a functional domain fused to the engineered Cas12i nuclease or a functional derivative thereof.

27. The engineered Cas12i effector protein according to claim 26, wherein the functional domain is one or more selected from the group consisting of: translation initiation domain, transcription repression domain, transactivation domain, epigenetic modification domain, nucleobase editing domain, reverse transcriptase domain, reporter domain and nuclease domain.

28. The engineered Cas12i effector protein according to any one of claims 23 to 27, comprising: a first polypeptide comprising the N-terminal portion of the engineered Cas 12i nuclease or a functional derivative thereof and a second polypeptide comprising the C-terminal portion of the engineered Cas12i nuclease or a functional derivative thereof, wherein the first polypeptide and the second polypeptide are capable of associating with each other in the presence of a guide RNA comprising a guide sequence to form a clustered regularly interspaced short palindromic repeats (CRISPR) complex that specifically binds to a target nucleic acid comprising a target sequence complementary to the guide sequence;
preferably, the first polypeptide comprises amino acid residues 1~X of the N-terminal portion of the engineered Cas12i nuclease according to any one of claims 1 to 22, and the second polypeptide comprises amino acid residues from X+1 to the C-terminal of the engineered Cas12i nuclease according to any one of claims 1 to 22;
optionally, the first polypeptide and the second polypeptide comprise a dimerization domain respectively;
optionally, the dimerization domain of the first polypeptide and the dimerization domain of the second polypeptide associate with each other in the presence of an inducer.

29. An engineered CRISPR-Cas12i system, comprising:
(a) the engineered Cas12i nuclease according to any one of claims 1 to 28, or the engineered Cas12i effector protein according to any one of claims 23 to 28; and
(b) a guide RNA comprising a guide sequence complementary to a target sequence, or one or more nucleic acids encoding the guide RNA,
wherein the engineered Cas12i nuclease or engineered Cas12i effector protein and the guide RNA can form a CRISPR complex that specifically binds to a target nucleic acid comprising the target sequence and induces modification of the target nucleic acid;
preferably, the guide RNA is crRNA comprising the guide sequence;
further preferably, the engineered CRISPR-Cas12i system comprises a precursor guide RNA array encoding multiple crRNAs;
or, preferably the engineered Cas12i nuclease or engineered Cas12i effector protein is a master editor, and the guide RNA is a prime editing guide RNA (pegRNA).

30. The engineered CRISPR-Cas12i system according to claim 29, comprising one or more vectors encoding the engineered Cas12i nuclease or engineered Cas12i effector protein;
preferably, the one or more vectors are selected from the group consisting of: retroviral vectors, lentiviral vectors, adenoviral vectors, adeno-associated virus vectors and herpes simplex vectors;
further preferably, the one or more vectors are adeno-associated virus (AAV) vectors;
further preferably, the AAV vector also encodes the guide RNA.

31. A method for detecting target nucleic acid in a sample, comprising:
(a) contacting the sample with the engineered CRISPR-Cas12i system according to claim 29 and a tagged detection nucleic acid that is single-stranded and does not hybridize to the guide sequence of the guide RNA; and
(b) measuring a detectable signal generated by cleavage of the tagged detection nucleic acid by the engineered Cas12i nuclease or engineered Cas12i effector protein, thereby detecting the target nucleic acid.

32. A method for modifying a target nucleic acid comprising a target sequence, comprising contacting the target nucleic acid with the engineered CRISPR-Cas12i system according to claim 29 or 30;
preferably, the method is performed in vitro, ex vivo or in vivo;
further preferably, the target nucleic acid is present in a cell;
further preferably, the cell is a bacterial cell, a yeast cell, a mammalian cell, a plant cell, or an animal cell;
further preferably, the target nucleic acid is genomic DNA;
further preferably, the target sequence is associated with a disease or disorder;
further preferably, the engineered CRISPR-Cas12i system comprises a precursor guide RNA array encoding multiple crRNAs, wherein each crRNA comprises a different guide sequence.

33. Use of the engineered CRISPR-Cas12i system according to claim 29 or 30 in the preparation of a medicament for the treatment of a disease or disorder associated with a target nucleic acid in a cell of an individual; preferably, the disease or disorder is selected from the group consisting of: cancer, cardiovascular disease, genetic disease, autoimmune disease, metabolic disease, neurodegenerative disease, eye disease, bacterial infection and viral infection.

34. A method for treating a disease or disorder associated with a target nucleic acid in a cell of an individual, comprising using the method according to claim 32 to modify the target nucleic acid in the cell of the individual, thereby treating the disease or disorder; preferably, the disease or disorder is selected from the group consisting of: cancer, cardiovascular disease, genetic disease, autoimmune disease, metabolic disease, neurodegenerative disease, eye disease, bacterial infection and viral infection.

35. A method for modifying a target nucleic acid comprising a target sequence, comprising contacting the target nucleic acid with the engineered CRISPR-Cas12i system according to claim 29 or 30.

36. A composition or kit, comprising the engineered Cas12i nuclease according to any one of claims 1 to 22, the engineered Cas12i effector protein according to any one of claims 23 to 28.

37. An engineered cell, comprising a modified target nucleic acid, wherein the target nucleic acid is modified by the method according to claim 32 or 35.

38. An engineered non-human animal, comprising one or more engineered cells according to claim 37.
